# EUROPEAN PATENT SPECIFICATION

(11) **EP 3 125 866 B1**
(45) Date of publication and mention of the grant of the patent: **03.03.2021**
(21) Application number: 15773877.4
(22) Date of filing: 06.04.2015
(51) Int. Cl.: A61K 9/00

(54) **REFILLABLE DRUG DELIVERY DEVICES AND METHODS OF USE THEREOF**
NACHFÜLLBARE WIRKSTOFFFREISETZUNGSVORRICHTUNGEN UND VERFAHREN ZUR VERWENDUNG DAVON
DISPOSITIFS RECHARGEABLES D'ADMINISTRATION DE MÉDICAMENTS, ET PROCÉDÉS POUR LEUR UTILISATION

(30) Priority: 04.04.2014 US 201461975443 P; 01.12.2014 US 201462085898 P
(43) Date of publication of application: 08.02.2017
(73) Proprietor: President and Fellows of Harvard College, Cambridge, MA 02138 (US)
(72) Inventor: BRUDNO, Yevgeny, Somerville, MA 02143 (US); KEARNEY, Cathal, J., Boston, MA 02130 (US); SILVA, Eduardo, Davis, CA 95618 (US); AIZENBERG, Michael, Cambridge, MA 02138 (US); KWEE, Brian, Cambridge, MA 02138 (US); DESAI, Rajiv, San Diego, CA 92131 (US); JOSHI, Neel, S., Somerville, MA 02143 (US); MOONEY, David, J., Sudbury, MA 01776 (US)
(74) Representative: Lock, Graham James
(86) International application number: PCT/US2015/024540
(87) International publication number: WO 2015/154082

(56) References cited:
- WO-A1-2012/049624
- WO-A1-2012/156918
- WO-A1-2014/205126
- WO-A2-2008/103693
- WO-A2-2010/096449
- US-A- 5 690 954
- US-B2- 7 465 298
- GUO ET AL.: 'Functional alginate nanoparticles for efficient intracellular release of doxorubicin and hepatoma carcinoma cell targeting therapy' INT J PHARM. vol. 451, no. 1-2, 22 April 2013, pages 1 - 11, XP028556769
- PARK ET AL.: 'Replenishable dendrimer-nanoparticle hybrid membranes for sustained release of therapeutics' NANOSCALE vol. 5, no. 17, 07 September 2013, pages 7805 - 780, XP055229945
- GAWEL ET AL.: 'Responsive Hydrogels for Label-Free Signal Transduction within Biosensors' SENSORS vol. 10, 30 April 2010, pages 4381 - 4409, XP002697114
- BRUDNO ET AL.: 'Refilling drug delivery depots through the blood' PROC NATL ACAD SCI USA vol. 111, no. 35, 19 August 2014, pages 12722 - 12727, XP055229948
- Janek Szychowski ET AL: "Cleavable Biotin Probes for Labeling of Biomolecules via Azide-Alkyne Cycloaddition", Journal of the American Chemical Society, vol. 132, no. 51, 29 December 2010 (2010-12-29), pages 18351-18360, XP055029467, ISSN: 0002-7863, DOI: 10.1021/ja1083909
- Enrique Lallana Ozores ET AL: "Click Chemistry with Polymers, Dendrimers, and Hydrogels for Drug Delivery Methods for the simplification of the NMR of complex mixtures and pseudo separations View project Dendrimers for biomedical applications View project", , 25 January 2012 (2012-01-25), XP055598051, DOI: 10.1007/s11095-012-0683-y.Source: Retrieved from the Internet: URL:file:///C:/Users/HS51195/Downloads/art 3A10.10072Fs11095-012-0683-y.pdf [retrieved on 2019-06-19]
- Jos? M. Mej?a Oneto ET AL: "Implantable biomaterial based on click chemistry for targeting small molecules", Acta Biomaterialia, vol. 10, no. 12, 24 August 2014 (2014-08-24), pages 5099-5105, XP055301001, AMSTERDAM, NL ISSN: 1742-7061, DOI: 10.1016/j.actbio.2014.08.019

## Description

### BACKGROUND OF THE INVENTION

Drug-eluting polymer systems have proven useful in a variety of clinical settings, including prevention of restenosis with stenting, cancer treatment and enhancing wound healing. See, e.g., Simard T, et al. (2013) The Canadian journal of cardiology; Wessely R (2010) Nature reviews. Cardiology 7(4):194-203; Iwamoto T (2013) Biological & pharmaceutical bulletin 36(5):715-718; Attanasio S & Snell J (2009) Cardiology in review 17(3):115-120; Freedman S & Isner J (2001) Journal of molecular and cellular cardiology 33(3):379-393; and Losordo D & Dimmeler S (2004) Circulation 109(21):2487-2491. These systems benefit from tunable drug release kinetics, days or even weeks of continuous drug release, and local delivery which together provide spatiotemporal control over drug availability and can diminish drug toxicity. See Kearney C & Mooney D (2013) Nature materials 12(11):1004-1017. However, existing drug-eluting systems have a finite depot of drug and become unneeded when spent and, in the case of non-degrading systems, may need surgical removal. For many therapeutic applications, an invasive procedure is needed to inject or implant a drug-eluting device, and these devices cannot be refilled or replaced without another invasive surgery.

WO 2010/096649 A2 discloses a system comprising an implantable drug delivery pump device comprising a chamber containing a drug fluid having attached thereto a catheter with a dispensing end, and a refill container comprising a drug fluid and a needle attached thereto in flow communication with the drug fluid, wherein the implanted pump device is refilled by positioning the needle on the pump device such that refill drug fluid is drawn from the refill container into the chamber of the pump device.

Guo et al., Int. J. Pharm., 2013, 451(1-2):1-11 describes a pH-sensitive system for the delivery of chemotherapy drugs into hepatoma cells. The system comprises nanoparticles of alginate conjugated to the hepatocyte-targeting ligand glycyrrhetinic acid and alginate conjugated to doxorubicin (via a hydrazine linker). The nanoparticles are internalized by hepatocytes which allow for delivery of the chemotherapeutic drug doxorubicin to hepatoma cells.

Park et al. (Nanoscale, 2013, 5(17):7805-7808) discloses porous hybrid membranes composed of anodic aluminum oxide (AAO) disk coated with poly(amido amine) (PAMAM) dendrimer-gold nanoparticle composites, wherein the porosity of the dendrimer-nanoparticle coating is regulated in order to control the release of the therapeutic agent loaded in the AAO disk.

US 7 465 298 B2 discloses coated drug entrapped polymer matrix on a stent, US 5 690 954 A discloses nasal compositions, and Gawel et al., "Responsive Hydrogels for Label-Free Signal Transduction within Biosensors", SENSORS, vol. 10, (2010) pages 4381-4409 describes in general the use of hydrogels in drug delivery devices.

Drug delivery depots used in the clinic today are single use, with no ability to refill once exhausted of drug. There is currently no non-invasive technique to refill these systems once their payload is exhausted. Thus, there is a need for a non-invasive method to refill a localized drug delivery device.

### SUMMARY OF THE INVENTION

The invention is based in part on the development of systemically administered drug payloads that home to and refill resident, e.g., previously administered/implanted, drug delivery systems, e.g., hydrogel drug delivery systems. The invention addresses the needs described above.

According to one aspect, the invention is a refillable drug delivery system as defined in the appended claims.

According to other aspects, the refillable drug delivery system according to the invention is for use in (1) refilling a drug delivery device in vivo; (2) maintaining or reducing the size of a tumor in a subject, wherein the pharmaceutical composition comprises an anti-cancer drug; (3) reducing cancer progression in a subject, wherein the pharmaceutical composition comprises an anti-cancer drug; (4) promoting wound healing in a subject, wherein the pharmaceutical composition promotes angiogenesis and/or maturation or remodeling of an existing blood vessel; (5) reducing or controlling inflammation in a subject, wherein the pharmaceutical composition comprises an anti-inflammatory agent; (6) treating an eye disease in a subject, wherein the pharmaceutical composition treats said eye disease; or (7) treating arrhythmia in a subject, wherein the pharmaceutical composition treats said arrhythmia, as defined in the appended claims.

The drug delivery depots of the invention are systemically administered, e.g., enterally administerd (e.g., orally, buccally, sublingually, or rectally) or parenterally administered (e.g., intravenously, intra-arterially, intraosseously, intra-muscularly, intracerebrally, intracerebroventricularly, intrathecally, or subcutaneously). Other suitable modes of administration of the drug delivery systems of the invention include hypodermal, intraperitoneal, intraocular, intranasal, transdermal (e.g., via a skin patch), epidural, intracranial, percutaneous, intravaginal, intrauterineal, intravitreal, or transmucosal administration, or administration via injection, via aerosol-based delivery, or via implantation.

A device is a structure, such as an apparatus, a delivery scaffold or vehicle, an implant, an instrument, or similar article that is administered to the body. For example, the device is injected or implanted into a bodily tissue to deliver a therapeutic agent and/or receive an initial dose of therapeutic agent or subsequent, e.g., refill, dose of the therapeutic agent. The devices and systems described herein mediate targeting and accumulation of therapeutic agents, e.g., small molecules, therapeutic peptides and proteins, therapeutic nucleic acids, to drug delivery devices in a minimally invasive manner. For example, devices implanted or injected for local drug delivery, such as gels or stents, are modified to capture small molecule drugs or drug refills infused into the blood or ingested. Drug payloads infused into the blood of a patient localize to target areas and are bound by the device, permitting subsequent sustained release of the drug at the target site.

In some examples, the drug delivery system described herein comprises the use of non-viral vectors such as inorganic nanoparticles, e.g., calcium phosphate polymer nano-formulations, condensed nucleic acids, and liposomal nucleic acids to deliver the drug to the device. For example, cationic liposomes or cationic polymers are used to deliver a nucleic acid, e.g., ribonucleic acid (RNA) or deoxyribonucleic acid (DNA), to the device. In some cases, the nucleic acid comprises siRNA, RNAi, mRNA, and microRNA. Other forms of nucleic acids include antisense and antigene molecules, splice-correction nucleic acids, gapmers, and antigomirs. For example, non-viral vectors are used to deliver an individual nucleic acid molecule comprising a click chemistry homing motif to a device. An antisense molecule may be administered systemically, e.g., intravenously, without a nanocarrier.

Described herein, hydrogels are modified with single-stranded DNA that provide a target for drug payloads in the form of nanoparticles carrying complementary DNA. Coupling DNA to free alginate polymer leads to specific binding to complementary-DNA-carrying alginate gels *in vitro,* and to injected gels *in vivo.* The results described herein show that when coupled to a drug payload, DNA-targeted refilling of a delivery depot, e.g., intra-tumor hydrogels, completely abrogated tumor growth.

Described herein is a method of nanotherapeutic drug delivery, e.g., a DNA nanotechnology-based approach for blood-based drug refilling of intra-tumor drug depots. The utility of this approach was demonstrated by the ability of the devices and methods described herein to inhibit tumor growth to a greater extent than strategies that rely on enhanced permeability and retention alone. The drug delivery devices of the invention have applications in numerous diseases, e.g., refilling drug depots in cancer therapy, wound healing, inflammation, and drug-eluting vascular grafts and stents.

The invention features a system comprising a drug delivery device, e.g., stationary drug delivery device, and a systemically delivered drug refill composition. The drug delivery device comprises a target recognition molecule and a hydrogel (e.g., a synthetic hydrogel or a biological hydrogel. Described herein, the drug delivery device also comprises a pharmaceutical composition.. The drug refill comprises a pharmaceutical composition attached to a target molecule via a cleavable linker. The target molecule and the target recognition molecule form a two-component binding pair. The drug refill is mobile until the target molecule on the drug refill binds to the target recognition molecule on the drug delivery device. Upon binding of the target molecule to the target recognition molecule, the drug refill delivers the pharmaceutical composition to the drug delivery device, thereby refilling the drug delivery device. In some embodiments, the drug refill further comprises a nanocarrier, e.g., a polymer. For example, the pharmaceutical composition is connected e.g., by a covalent, non-covalent, or ionic bond, to the target molecule. Alternatively, the pharmaceutical composition is connected, e.g., by a covalent, non-covalent, or ionic bond, to the polymer. In another embodiment, the target molecule is connected, e.g., by a covalent, non-covalent, or ionic bond, to the polymer.

In some cases, the system comprises multiple delivery devices, e.g., at least 2; at least 3; at least 4; at least 5, at least 6; at least 7; at least 8; at least 9; at least 10; at least 11; at least 12; at least 13; at least 14; at least 15; at least 20; at least 25; at least 30; at least 35; at least 40; at least 45; at least 50 or more devices. In some cases, the system comprises multiple drug refills, e.g., at least 2; at least 3; at least 4; at least 5, at least 6; at least 7; at least 8; at least 9; at least 10; at least 11; at least 12; at least 13; at least 14; at least 15; at least 20; at least 25; at least 30; at least 35; at least 40; at least 45; at least 50 or more drug refills.

Optionally, each drug refill comprises a pharmaceutical composition and a different target molecule and each drug delivery device comprises a different target recognition molecule. Optionally, each drug refill comprises a different pharmaceutical composition. In this manner, each drug refill binds to a different drug delivery device in a different location, thereby allowing for independent refill of each device, e.g., at multiple disease sites. Alternatively, each drug refill comprises multiple targets and each delivery device comprises multiple target recognition molecules.

Some drug delivery devices described herein comprise protein. Other drug delivery devices described herein do not comprise protein. Some devices described herein do not comprise a living cell, e.g., a cancer cell, or other biological entity. Preferably, the target recognition molecule is not bound to a living cell, a protein, or a membrane-bound receptor or ligand. Instead, the target recognition molecule is attached directly to the device, i.e., there is no intermediate moiety, i.e., a receptor, a linker, a ligand, etc., between the device and the target recognition molecule. For this reason, unlike intermediate binding moieties that could cause non-specific interactions, there is no non-specific binding associated with the drug delivery systems described herein. In this manner, the pharmaceutical composition (e.g., drug) and target molecule are directed to a target recognition molecule on the device itself.

The drug delivery device in the drug delivery system of the invention comprises a hydrogel (e.g., a synthetic hydrogel or a biological hydrogel) and a target recognitionmolecule. For example, the device is implanted or injected without a pharmaceutical composition, e.g., a drug. In this case, the pharmaceutical composition and target molecule is first loaded to the drug delivery device after the drug delivery device is implanted or injected. For example, a drug delivery device comprising a hydrogel and a target recognition molecule is implanted or injected. Subsequently, if infection occurs, an antibiotic and target molecule is first loaded to the drug delivery device.

The drug refill in the drug delivery system of the invention comprises a pharmaceutical composition attached to a target molecule via a cleavable linker, wherein the target molecule is capable of binding to a target recognition molecule, on a drug delivery device, wherein the drug refill is mobile until the target molecule binds to the target recognition molecule, and wherein upon binding of the target molecule to the target recognition molecule, the drug refill delivers/replenishes the pharmaceutical composition in or on the drug delivery device. The drug refill may further comprise a nanocarrier, e.g., a polymer, a nanoparticle, a liposome nanoparticle, dendrimer, a carbon nanotube, a micelle, protein (e.g., albumin), silica, or metal (e.g, gold, silver, or iron) nanoparticle. For example, the pharmaceutical composition is connected e.g., by a covalent, non-covalent, or ionic bond, to the target molecule. Alternatively, the pharmaceutical composition is connected, e.g., by a covalent, non-covalent, or ionic bond, to the nanocarrier. The target molecule may be connected, e.g., by a covalent, non-covalent, or ionic bond, to the polymer.

Described herein, the target and/or target recognition moiety may comprise a nucleic acid, peptide, polysaccharide, lipid, small molecule, or combination thereof, and the homing agent may comprise a nucleic acid, peptide, polysaccharide, lipid, small molecule, or combination thereof.

Described herein, the target may comprise one or more nucleotides that are complementary to one or more nucleotides of the target recognition moiety. For example, 50% or more (e.g., 50%, 60%, 70%, 80%, 90%, 95%, or 100%) of the nucleotides of the target may be complementary to 50% or more (e.g., 50%, 60%, 70%, 80%, 90%, 95%, or 100%) of the nucleotides of the target recognition moiety.

The melting temperature (Tm) of the target:target recognition moiety hybridization is at least 40 °C, e.g., at least 40 °C, 45 °C, 50 °C, 55 °C, 60 °C, 65 °C, 70 °C, 75 °C, 80 °C, 85 °C, 90 °C, 95 °C, or 100 °C, or more.

Nucleic acids described herein include DNA, modified DNA, RNA, modified RNA, locked nucleic acids (LNAs), peptide nucleic acids (PNAs), threose nucleic acids (TNA), hexitol nucleic acids (HNAs), bridge nucleic acids, cyclohexenyl nucleic acids, glycerol nucleic acids, morpholinos, phosphomorpholinos, as well as aptamers and catalytic nucleic acid versions thereof. For example, the nucleic acid contains a modified nitrogenous base. For example, modified DNA and RNA include 2'-o-methyl DNA, 2'-o-methyl RNA, 2'-fluoro-DNA, 2'-fluoro-RNA, 2'-methoxy-purine, 2'-fluoro-pyrimidine, 2'-methoxymethyl-DNA, 2'-methoxymethyl-RNA, 2'-acrylamido-DNA, 2'-acrylamido-RNA, 2'-ethanol-DNA, 2'-ethanol-RNA, 2'-methanol-DNA, 2'-methanol-RNA, and combinations thereof. The nucleic acid may contain a phosphate backbone. Also described herein, the nucleic acid contains a modified backbone, e.g., a phosphorothioate backbone, phosphoroborate backbone, methyl phosphonate backbone, phosphoroselenoate backbone, or phosphoroamidate backbone.

The nucleic acid described herein may be single-stranded. For example, the nucleic acid is partially single-stranded and partially double-stranded, e.g., due to secondary structures, such as hairpins.

Described herein, each nucleic acid molecule may comprise 8-50 nucleotides, e.g., 8, 10, 12, 14, 16, 18, 20, 22, 24, 26, 28, 30, 32, 34, 36, 38, 40, 42, 44, 46, 48, or 50 nucleotides, e.g., 20 nucleotides.

Described herein, the target and/or the target recognition moiety may comprise a nucleic acid molecule comprising 8-50 nucleotides, e.g., 8, 10, 12, 14, 16, 18, 20, 22, 24, 26, 28, 30, 32, 34, 36, 38, 40, 42, 44, 46, 48, or 50 nucleotides, e.g., 20 nucleotides.

Described herein, the target may comprise a phosphorothioate nucleic acid molecule having the sequence of TTTTTTTTTTTTTTTTTTTT (SEQ ID NO: 1), also called (T)₂₀. Descibed herein, the target may comprise a phosphorothioate nucleic acid molecule having the sequence of AAAAAAAAAAAAAAAAAAAA (SEQ ID NO: 2), also called (A)₂₀.

Described herein, the target recognition moiety may comprise a phosphorothioate nucleic acid molecule having the sequence of TTTTTTTTTTTTTTTTTTTT (SEQ ID NO: 1), also called (T)₂₀. Described herein, the target recognition moiety may comprise a phosphorothioate nucleic acid molecule having the sequence of AAAAAAAAAAAAAAAAAAAA (SEQ ID NO: 2), also called (A)₂₀.

Described herein, the target may comprise biotin or desthiobiotin, and the target recognition moiety may comprise avidin, neutravidin, streptavidin, or other form of avidin. Alternatively, the target may comprise avidin, neutravidin, streptavidin, or other form of avidin and the target recognition moiety may comprise biotin or desthiobiotin.

According to the invention, the target molecule comprises a bioorthogonal functional group and the target recognition molecule comprises a complementary functional group, where the bioorthogonal functional group is capable of reacting by click chemistry with the complementary functional group to form a covalent bond.

Exemplary bioorthogonal functional group/complementary functional group pairs include azide with phosphine; azide with cyclooctyne; nitrone with cyclooctyne; nitrile oxide with norbornene; oxanorbornadiene with azide; trans-cyclooctene with s-tetrazine; quadricyclane with bis(dithiobenzil)nickel(II).

In some cases, the bioorthogonal functional group comprises an alkene, e.g., a cyclooctene, e.g., a transcyclooctene (TCO) or norbornene (NOR), and the complementary functional group comprises a tetrazine (Tz). In some examples, the bioorthogonal functional group comprises an alkyne, e.g., a cyclooctyne such as dibenzocyclooctyne (DBCO), and the complementary functional group comprises an azide (Az). In other examples, the bioorthogonal functional group comprises a Tz, and the complementary functional group comprises an alkene such as transcyclooctene (TCO) or norbornene (NOR). Alternatively or in addition, the bioorthogonal functional group comprises an Az, and the complementary functional group comprises a cyclooctyne such as dibenzocyclooctyne (DBCO). This list is non-exhaustive and non-limiting, as a person skilled in the art could readily identify a number of other possible biorthogonal-complementary functional group pairs.

Described herein, the ratio of target molecules (e.g., nucleic acid molecules, biotin, streptavidin, avidin, or a bioorthogonal functional group described herein) to polymer molecules on the refill (e.g., alginate strands) may be 1:1, 2:1, 3:1, 4:1, 5:1, 6:1, 7:1, 8:1, 9:1, 10:1, or higher.

Described herein, the ratio of the target recognition molecule (e.g., nucleic acid molecules, biotin, streptavidin, avidin, or a bioorthogonal functional group described herein) to the polymer molecule of the device (e.g., alginate hydrogel) may be at least 5:1, e.g., at least 5:1, 10:1, 20:1, 30:1, 40:1, 50:1, or greater.

The binding affinity of the target molecule for the target recognition molecule may be 500 µM or less.

The binding affinity between the target molecule and target recognition molecule, e.g., measured by dissociation constant (K_{D}) may be 1 mM or less, e.g., 1 mM, 900 µM, 800 µM, 700 µM, 600 µM, 500 µM, 400 µM, 300 µM, 200 µM, 100 µM, 50 µM, 10 µM, 1 µM, 500 nM, 250 nM, 100 nM, 50 nM, 10 nM, 1 nM, 500 pM, 250 pM, 100 pM, 50 pM, 10 pM, 1 pM, 0.1 pM, 0.01 pM, or less. The K_{D} of the interaction between the homing agent and the targeting agent is measured using standard methods in the art.

In some embodiments the pharmaceutical composition in the drug refill of the refillable drug delivery system comprises an anti-cancer drug, a drug that promotes wound healing, a drug that treats or prevents infection, or a drug that promotes vascularization. For example, the pharmaceutical composition comprises an anti-cancer drug. For example, the anti-cancer drug comprises a chemotherapeutic, e.g., a cancer vaccine.

Exemplary anti-cancer drugs include but are not limited to Abiraterone Acetate, Abitrexate (Methotrexate), Abraxane (Paclitaxel Albumin-stabilized Nanoparticle Formulation), ABVD, ABVE, ABVE-PC, AC, AC-T, Adcetris (Brentuximab Vedotin), ADE, Ado-Trastuzumab Emtansine, Adriamycin (Doxorubicin Hydrochloride), Adrucil (Fluorouracil), Afatinib Dimaleate, Afinitor (Everolimus), Aldara (Imiquimod), Aldesleukin, Alemtuzumab, Alimta (Pemetrexed Disodium), Aloxi (Palonosetron Hydrochloride), Ambochlorin (Chlorambucil), Amboclorin (Chlorambucil), Aminolevulinic Acid, Anastrozole, Aprepitant, Aredia (Pamidronate Disodium), Arimidex (Anastrozole), Aromasin (Exemestane), Arranon (Nelarabine), Arsenic Trioxide, Arzerra (Ofatumumab), Asparaginase Erwinia chrysanthemi, Avastin (Bevacizumab), Axitinib, Azacitidine, BEACOPP, Bendamustine Hydrochloride, BEP, Bevacizumab, Bexarotene, Bexxar (Tositumomab and I 131 Iodine Tositumomab), Bicalutamide, Bleomycin, Bortezomib, Bosulif (Bosutinib), Bosutinib, Brentuximab Vedotin, Busulfan, Busulfex (Busulfan), Cabazitaxel, Cabozantinib-S-Malate, CAF, Campath (Alemtuzumab), Camptosar (Irinotecan Hydrochloride), Capecitabine, CAPOX, Carboplatin, Carboplatin-Taxol, Carfilzomib, Casodex (Bicalutamide), CeeNU (Lomustine), Cerubidine (Daunorubicin Hydrochloride), Cervarix (Recombinant HPV Bivalent Vaccine), Cetuximab, Chlorambucil, Chlorambucil-Prednisone, CHOP, Cisplatin, Clafen (Cyclophosphamide), Clofarabine, Clofarex (Clofarabine), Clolar (Clofarabine), CMF, Cometriq (Cabozantinib-S-Malate), COPP, COPP-ABV, Cosmegen (Dactinomycin), Crizotinib, CVP, Cyclophosphamide, Cyfos (Ifosfamide), Cytarabine, Cytarabine, Liposomal, Cytosar-U (Cytarabine), Cytoxan (Cyclophosphamide), Dabrafenib, Dacarbazine, Dacogen (Decitabine), Dactinomycin, Dasatinib, Daunorubicin Hydrochloride, Decitabine, Degarelix, Denileukin Diftitox, Denosumab, DepoCyt (Liposomal Cytarabine), DepoFoam (Liposomal Cytarabine), Dexrazoxane Hydrochloride, Docetaxel, Doxil (Doxorubicin Hydrochloride Liposome), Doxorubicin Hydrochloride, Doxorubicin Hydrochloride Liposome, Dox-SL (Doxorubicin Hydrochloride Liposome), DTIC-Dome (Dacarbazine), Efudex (Fluorouracil), Elitek (Rasburicase), Ellence (Epirubicin Hydrochloride), Eloxatin (Oxaliplatin), Eltrombopag Olamine, Emend (Aprepitant), Enzalutamide, Epirubicin Hydrochloride, EPOCH, Erbitux (Cetuximab), Eribulin Mesylate, Erivedge (Vismodegib), Erlotinib Hydrochloride, Erwinaze (Asparaginase Erwinia chrysanthemi), Etopophos (Etoposide Phosphate), Etoposide, Etoposide Phosphate, Evacet (Doxorubicin Hydrochloride Liposome), Everolimus, Evista (Raloxifene Hydrochloride), Exemestane, Fareston (Toremifene), Faslodex (Fulvestrant), FEC, Femara (Letrozole), Filgrastim, Fludara (Fludarabine Phosphate), Fludarabine Phosphate, Fluoroplex (Fluorouracil), Fluorouracil, Folex (Methotrexate), Folex PFS (Methotrexate), Folfiri, Folfiri-Bevacizumab, Folfiri-Cetuximab, Folfirinox, Folfox, Folotyn (Pralatrexate), FU-LV, Fulvestrant, Gardasil (Recombinant HPV Quadrivalent Vaccine), Gazyva (Obinutuzumab), Gefitinib, Gemcitabine Hydrochloride, Gemcitabine-Cisplatin, Gemcitabine-Oxaliplatin, Gemtuzumab Ozogamicin, Gemzar (Gemcitabine Hydrochloride), Gilotrif (Afatinib Dimaleate), Gleevec (Imatinib Mesylate), Glucarpidase, Goserelin Acetate, Halaven (Eribulin Mesylate), Herceptin (Trastuzumab), HPV Bivalent Vaccine, Recombinant, HPV Quadrivalent Vaccine, Recombinant, Hycamtin (Topotecan Hydrochloride), Hyper-CVAD, Ibritumomab Tiuxetan, Ibrutinib, ICE, Iclusig (Ponatinib Hydrochloride), Ifex (Ifosfamide), Ifosfamide, Ifosfamidum (Ifosfamide), Imatinib Mesylate, Imbruvica (Ibrutinib), Imiquimod, Inlyta (Axitinib), Intron A (Recombinant Interferon Alfa-2b), Iodine 131 Tositumomab and Tositumomab, Ipilimumab, Iressa (Gefitinib), Irinotecan Hydrochloride, Istodax (Romidepsin), Ixabepilone, Ixempra (Ixabepilone), Jakafi (Ruxolitinib Phosphate), Jevtana (Cabazitaxel), Kadcyla (Ado-Trastuzumab Emtansine), Keoxifene (Raloxifene Hydrochloride), Kepivance (Palifermin), Kyprolis (Carfilzomib), Lapatinib Ditosylate, Lenalidomide, Letrozole, Leucovorin Calcium, Leukeran (Chlorambucil), Leuprolide Acetate, Levulan (Aminolevulinic Acid), Linfolizin (Chlorambucil), LipoDox (Doxorubicin Hydrochloride Liposome), Liposomal Cytarabine, Lomustine, Lupron (Leuprolide Acetate), Lupron Depot (Leuprolide Acetate), Lupron Depot-Ped (Leuprolide Acetate), Lupron Depot-3 Month (Leuprolide Acetate), Lupron Depot-4 Month (Leuprolide Acetate), Marqibo (Vincristine Sulfate Liposome), Matulane (Procarbazine Hydrochloride), Mechlorethamine Hydrochloride, Megace (Megestrol Acetate), Megestrol Acetate, Mekinist (Trametinib), Mercaptopurine, Mesna, Mesnex (Mesna), Methazolastone (Temozolomide), Methotrexate, Methotrexate LPF (Methotrexate), Mexate (Methotrexate), Mexate-AQ (Methotrexate), Mitomycin C, Mitozytrex (Mitomycin C), MOPP, Mozobil (Plerixafor), Mustargen (Mechlorethamine Hydrochloride), Mutamycin (Mitomycin C), Myleran (Busulfan), Mylosar (Azacitidine), Mylotarg (Gemtuzumab Ozogamicin), Nanoparticle Paclitaxel (Paclitaxel Albumin-stabilized Nanoparticle Formulation), Navelbine (Vinorelbine Tartrate), Nelarabine, Neosar (Cyclophosphamide), Neupogen (Filgrastim), Nexavar (Sorafenib Tosylate), Nilotinib, Nolvadex (Tamoxifen Citrate), Nplate (Romiplostim), Obinutuzumab, Ofatumumab, Omacetaxine Mepesuccinate, Oncaspar (Pegaspargase), Ontak (Denileukin Diftitox), OEPA, OPPA, Oxaliplatin, Paclitaxel, Paclitaxel Albumin-stabilized Nanoparticle Formulation, Palifermin, Palonosetron Hydrochloride, Pamidronate Disodium, Panitumumab, Paraplat (Carboplatin), Paraplatin (Carboplatin), Pazopanib Hydrochloride, Pegaspargase, Peginterferon Alfa-2b, PEG-Intron (Peginterferon Alfa-2b), Pemetrexed Disodium, Perjeta (Pertuzumab), Pertuzumab, Platinol (Cisplatin), Platinol-AQ (Cisplatin), Plerixafor, Pomalidomide, Pomalyst (Pomalidomide), Ponatinib Hydrochloride, Pralatrexate, Prednisone, Procarbazine Hydrochloride, Proleukin (Aldesleukin), Prolia (Denosumab), Promacta (Eltrombopag Olamine), Provenge (Sipuleucel-T), Purinethol (Mercaptopurine), Radium 223 Dichloride, Raloxifene Hydrochloride, Rasburicase, R-CHOP, R-CVP, Recombinant HPV Bivalent Vaccine, Recombinant HPV Quadrivalent Vaccine, Recombinant Interferon Alfa-2b, Regorafenib, Revlimid (Lenalidomide), Rheumatrex (Methotrexate), Rituxan (Rituximab), Rituximab, Romidepsin, Romiplostim, Rubidomycin (Daunorubicin Hydrochloride), Ruxolitinib Phosphate, Sclerosol Intrapleural Aerosol (Talc), Sipuleucel-T, Sorafenib Tosylate, Sprycel (Dasatinib), Stanford V, Sterile Talc Powder (Talc), Steritalc (Talc), Stivarga (Regorafenib), Sunitinib Malate, Sutent (Sunitinib Malate), Sylatron (Peginterferon Alfa-2b), Synovir (Thalidomide), Synribo (Omacetaxine Mepesuccinate), Tafinlar (Dabrafenib), Talc, Tamoxifen Citrate, Tarabine PFS (Cytarabine), Tarceva (Erlotinib Hydrochloride), Targretin (Bexarotene), Tasigna (Nilotinib), Taxol (Paclitaxel), Taxotere (Docetaxel), Temodar (Temozolomide), Temozolomide, Temsirolimus, Thalidomide, Thalomid (Thalidomide), Toposar (Etoposide), Topotecan Hydrochloride, Toremifene, Torisel (Temsirolimus), Tositumomab and 1131 Iodine Tositumomab, Totect (Dexrazoxane Hydrochloride), Trametinib, Trastuzumab, Treanda (Bendamustine Hydrochloride), Trisenox (Arsenic Trioxide), Tykerb (Lapatinib Ditosylate), Vandetanib, VAMP, Vectibix (Panitumumab), VeIP, Velban (Vinblastine Sulfate), Velcade (Bortezomib), Velsar (Vinblastine Sulfate), Vemurafenib, VePesid (Etoposide), Viadur (Leuprolide Acetate), Vidaza (Azacitidine), Vinblastine Sulfate, Vincasar PFS (Vincristine Sulfate), Vincristine Sulfate, Vincristine Sulfate Liposome, Vinorelbine Tartrate, Vismodegib, Voraxaze (Glucarpidase), Vorinostat, Votrient (Pazopanib Hydrochloride), Wellcovorin (Leucovorin Calcium), Xalkori (Crizotinib), Xeloda (Capecitabine), XELOX, Xgeva (Denosumab), Xofigo (Radium 223 Dichloride), Xtandi (Enzalutamide), Yervoy (Ipilimumab), Zaltrap (Ziv-Aflibercept), Zelboraf (Vemurafenib), Zevalin (Ibritumomab Tiuxetan), Zinecard (Dexrazoxane Hydrochloride), Ziv-Aflibercept, Zoladex (Goserelin Acetate), Zoledronic Acid, Zolinza (Vorinostat), Zometa (Zoledronic Acid), and Zytiga (Abiraterone Acetate).

In some examples, the anti-cancer drug comprises doxorubicin.

In some embodiments, the pharmaceutical composition comprises a drug that promotes wound healing or vascularization. In some examples, the pharmaceutical composition comprises a drug that reduces ischemia, e.g., due to peripheral artery disease (PAD) or damaged myocardial tissues due to myocardial infarction. For example, the drug comprises a protein or fragment thereof, e.g., a growth factor or angiogenic factor, such as vascular endothelial growth factor (VEGF), e.g., VEGFA, VEGFB, VEGFC, or VEGFD, and/or IGF, e.g., IGF-1, fibroblast growth factor (FGF), angiopoietin (ANG) (e.g., Ang1 or Ang2), matrix metalloproteinase (MMP), delta-like ligand 4 (DLL4), or combinations thereof. These drugs that promote wound healing or vascularization are non-limiting, as the skilled artisan would be able to readily identify other drugs that promote wound healing or vascularization.

In some examples, the pharmaceutical composition comprises an anti-proliferative drug, e.g., mycophenolate mofetil (MMF), azathioprine, sirolimus, tacrolimus, paclitaxel, biolimus A9, novolimus, myolimus, zotarolimus, everolimus, or tranilast. These anti-proliferative drugs are non-limiting, as the skilled artisan would be able to readily identify other anti-proliferative drugs.

In some embodiments, the pharmaceutical composition comprises an anti-inflammatory drug, e.g., corticosteroid anti-inflammatory drugs (e.g., beclomethasone, beclometasone, budesonide, flunisolide, fluticasone propionate, triamcinolone, methylprednisolone, prednisolone, or prednisone); or non-steroidal anti-inflammatory drugs (NSAIDs) (e.g., acetylsalicylic acid, diflunisal, salsalate, choline magnesium trisalicylate, ibuprofen, dexibuprofen, naproxen, fenoprofen, ketoprofen, dexketoprofen, fluribiprofen, oxaprozin, loxoprofen, indomethacin, tolmetin, sulindac, etodolac, ketorolac, diclofenac, aceclofenac, nabumetone, piroxicam, meloxicam, tenoxicam, droxicam, lornoxicam, isoxicam, mefenamic acid, meclofenamic acid, flufenamic acid, tolfenamic acid, celecoxib, rofecoxib, valdecoxib, parecoxib, lumiracoxib, etoricoxib, firocoxib, nimesulide, licofelone, H-harpaide, or lysine clonixinate). These anti-inflammatory drugs are non-limiting, as the skilled artisan would be able to readily identify other anti-inflammatory drugs.

In some examples, the pharmaceutical composition comprises a drug that prevents or reduces transplant rejection, e.g., an immunosuppressant. Exemplary immunosuppressants include calcineurin inhibitors (e.g., cyclosporine, Tacrolimus (FK506)); mammalian target of rapamycin (mTOR) inhibitors (e.g., rapamycin, also known as Sirolimus); antiproliferative agents (e.g.,azathioprine, mycophenolate mofetil, mycophenolate sodium); antibodies (e.g., basiliximab, daclizumab, muromonab); corticosteroids (e.g., prednisone). These drugs that prevent or reduce transplant rejection are non-limiting, as the skilled artisan would be able to readily identify other drugs that prevent or reduce transplant rejection.

In some cases, the pharmaceutical composition comprises an anti-thrombotic drug, e.g., an anti-platelet drug, an anticoagulant drug, or a thrombolytic drug.

Exemplary anti-platelet drugs include an irreversible cyclooxygenase inhibitor (e.g., aspirin or triflusal); an adenosine diphosphate (ADP) receptor inhibitor (e.g., ticlopidine, clopidogrel, prasugrel, or tricagrelor); a phosphodiesterase inhibitor (e.g., cilostazol); a glycoprotein IIB/IIIA inhibitor (e.g., abciximab, eptifibatide, or tirofiban); an adenosine reuptake inhibitor (e.g., dipyridamole); or a thromboxane inhibitor (e.g., thromboxane synthase inhibitor, a thromboxane receptor inhibitor, such as terutroban). These anti-platelet drugs are non-limiting, as the skilled artisan would be able to readily identify other anti-platelet drugs.

Exemplary anticoagulant drugs include coumarins (e.g., warfarin, acenocoumarol, phenprocoumon, atromentin, brodifacoum, or phenindione); heparin and derivatives thereof (e.g., heparin, low molecular weight heparin, fondaparinux, or idraparinux); factor Xa inhibitors (e.g., rivaroxaban, apixaban, edoxaban, betrixaban, darexaban, letaxaban, or eribaxaban); thrombin inhibitors (e.g., hirudin, lepirudin, bivalirudin, argatroban, or dabigatran); antithrombin protein; batroxobin; hementin; and thrombomodulin. These anticoagulant drugs are non-limiting, as the skilled artisan would be able to readily identify other anticoagulant drugs.

Exemplary thrombolytic drugs include tissue plasminogen activator (t-PA) (e.g., alteplase, reteplase, or tenecteplase); anistreplase; streptokinase; or urokinase.

In other examples, the pharmaceutical composition comprises a drug that prevents restenosis, e.g., an anti-proliferative drug, an anti-inflammatory drug, or an anti-thrombotic drug. Exemplary anti-proliferative drugs, anti-inflammatory drugs, and anti-thrombotic drugs are described herein.

In some embodiments, the pharmaceutical composition comprises a drug that treats or prevents infection, e.g., an antibiotic. Suitable antibiotics include, but are not limited to, beta-lactam antibiotics (e.g., penicillins, cephalosporins, carbapenems), polymyxins, rifamycins, lipiarmycins, quinolones, sulfonamides, macrolides lincosamides, tetracyclines, aminoglycosides, cyclic lipopeptides (e.g., daptomycin), glycylcyclines (e.g., tigecycline), oxazonidinones (e.g., linezolid), and lipiarmycines (e.g., fidazomicin). For example, antibiotics include erythromycin, clindamycin, gentamycin, tetracycline, meclocycline, (sodium) sulfacetamide, benzoyl peroxide, and azelaic acid. Suitable penicillins include amoxicillin, ampicillin, bacampicillin, carbenicillin, cloxacillin, dicloxacillin, flucloxacillin, mezlocillin, nafcillin, oxacillin, penicillin g, penicillin v, piperacillin, pivampicillin, pivmecillinam, and ticarcillin. Exemplary cephalosporins include cefacetrile, cefadroxil, cephalexin, cefaloglycin, cefalonium, cefaloridine, cefalotin, cefapirin, cefatrizine, cefazaflur, cefazedone, cefazolin, cefradine, cefroxadine, ceftezole, cefaclor, cefamandole, cefmetazole, cefonicid, cefotetan, cefoxitin, cefprozil, cefuroxime, cefuzonam, cfcapene, cefdaloxime, cefdinir, cefditoren, cefetamet, cefixime, cefmenoxime, cefodizime, cefotaxime, cefpimizole, cefpodoxime, cefteram, ceftibuten, ceftiofur, ceftiolene, ceftizoxime, ceftriaxone, ceftazidime, cefclidine, cefepime, ceflurprenam, cefoselis, cefozopran, cefpirome, cequinome, ceftobiprole, ceftaroline, cefaclomezine, cefaloram, cefaparole, cefcanel, cefedrlor, cefempidone, cefetrizole, cefivitril, cefmatilen, cefmepidium, cefovecin, cefoxazole, cefrotil, cefsumide, cefuracetime, and ceftioxide. Monobactams include aztreonam. Suitable carbapenems include imipenem/cilastatin, doripenem, meropenem, and ertapenem. Exemplary macrolides include azithromycin, erythromycin, larithromycin, dirithromycin, roxithromycin, and telithromycin. Lincosamides include clindamycin and lincomycin. Exemplary streptogramins include pristinamycin and quinupristin/dalfopristin. Suitable aminoglycoside antibiotics include amikacin, gentamycin, kanamycin, neomycin, netilmicin, paromomycin, streptomycin, and tobramycin. Exemplary quinolones include flumequine, nalidixic acid, oxolinic acid, piromidic acid, pipemidic acid, rosoxacin, ciprofloxacin, enoxacin, lomefloxacin, nadifloxacin, norfloxacin, ofoxacin, pefloxacin, rufloxacin, balofloxacin, gatifloxacin, repafloxacin, levofloxacin, moxifloxacin, pazufloxacin, sparfloxacin, temafloxacin, tosufloxacin, besifloxacin, clinafoxacin, gemifloxacin, sitafloxacin, trovafloxacin, and prulifloxacin. Suitable sulfonamides include sulfamethizole, sulfamethoxazole, and trimethoprim-sulfamethoxazone. Exemplary tetracyclines include demeclocycline, doxycycline, minocycline, oxytetracycline, tetracycline, and tigecycline. Other antibiotics include chloramphenicol, metronidazole, tinidazole, nitrofurantoin, vancomycin, teicoplanin, telavancin, linezolid, cycloserine, rifampin, rifabutin, rifapentin, bacitracin, polymyxin B, viomycin, and capreomycin. The skilled artisan could readily identify other antibiotics useful in the devices and methods described herein.

In some embodiments, the pharmaceutical composition comprises a drug that reduces macular degeneration. One common current treatment for macular degeneration involves the injection of anti-angiogenesis compounds intraocularly (Lucentis, Eylea). The repeated intraocular injections are sometimes poorly tolerated by patients, leading to low patient compliance. As described herein, the ability to noninvasively refill drug depots for macular degeneration significantly improves patient compliance and patient tolerance of disease, e.g., macular degeneration, treatment. Controlled, repeated release made possible by device refilling allows for fewer drug dosings and improved patient comfort.

In some embodiments, the pharmaceutical composition comprises a drug that prevents immunological rejection. Prior to the invention described herein, to prevent immunological rejection of cells, tissues or whole organs, patients required lifelong therapy of systemic anti-rejection drugs that cause significant side effects and deplete the immune system, leaving patients at greater risk for infection and other complications. The ability to locally release anti-rejection drugs from a drug delivery device and to repeatedly refill the drug delivery device allows for more local anti-rejection therapy with fewer systemic side effects, improved tolerability and better efficacy.

In some embodiments, the pharmaceutical composition comprises a drug that prevents thrombosis. Some vascular devices such as vascular grafts and coated stents suffer from thrombosis, in which the body mounts a thrombin-mediated response to the devices. Anti-thrombotic drugs, released from these devices, allows for temporary inhibition of the thrombosis process, but the devices have limited drugs and cannot prevent thrombosis once the drug supply is exhaused. Since these devices are implanted for long periods of time (potentially for the entire lifetime of the patient), temporary thrombosis inhibition is not sufficient. The ability to repeatedly refill these devices with anti-thrombotic drugs and release the drug significantly improves clinical outcomes and allows for long-term thrombosis inhibition.

In some embodiments, the pharmaceutical composition comprises a drug that treats inflammation. Chronic inflammation is characterized by persistent inflammation due to nondegradable pathogens, viral infections, or autoimmune reactions and can last years and lead to tissue destruction, fibrosis, and necrosis. In some cases, inflammation is a local disease, but clinical interventions are almost always systemic. Anti-inflammatory drugs given systemically have significant side-effects including gastrointestinal problems, cardiotoxicity, high blood pressure and kidney damage, allergic reactions, and possibly increased risk of infection. The ability to repeatedly release anti-inflammatory drugs such as NSAIDs and COX-2 inhibitors could reduce these side effects. Refillable drug delivery devices implanted or injected at a site of inflammation creates the ability to deliver long term and local anti-inflammatory care while avoiding systemic side effects.

In some embodiments, the polymer of the device and/or drug refill comprises a polypeptide and/or a polysaccharide. In some examples, the polymer of the device and/or the drug refill comprises a polymer selected from the group consisting of collagen, alginate, polysaccharides, polyethylene glycol (PEG), poly(glycolide) (PGA), poly(L-lactide) (PLA), or poly(lactide-co-glycolide) (PLGA), and poly lactic-coglycolic acid. In a preferred embodiment, the polymer of the device and/or the polymer of the drug refill comprise an alginate.

In some cases, the polymer of the device is in the form of hydrogel, e.g., an alginate hydrogel. In some examples, the alginate is modified by a cell adhesion peptide, e.g., RGD. In some examples, the polymer of the refill comprises alginate, e.g., modified by a cell adhesion peptide, e.g., RGD.

In some examples, the polymer, e.g., alginate, such as alginate hydrogel, degrades over time, e.g., in vivo. For example, the degradation rate is controllable by tuning the temperature, pH, hydration status, porosity, molecular weight, degree of oxidation, and/or cross-linking density of the polymer, e.g., alginate.

In some cases, the polymer of the device has a higher molecular weight (MW) than the polymer of the refill. For example, the polymer of the device comprises more than one strand of a polymer, e.g., alginate. In some cases, the polymer, e.g., alginate, strands are cross-linked. For example, the polymer is cross-linked by a cation, e.g., a divalent or trivalent cation, such as Ca²⁺.

In some embodiments, the polymer of the refill comprises one strand of a polymer, e.g., alginate. In some cases, the polymer of the refill comprises a free polymer, e.g., alginate, strand, i.e., the polymer does not comprise cross-linked polymer strands, e.g., alginate strands.

For example, the polymer of the refill, e.g., comprising alginate, has a MW of 1000 kDa or less, e.g., 1000 kDa, 900 kDa, 800 kDa, 700 kDa, 600 kDa, 500 kDa, 400 kDa, 350 kDa, 325 kDa, 300 kDa, 290 kDa, 285 kDa, 280 kDa, 275, kDa, 270 kDa, 260 kDa, 250 kDa, 240 kDa, 230 kDa, 220 kDa, 210 kDa, 200 kDa, 180 kDa, 160 kDa, 150 kDa, 140 kDa, 120 kDa, 100 kDa, or less.

In other examples, the polymer of the refill, e.g., comprising alginate, has a hydrodynamic radius (Rh) of 200 nm or less, e.g., 200 nm, 180 nm, 160 nm, 140 nm, 120 nm, 100 nm, 90 nm, 80 nm, 70 nm, 60 nm, 50 nm, 44 nm, 40 nm, 35 nm, 30 nm, 25 nm, 20 nm, 17 nm, 15 nm, 12 nm, 10 nm, or less. The hydrodynamic radius is measured by using standard methods in the art.

In some embodiments, the polymer of the refill is conjugated, e.g., via a covalent bond, to the target molecule. In some embodiments, the polymer of the device is conjugated, e.g., via a covalent bond, to the target recognition moiety. In some cases, the polymer, e.g., alginate, is conjugated to a nucleic acid at the 3' end, the 5' end, or in the middle of the nucleic acid. For example, the polymer, e.g., alginate, is conjugated to the 3' end of a nucleic acid molecule, e.g., DNA.

Described herein is a kit for drug delivery comprising a drug delivery device and a drug refill, where the drug delivery device comprises a material, a pharmaceutical composition, and a target recognition moiety. Suitable materials include a polymer, a protein, a hydrogel (e.g., a synthetic hydrogel or a biological hydrogel), an organogel, a ceramic, a composite, a metal, a wood, or a glass material. The drug refill comprises a pharmaceutical composition and a target. Optionally, the drug refill further comprises a nanocarrier, e.g., a polymer. The target and the target recognition moiety form a two-component binding pair. The drug refill is mobile until the target on the drug refill binds to the target recognition moiety on the drug delivery device, and upon binding of the target to the target recognition moiety, the drug refill delivers the pharmaceutical composition to the drug delivery device, thereby refilling the drug delivery device.

The invention also features the refillable drug delivery system according to the invention for use in refilling a drug delivery device *in vivo,* wherein i) the drug delivery device is for administration to the subject and implant at a desired location within the subject; and ii) the drug refill is for subsequent administration to the subject; optionally wherein the drug refill is for subsequent administration to the subject at a site located away from the device; wherein the drug refill is mobile within the subject until the target molecule on the drug refill binds to the target recognition molecule on the drug delivery device at the desirable location within the subject; and the target recognition molecule and the target recognition molecule form a two-component binding pair; and wherein upon binding of the target molecule to the target recognition molecule, the cleavable linker is cleaved and the drug refill delivers the pharmaceutical composition to the drug delivery device, thereby refilling the drug delivery device. The drug is subsequently released from the drug delivery device.

In accordance with the systems for use described herein, a refill is for administration orally, intraperitoneally, intravenously, intraarterially, rectally, buccally, sublingually, intraocularly, intranasally, transdermally, transmucosally, subcutaneously, intramuscularly, epidurally, intracranially, intracerebrally, percutaneously, intravaginally, intrauterineally, intravitreally, via injection, via aerosol-based delivery, or via implantation.

The methods described herein also include refilling a drug delivery device with magnetic-based refilling. For example, the target and the target recognition moiety, e.g., molecule form a magnetic two-component binding pair. Alternatively, the device and the target form a magnetic two-component binding pair or the device and the pharmaceutical composition form a magnetic two-component binding pair. In some cases, the two-component binding pair is intrinsically magnetic. Alternatively, a magnetic field is applied to the drug delivery device to magnetize the drug delivery device prior to administering the magnetic drug refill.

In some examples, the system for use further comprises repeating step ii) as described above for at least 1, 2, 3, 4, 5, 6, 7, 8, 9, 10, or more times, e.g., indefinitely.

For example, the drug refill is for subsequent administration at a site that is located at least 5 cm (e.g., at least 5 cm, 10 cm, 15 cm, 20 cm, 30 cm, 40 cm, 50 cm, 1 m, 1.5 m, 2 m, 2.5 m, 3 m, or more) away from the center of the device.

In some embodiments, the time interval between administration of the drug delivery device and administration of a drug refill is at least 1 day, e.g., at least 1, 2, 3, 4, 5, 6, or 7 days, 1, 2, 3, or 4 weeks, 1, 2, 3, 4, 5, 6, 7, 8, 9, 10, 11, or 12 months, 1, 2, 3, 4, 5, 6, 7, 8, 9, or 10 years, or longer. In some cases, the time interval between administration of a drug refill and a subsequent refill is at least 1 day, e.g., at least 1, 2, 3, 4, 5, 6, or 7 days, 1, 2, 3, or 4 weeks, 1, 2, 3, 4, 5, 6, 7, 8, 9, 10, 11, or 12 months, 1, 2, 3, 4, 5, 6, 7, 8, 9, or 10 years, or longer.

The invention also provides the refillable drug delivery system according to the invention for use in maintaining or reducing the size of a tumor in a subject, wherein the pharmaceutical composition comprises an anti-cancer drug; optionally wherein the drug delivery device is refilled a plurality of times by administration of the drug refill; optionally
wherein the drug refill is for administration orally, buccally, sublingually, rectally, intravenously, intra-arterially, intraosseously, intra-muscularly, intracerebrally, intracerebroventricularly, intrathecally, subcutaneously, intraperitoneally, intraocularly, intranasally, transdermally, epidurally, intracranially, percutaneously, intravaginally, intrauterineally, intravitreally, transmucosally, or via injection, via aerosol-based delivery, or via implantation; optionally wherein the anti-cancer drug comprises doxorubicin; optionally wherein the subject suffers from a cancer which is a solid cancer or a hematological cancer.

Also provided by the invention is the refillable drug delivery system according to the invention for use in reducing cancer progression in a subject in need thereof, wherein the pharmaceutical composition comprises an anti-cancer drug; optionally wherein (i) the drug delivery device is refilled by administering the drug refill to the subject orally, intraperitoneally, intravenously, or intra-arterially;
(ii) optionally repeating step (i);
thereby reducing cancer progression in the subject.

In accordance with the methods described herein, the device may be administered into a tumor void space, e.g., during tumor resection. The device may be administered into a tumor, or near (e.g., within 10 cm or less from a perimeter, e.g., 10 cm, 5 cm, 2.5 cm, 1 cm, 5 mm, 2.5 mm, 1 mm, or less from a perimeter) of a tumor.

The size of the tumor may be maintained, i.e., the size of the tumor after administration of the anti-cancer drug remains within 10% of the size of the tumor prior to administration of the anti-cancer drug. In some examples, the size of the tumor is reduced by at least 1.5-fold, e.g., at least 1.5-fold, 2-fold, 3-fold, 4-fold, 5-fold, 6-fold, 7-fold, 8-fold, 9-fold, 10-fold, 20-fold, 50-fold, 100-fold, or more compared to the size of the tumor prior to administration of the device and/or a refill. The methods/devices described herein may completely eliminate a tumor in the subject. The size of a tumor may be measured by the area or diameter of the tumor, e.g., on an X-ray, positron emission tomograph (PET) scan, magnetic resonance imaging (MRI), or computed tomograph (CT) scan.

In other examples, the methods described herein are effective to slow cancer progression and/or tumor growth. For example, a tumor growth rate is reduced by at least 1.5-fold, e.g., at least 1.5-fold, 2-fold, 3-fold, 4-fold, 5-fold, 6-fold, 7-fold, 8-fold, 9-fold, 10-fold, 20-fold, 50-fold, 100-fold, or more, compared to the growth rate prior to administration of a device or refill described herein. In some cases, the methods described herein stop cancer progression and/or tumor growth completely.

In some cases, an anti-cancer drug includes a small molecule, a peptide or polypeptide, a protein or fragment thereof (e.g., an antibody or fragment thereof), or a nucleic acid.

Exemplary anti-cancer drugs are described above. In an embodiment, the anti-cancer drug comprises doxorubicin.

In accordance with some systems for use of the invention, the subject suffers from a cancer. For example, the cancer is a solid cancer of a hematological cancer. Exemplary solid cancers include but are not limited to melanoma (e.g., unresectable, metastatic melanoma), renal cancer (e.g., renal cell carcinoma), prostate cancer (e.g., metastatic castration resistant prostate cancer), ovarian cancer (e.g., epithelial ovarian cancer, such as metastatic epithelial ovarian cancer), breast cancer (e.g., triple negative breast cancer), glioblastoma, and lung cancer (e.g., non-small cell lung cancer). Exemplary hematological cancers include leukemia or lymphoma. For example, a leukemia is acute lymphoblastic leukemia (ALL), acute myelogenous leukemia (AML), chronic lymphocytic leukemia (CLL), small lymphocytic lymphoma (SLL), chronic myelogenous leukemia (CML), or acute monocytic leukemia (AMoL). For example, a lymphoma is follicular lymphoma, Hodgkin's lymphoma (e.g., Nodular sclerosing subtype, mixed-cellularity subtype, lymphocyte-rich subtype, or lymphocyte depleted subtype), or Non-Hodgkin's lymphoma. In some embodiments, the subject suffers from a non-metastatic cancer.

The invention also provides a refillable drug delivery system for use in promoting wound healing in a subject in need thereof, wherein the pharmaceutical composition promotes angiogenesis and/or maturation or remodeling of an existing blood vessel; optionally wherein
(i) the drug delivery device is refilled by administering the drug refill to the subject;
(ii) optionally repeating step (i);
thereby promoting wound healing in the subject.

In some cases, the drug refill is for administration orally, buccally, sublingually, rectally, intravenously, intra-arterially, intraosseously, intra-muscularly, intracerebrally, intracerebroventricularly, intrathecally, subcutaneously, intraperitoneally, intraocularly, intranasally, transdermally, epidurally, intracranially, percutaneously, intravaginaly, intrauterineally, intravitreally, transmucosally, or via injection, via aerosol-based delivery, or via implantation.

Pharmaceutical compositions that promote angiogenesis and/or maturation or remodeling of an existing blood vessel include but are not limited to vascular endothelial growth factor (VEGF), e.g., VEGFA, VEGFB, VEGFC, or VEGFD, and/or IGF, e.g., IGF-1, fibroblast growth factor (FGF), angiopoietin (ANG) (e.g., Ang1 or Ang2), matrix metalloproteinase (MMP), delta-like ligand 4 (DLL4), and combinations thereof.

The invention also includes a refillable drug delivery system according to the invention for use in reducing or controlling inflammation in a subject, wherein the pharmaceutical composition comprises an anti-inflammatory agent; optionally wherein (i) the drug delivery device is refilled by administering the drug refill to the subject;
(ii) optionally repeating step (i);
thereby reducing or controlling inflammation in the subject. Anti-inflammatory agents are described herein.

In some cases, the drug refill is for administration orally, buccally, sublingually, rectally, intravenously, intra-arterially, intraosseously, intra-muscularly, intracerebrally, intracerebroventricularly, intrathecally, subcutaneously, intraperitoneally, intraocularly, intranasally, transdermally, epidurally, intracranially, percutaneously, intravaginaly, intrauterineally, intravitreally, transmucosally, or via injection, via aerosol-based delivery, or via implantation.

Described herein is a method of preventing or reducing restenosis in a subject in need thereof, comprising the steps of:
i) administering a drug delivery device to the subject, wherein the pharmaceutical composition comprises an anti-inflammatory agent, an anti-proliferative agent, and/or an anti-thrombotic agent;
ii) subsequently administering a refill described herein to the subject orally, intraperitoneally, intravenously, or intraarterially;
iii) optionally repeating step ii);
iv) thereby preventing or reducing restenosis in the subject.

Exemplary anti-inflammatory, anti-proliferative, and anti-thrombotic agents are described herein. In some examples, the drug delivery device comprises a medical device, e.g., a stent, graft, or catheter, e.g., a drug-eluting stent, graft, or catheter. For example, a hydrogel of the invention is incorporated into/onto a medical device, e.g., a stent, graft, or catheter, e.g., drug-eluting stent, graft, or catheter. In some examples, a drug-eluting stent, graft, or catheter is coated with a pharmaceutical composition that inhibits tissue growth and/or reduces the risk of (prevents) restenosis, e.g., restenosis from scar tissue and/or cell proliferation.

Also described herein are vascular stents coated with nondegradable polymer coatings including, but not limited to poly(n-butylmethacrylate), poly(styrene-b-isobutylene-b-styrene), poly(ethylene-co-vinyl acetate), fluoropolymer, and polyphosphorylcholine incorporating a click chemistry motif (including, but not limited to azide, cyclooctyne, norbornene, tetrazine, cyclooctene, etc.) incorporated as a monomer during polymerization or as a cap to the polymer in a subsequent step. Alternatively, the coating could be a degradable polymer such as poly(lactic-co-glycolic acid), poly-L-lactide incorporating a click chemistry building block (including, but not limited to azide, cyclooctyne, norbornene, tetrazine, cyclooctene, etc) building block incorporated during polymerization or as a capping block in a subsequent step.

For example, the pharmaceutical composition, e.g., in/on a medical device, comprises an anti-inflammatory agent, antineoplastic, anti-proliferative agent, migration inhibitor, extracellular matrix (ECM) modulator, healing enhancer, re-endothelialization factor, and/or anti-thrombotic agent as described herein. For example, a refill described herein delivers a pharmaceutical composition, e.g., comprising an anti-inflammatory agent, antineoplastic, anti-proliferative agent, migration inhibitor, extracellular matrix (ECM) modulator, healing enhancer, re-endothelialization factor, and/or anti-thrombotic agent, to the medical device, thereby refilling the medical device.

Exemplary antineoplastics/anti-inflammatory agents include Sirolimus, Tacrolimus, Everolimus, Leflunomide, M-Predinisolone, Dexamethasone, Interferon r-1b, Mycophenolic acid, Mizoribine, Cyclosporine, and Tranilast. Exemplary antiproliferative agents include 7-hexanoyltaxol (QP-2), Taxol (paclitaxel), Actinomycin, Methotraxate, Angiopeptin, Vincristine, Mitomycine, Statins (e.g., Atorvastatin, Cerivastatin, Fluvastatin, Lovastatin, Mevastatin, Pitavastatin, Pravastatin, Rosuvastatin, and Simvastatin), C-myc antisense, Abbott ABT-578 (also called Zotarolimus), RestenASE, 2-choloro-deoxyadenosine, proliferating cell nuclear antigen (PCNA) ribozyme, Biolimus A9, Sirolimus, Novolimus, and Myolimus. Exemplary migration inhibitors/EMC modulators include Batmastat, Prolyl hydroxylase inhibitors (e.g., FG-4539 and FG-2216), Halfunginone, C-proteinase inhibitors, and Probucol. Healing enhancers/re-endothelialization factors include BCP671, vascular endothelial growth factor (VEGF), estradiols, nitric oxide (NO) donor compounds, endothelial progenitor cell (EPC) antibodies, and Biorest. See, e.g., Htay et al. Vasc. Health Risk Manag. 1.4(2005):263-76 at Table 1; and Abizaid et al. Circulation: Cardiovascular Interventions. 3(2010):384-93 at Table 1.

In some examples, the inflammation is chronic, e.g., caused by rheumatoid arthritis.

For example, the subject is a mammal, e.g., human, monkey, primate, dog, cat, horse, cow, pig, sheep, or goat. For example, the subject is a human.

Also described herein is a method of preventing a cardiac infarction in a subject in need thereof, comprising the steps of:
i) administering a drug delivery device to the subject;
ii) subsequently administering a refill described herein to the subject orally, intraperitoneally, intravenously, intraarterially, or via injection directly into the heart; optionally repeating step ii);
thereby preventing a cardiac infarction in the subject.

The device may be administered, e.g., injected or implanted, directly into the heart of the subject. The device may comprise a drug eluting stent; or the device may be coated onto/incorporated into/adhered to a drug eluting stent. The drug delivery device and/or the refill may comprise a pharmaceutical composition described herein, e.g., an anti-inflammatory agent, antineoplastic, anti-proliferative agent, migration inhibitor, extracellular matrix (ECM) modulator, healing enhancer, re-endothelialization factor, and/or anti-thrombotic agent described above. The pharmaceutical composition prevents future myocardial infarction, e.g., by reducing stenosis of blood vessels and reducing restenosis of vessels, e.g., vessels into which a stent has been placed or vessels in subjects that have undergone corrective surgery, e.g., bypass surgery.

Also described herein is a method of preventing or reducing organ/tissue/cell transplant rejection in a subject in need thereof, comprising the steps of:
i) administering a drug delivery device to the subject, wherein the device comprises a pharmaceutical composition comprising an anti-transplant rejection drug;
ii) subsequently administering a refill described herein to the subject orally, intraperitoneally, intravenously, intraarterially, or via injection or implantation;
iii) optionally repeating step ii);
thereby preventing or reducing organ/tissue/cell transplant rejection in the subject.

For example, the device is administered (e.g., implanted or injected) locally at or near the transplant site (e.g., within 10 cm or less from a perimeter, e.g., 10 cm, 5 cm, 2.5 cm, 1 cm, 5 mm, 2.5 mm, 1 mm, or less from a perimeter) of the transplanted organ/tissue. Alternatively, the device is administered in the transplanted organ/tissue. At least two, e.g., 2, 3, 4, 5, 6, 7, 8, 9, or 10, devices may be administered within or around the transplanted organ/tissue. The device and refill may contain an anti-transplant rejection drug, e.g., an immunosuppressant described above.

The invention also provides a refillable drug delivery system for use in treating an eye disease in a subject in need thereof, wherein the pharmaceutical composition treats said eye disease; optionally wherein (i) the drug delivery device is refilled by administering the drug refill to the eye of the subject; optionally (ii) repeating step (i); thereby treating an eye disease in the subject. For example, the eye disease includes cataracts, glaucoma, retinal disease, corneal disease, temporal arteritis, or macular degeneration.

This approach involves delivery of a refillable device to the eye, e.g., topically, or to the back of the eye, e.g., intraocularly, for local delivery. Refillable drug payloads are administered either through injection into the eye, as eye drops, or systemically. The ability to refill drug delivery devices as described herein allows for reduced dosing frequency, improved control over drug release and presentation, and improved disease efficacy.

Described herein are also uses of the drug delivery/refill systems to deliver anti-rejection and immunomodulatory medicines to sites of organ transplant, to deliver immunomodulatory medicines to gels administered (e.g., injected) for arthritis treatment, to deliver antibiotics to sites of implant infection (e.g., such as orthopedic implant-associated
infections), or to deliver antibiotics to devices implanted during surgery for osteomyelitis. For example, orthopedic implants such as any articulating joints (e.g., an artificial joint, e.g., knees, hips), bone screws, and other orthopedic devices are coated with a polymer containing a target recognition moiety and a pharmaceutical composition and refilled with a composition comprising a target molecule and pharmaceutical composition, as described above.

In some cases, the device comprises an orthopedic implant coated with a polymer coating, e.g., polyethylene, which incorporates a click chemistry motif (including, but not limited to azide, cyclooctyne, norbornene, tetrazine, cyclooctene, etc). Alternatively, the orthopedic implant comprises a soft polymer such as a polyethylene articulating cup, incorporating a click chemistry motif, such as a soft knee implant. In this application, refillable drug payloads targeted to the device comprise anti-microbial compounds, e.g., antibiotics.

In another example, the drug delivery device (e.g., polymer) is deposited or implanted at the time of a surgery to remove parts of a bone that is diseased or necrotic or a surgery to repair the damaged bone with insertion of a prosthetic or bone filler substance. For example, the drug delivery device containing the target recognition moiety is in the form of an antibiotic-loaded polymer paste, bone wax, or bone cement that is then recharged/refilled in situ by systemic administration of antibiotics (drug refill composition containing the target molecule) to combat osteomyelitis. In another example, the device comprising a gel or scaffold is implanted or injected into an infected portion of the bone. The device comprises a gel or scaffold modified with click chemistry motifs (including, but not limited to azide, cyclooctyne, norbornene, tetrazine, cyclooctene, etc). In this application, refillable drug payloads targeted to the device comprise anti-microbial compounds, e.g., antibiotics.

Bone cements have been used to anchor articulating joints (e.g., hip joints, knee joints, shoulder joints, and elbow joints) for more than half a century. Articulating joints (referred to as prostheses) are anchored with bone cement. The bone cement fills the free space between the prosthesis and the bone and plays the important role of an elastic zone. Bone cements are provided as two-component materials. Bone cements consist of a powder (i.e., pre-polymerized poly(methyl methacrylate) (PMMA) and/or PMMA or methylmethacrylate (MMA) co-polymer beads and/or amorphous powder, radio-opacifer, or initiator) and a liquid (MMA monomer, stabilizer, or inhibitor). The two components are mixed and a free radical polymerization occurs of the monomer when the initiator is mixed with the accelerator.

In one aspect, the refillable device described herein constitutes a modified version of bone cement in which the prepolymerized PMMA contains a click chemistry motif (including, but not limited to azide, cyclooctyne, norbornene, tetrazine, cyclooctene, etc). Alternatively, click chemistry motifs are incorporated in the liquid component as monomers.

In another example, the drug delivery device (e.g., polymer) is deposited or implanted at the time of surgery to locally provide pain medication. For example, the drug delivery device is placed at the site of surgery to concentrate drug release, including drug refill release, at the tissue site, thereby enabling the use of lower drug doses. In this manner, the incidence of drug/chemical dependency or addiction is decreased.

The invention includes the refillable drug delivery system according to the invention for use in treating arrhythmia in a subject in need thereof, wherein the pharmaceutical composition treats said arrhythmia; optionally wherein (i) the drug delivery device is refilled by administering the drug refill to the heart of the subject; (ii) optionally repeating step (i); thereby treating arrhythmia in the subject.

There are many classes of antiarrhythmic medications with different mechanisms of action and each class comprises many different individual drugs. Although the goal of drug therapy is to prevent arrhythmia, nearly every antiarrhythmic drug has the potential to act as a pro-arrhythmic, and have significant off-target toxicity. Delivery of the drug delivery device described herein reduces this off-target toxicity.

For example, in some cases, the device comprises a gel or scaffold implanted or injected into a site of the heart suffering from arrhythmia, e.g., during a surgical procedure. The device comprises of a gel or scaffold modified with click chemistry motifs (including, but not limited to azide, cyclooctyne, norbornene, tetrazine, cyclooctene, etc.). Refillable drug payloads are targeted to the device. Suitable anti-arrhythmic drugs include, but are not limited to: quinidine, procainamide, disopyramide, lidocaine, phenytoin, mexiletine, tocainide, encainide, flecainide, propafenone, moricizine, carvedilol, propranolol, esmolol, timolol, metoprolol, atenolol, bisoprolol, amiodarone, sotalol, ibutilide, dofetilide, dronedatrone, verapamil, dilitiazem, adenosine, and digoxin.

Also described herein are methods of evaluating patient medication adherence, i.e., the methods described herein are utilized to determine if a patient has complied with instructions, e.g., physician instructions, regarding the administration of a drug, e.g., a prescription drug. For example, methods of evaluating patient medication adherence are carried out by administering, e.g., orally administering, a medication adherence device to a subject in need thereof, wherein the device comprises a target recognition moiety, e.g., molecule. The drug delivery device optionally comprises a material, e.g., a polymer, a protein, a hydrogel (e.g., a synthetic hydrogel or a biological hydrogel), an organogel, a ceramic, a composite, a metal, a wood, or a glass material. Subsequently, a drug comprising a pharmaceutical composition, a target, and optionally, a nanocarrier, e.g., a polymer, is administered to the subject, wherein the target and the target recognition moiety form a two-component binding pair. In some cases, the target comprises a detectable label linked thereto. In other cases, the drug itself comprises a detectable label linked thereto. Suitable detectable labels are selected from the group consisting of a fluorescent dye, a radioactive molecule, and paramagnetic compound. The drug travels to and associates with, e.g., binds to, the device. Finally, the label on/in the device is detected and the level of the label on/in the device is compared to the level of the label on/in the device prior to administration of the drug. Each time the patient administers the drug, the level of the label on the device increases. Thus, determining the level of the label on the device confirms whether the patient has been administering the drug as prescribed - the level of the label is proportional to the total drug administered/taken by the patient.

The invention has certain advantages over existing methods, e.g., those described in Oneto et al. Acta Biomaterialia 10(2014):5099-5105. For example, the drug delivery/refill systems described herein permit the refilling of a device multiple times, the refilling of a device through oral administration, and the refilling of gels not only at a subcutaneous site but also gels resident at disease sites. In addition, the drug delivery/refill systems described herein permit the targeting of multiple (e.g., two or more) different locations with multiple (e.g., two or more) different chemistries, unlike the methods of Oneto et al., which use only one type of chemistry and is able to target only one location.

Other features and advantages of the invention will be apparent from the following description of the preferred embodiments thereof, and from the claims. Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. In addition, the materials, methods, and examples are illustrative only and are not intended to be limiting.

### BRIEF DESCRIPTION OF THE DRAWINGS

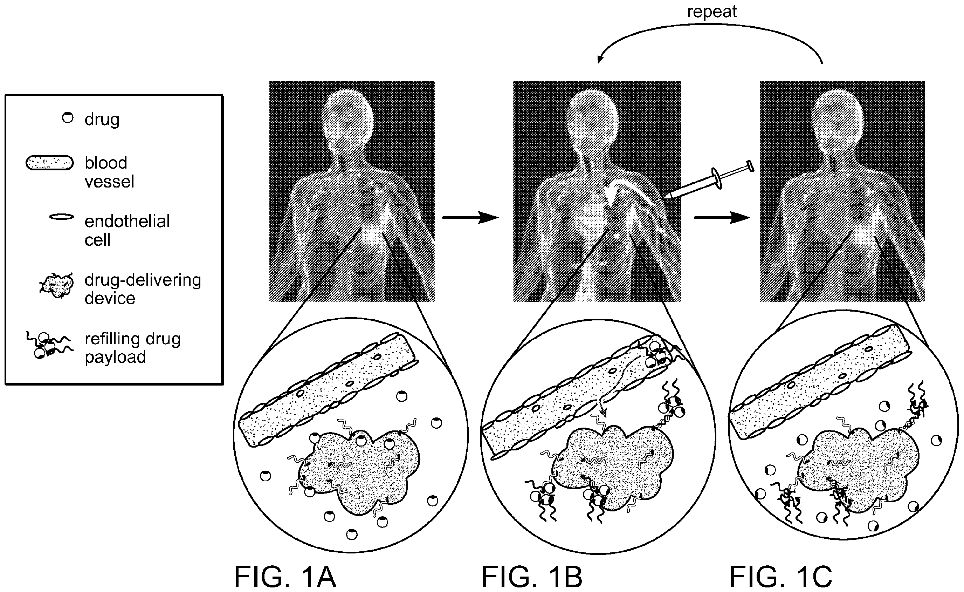

Figure 1A is a schematic for intravenous refilling of drug-delivering devices with therapeutic drugs payloads. A drug-delivering device is implanted into a target tissue site and releases active drug (yellow circles) in a controlled, localized manner. Figure 1B is a schematic showing that intravenously infused drug payload homes to the device site and refills polymer with a fresh depot of drug. Figure 1C is a schematic that shows that IV infused drug cargo binds to gel, and is released over time. IV-mediated drug refilling can be repeated with the same or a different drug payload multiple times.

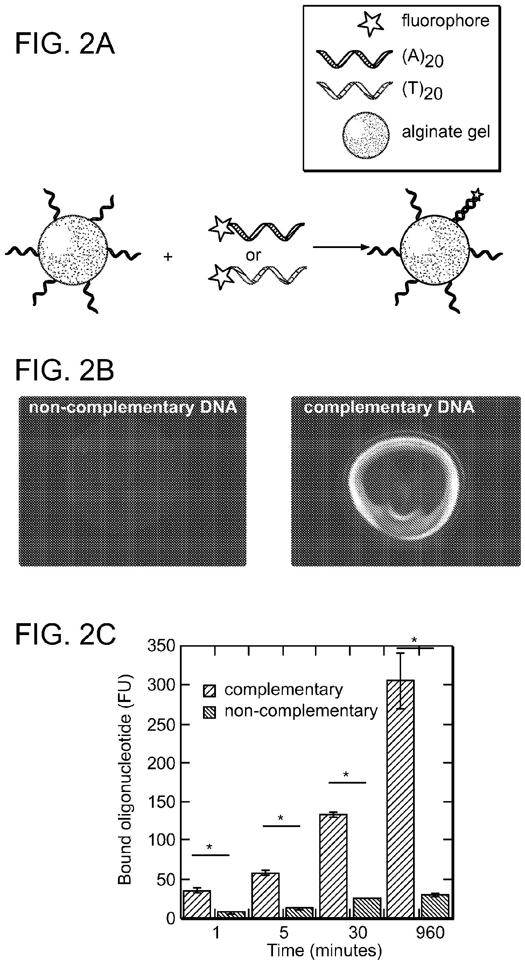

Figure 2A is a schematic showing DNA-conjugated calcium alginate gels incubated for various time periods with fluorescently-labeled complementary or non-complementary DNA. Figure 2B is a set of fluorescent images after 30 minutes of incubation. Figure 2C is a graph showing quantitation of retained fluorescence on beads after variable incubation times and washing away unbound DNA. Values represent mean and S.E.M. * represents p<.05 by Student's t-test. DNA-conjugated calcium-alginate gels retain oligonucleotide-binding properties.

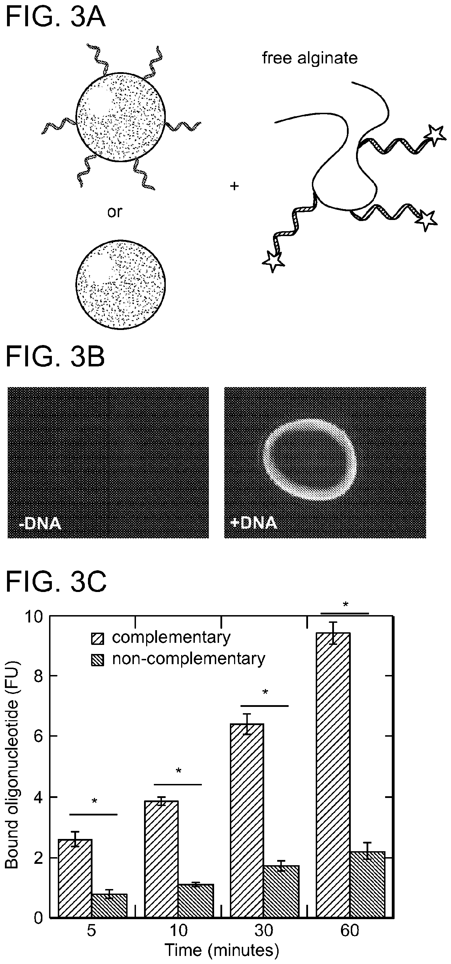

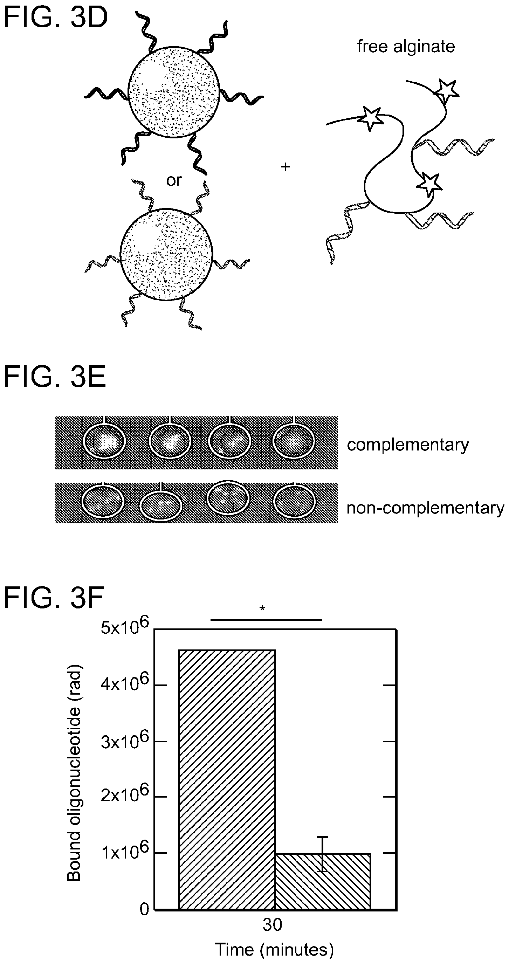

Figure 3A is a schematic showing calcium alginate gels comprised of polymer conjugated to DNA or unconjugated alginate that were incubated for various time periods with free alginate conjugated to fluorescently-labeled complementary DNA. Figure 3B is a set of fluorescent images after 1 hour. Figure 3C is a graph quantifying retained fluorescence on gels after incubation for different periods of time and washing away unbound alginate. Figure 3D is a schematic showing calcium alginate gels comprised of polymer conjugated to complementary or non-complementary DNA that were incubated for 30 mins with free alginate strands conjugated to DNA and near-IR fluorescent tags. Figure 3E is a set of epi-fluorescence images of gels after washing away unbound alginate. Figure 3F is a graph quantifying retained fluorescence on gels after washing away unbound alginate. Values represent mean and S.E.M. * represents p<.05 by Student's t-test. DNA-conjugated alginate gels selectively bind free alginate strands conjugated to complementary DNA.

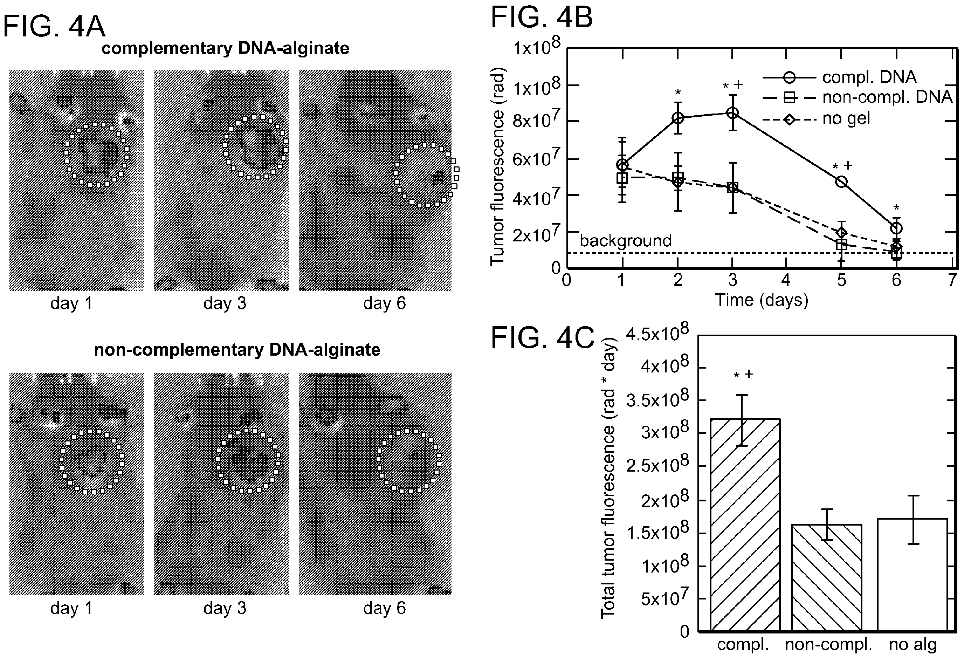

Figure 4A is a set of images of fluorescence in a tumor (yellow circles indicate tumor location). C57/B6 mice bearing B16/F10 melanoma tumors were injected intra-tumorally with alginate carrying complementary DNA, non-complementary DNA or no alginate. Homing to the tumor was tested through IV injection of a fluorescently-labeled alginate bearing DNA. Figure 4B is a graph quantifying the fluorescence in the tumor. Figure 4C is a graph showing the alginate residence at the tumor site, quantified by integrating area under the curve over the 6-day period for each experimental group. Values represent mean and S.E.M. (*) represents p<.05 vs. non-complementary and (⁺) p<.05 vs. no alg controls by Student's t-test. DNA mediated homing of alginate polymers to intra-tumor gels.

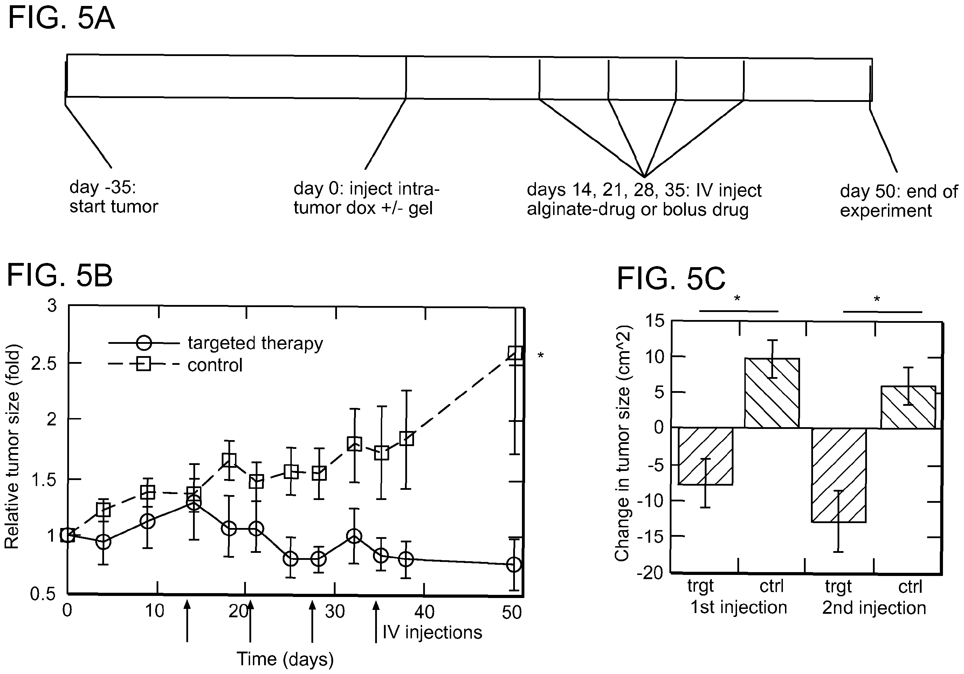

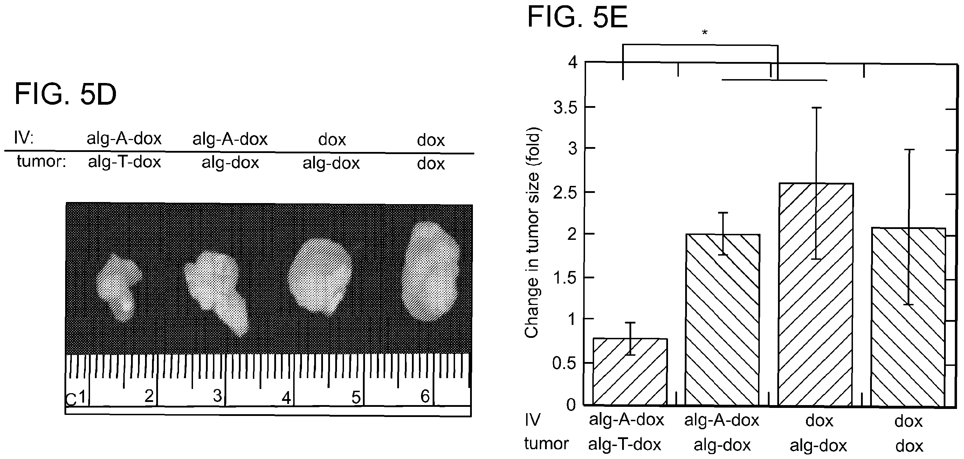

Figure 5A is a schematic showing the timeframe of experimental design. J:Nu mice were injected with human MDA-MB-231 breast cancer cells at day -35. Tumors were subsequently injected at day 0 with gels conjugated to DNA, control gels, or PBS with 80ug doxorubicin. At 2, 3, 4, and 5 weeks after gel injection, animals were submitted to IV injections of doxorubicin conjugated to alginate and DNA or bolus doxorubicin controls. Figure 5B is a graph showing the tumor sizes as they were monitored over 7 weeks after gel injection for targeted and control groups. Figure 5C is a graph showing the 3-day change in tumor size after each IV injection. Figure 5D is a photograph of median-sized tumors.
Figure 5E is a graph tumor growth seven weeks after intra-tumor injection in fully targeted alg-DNA-dox system or control systems with an alg-DNA-dox system incapable of DNA-mediated homing, bolus IV injections or bolus IV and intra-tumor injections. Tumor sizes were normalized to size at day 0. Values represent mean and S.E.M. N>5. * denotes statistical significance by Student's t-test. Drug refilling system led to arrest in tumor growth in vivo.

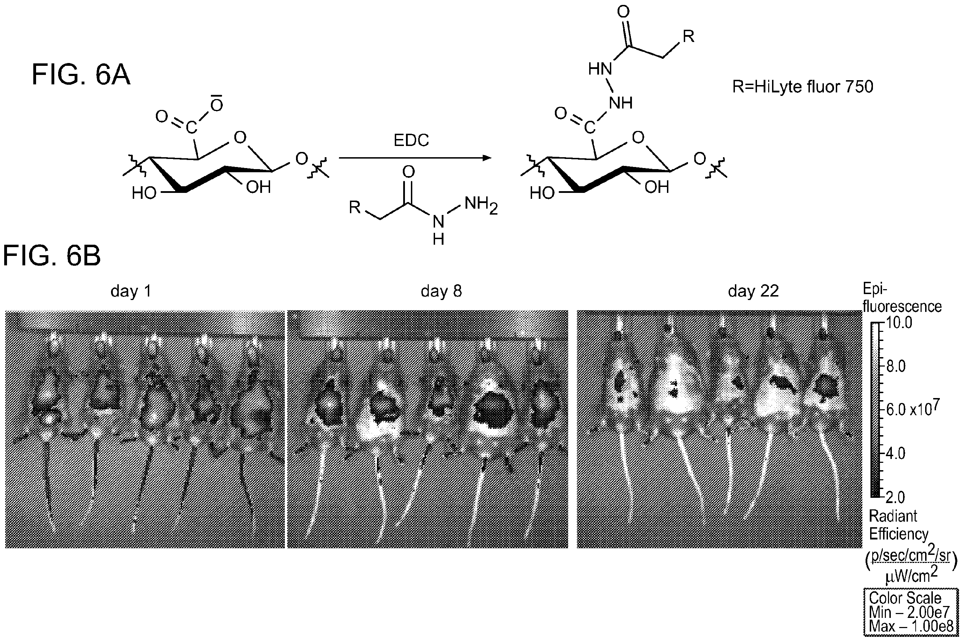

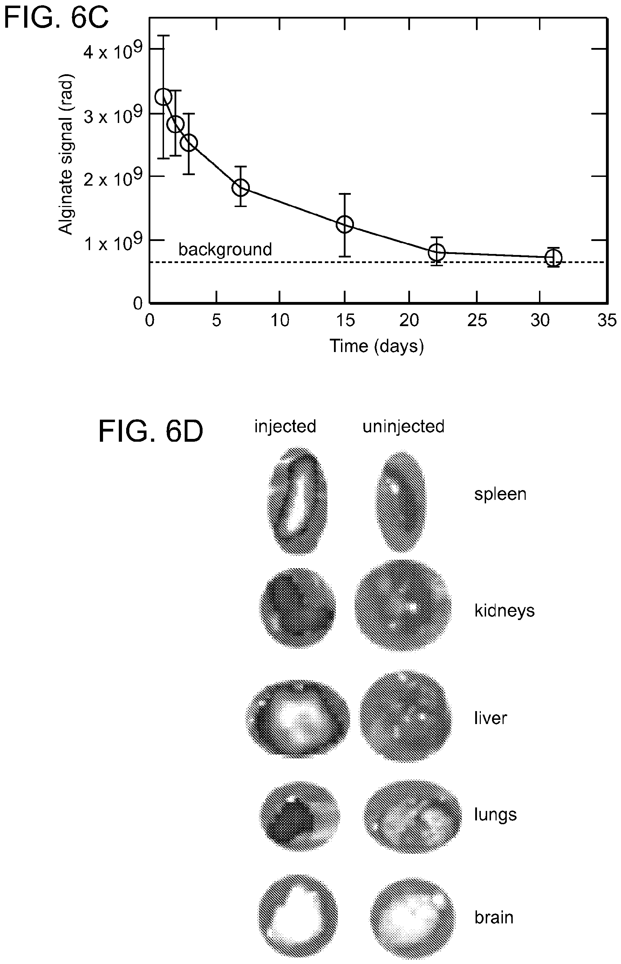

Figure 6A is a schematic for near-IR dye modification of alginate. Figure 6B is a set of fluorescence images of mice injected retro-orbitally with near-IR-dye-labeled (745 excitation, 820 emission) alginate. Images were taken on days 1, 8 and 22 post-injection. Figure 6C is a graph quantifying in vivo epi-fluorescence over four weeks in the blood by measuring snout fluorescence. Values represent mean and standard deviation. N=5. Figure 6D is a set of fluorescence images of internal organ two days after injection. These images illustrate the fate of IV-injected alginate in vivo.

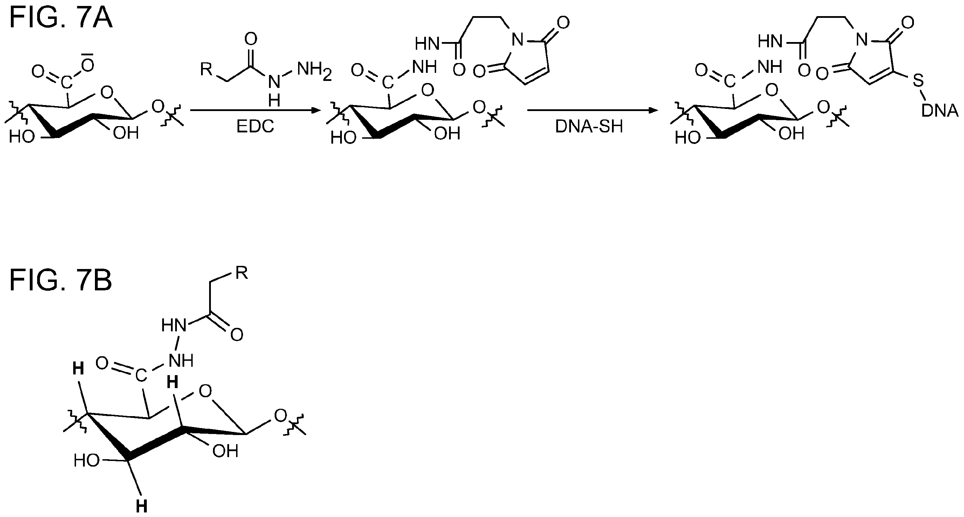

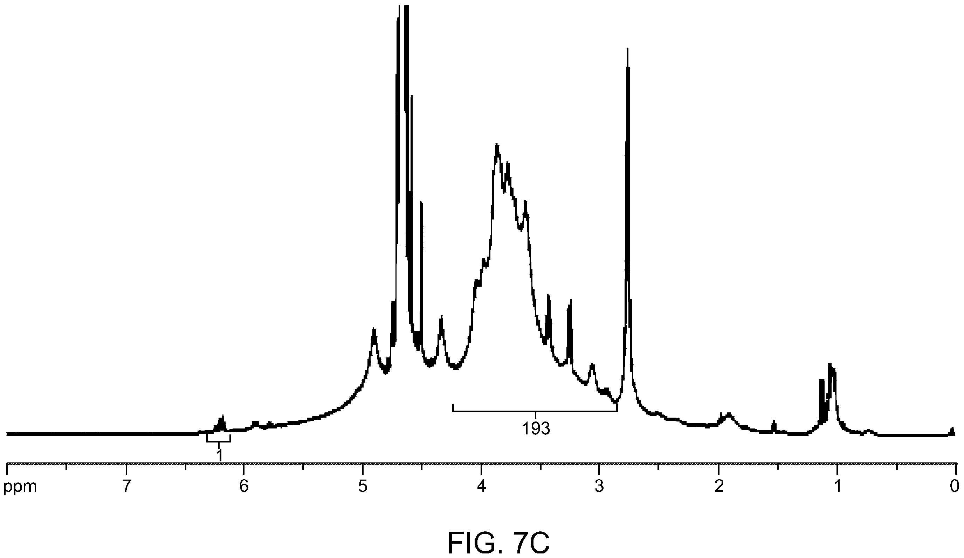

Figure 7A is schematic of conjugation of N-beta-Maleimidopropionic acid hydrazide-TFA (BMPH) and DNA to alginate. Figure 7B is a schematic of an alginate monomer (∼1,250 monomers / strand) modified with BMPH, red protons designate those with absorptions 3-4.4ppm, blue protons designate those with absorptions 6-6.56. Figure 7C is an nuclear magnetic resonance (NMR) spectrum of BMPH-modified alginate.

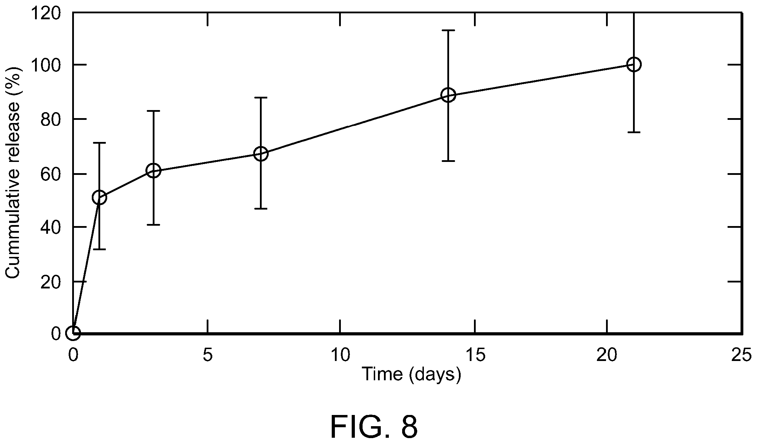

Figure 8 shows the release of doxorubicin from 2% calcium-crosslinked alginate gels over 21 days. Values represent mean and standard deviation. N=4.

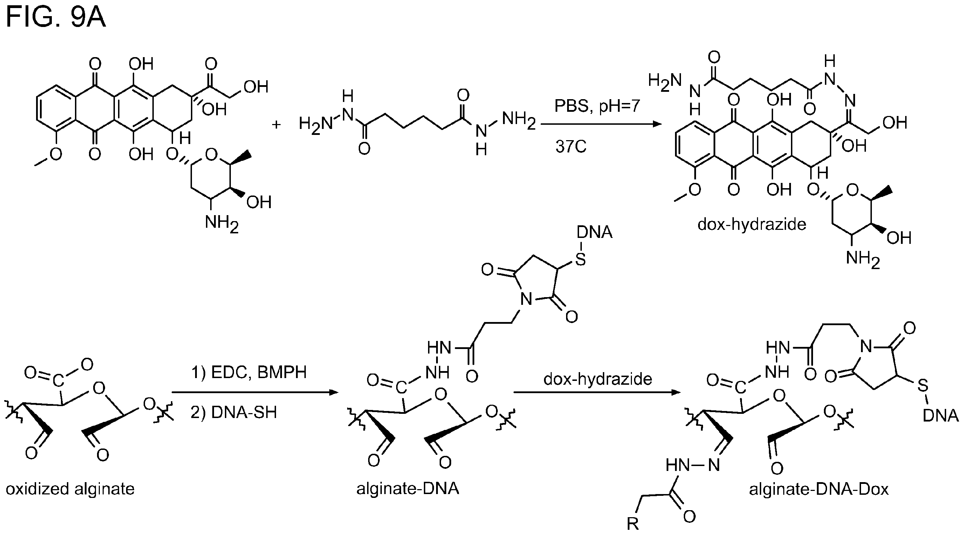

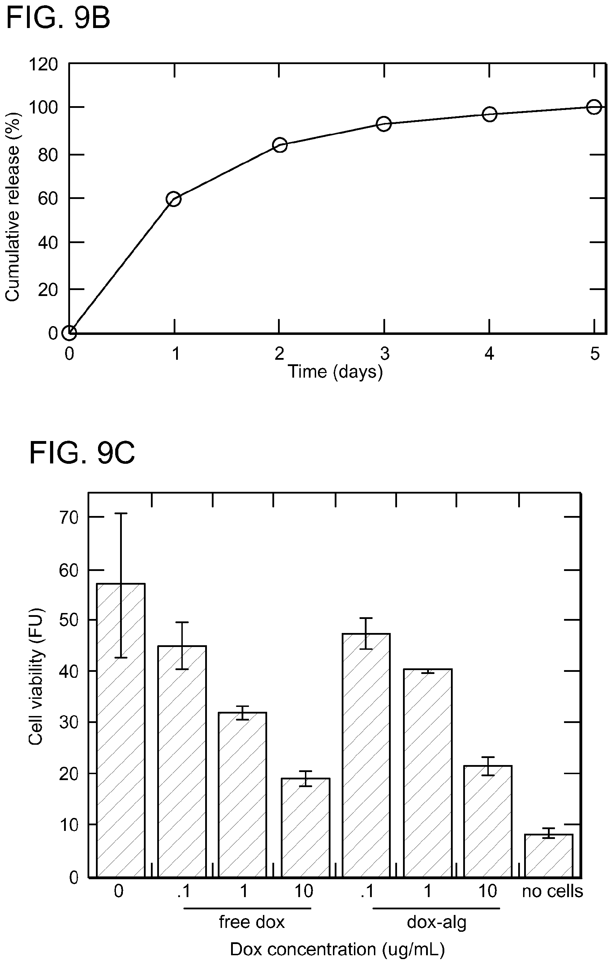

Figure 9A is a schematic of reactions performed on doxorubicin and alginate to bind doxorubicin to alginate through a hydrolyzable hydrazone linker. Figure 9B is a graph showing the release profile of hydrazone-linked doxorubicin from oxidized alginate. Figure 9C is a graph showing cell toxicity of free doxorubicin (dox) and of hydrazone-linked doxorubicin-alginate (dox-alg) against breast cancer MDA-MB-231 cells. Values represent mean and standard deviation. N=3. Doxorubicin loading and release from oxidized alginate is depicted in this figures.

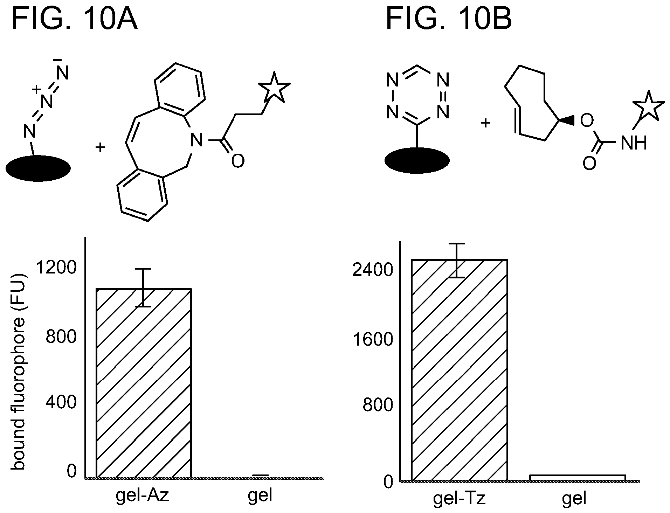

Figure 10A is a schematic and graph depicting click chemistry-mediated gel targeting. Azide-conjugated or control, calcium-crosslinked alginate gels were incubated with fluorophore carrying DBCO for 4 hours at 37 °C. Retained fluorescence was quantified. Figure 10B is a schematic and graph depicting click chemistry-mediated gel targeting. Tetrazine-conjugated or control, calcium-crosslinked alginate gels were incubated with fluorophore carrying trans-cyclooctene for 4 hours at 37 °C. Retained fluorescence was quantified.

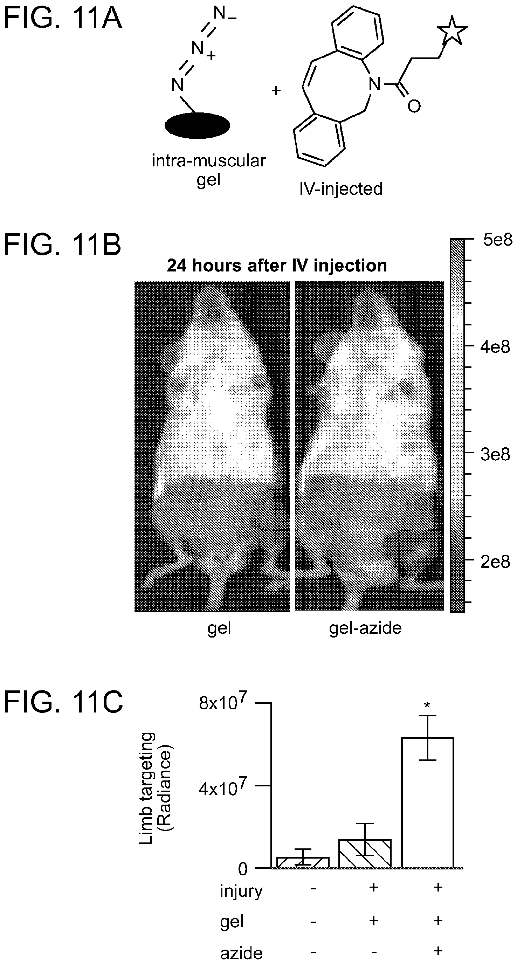

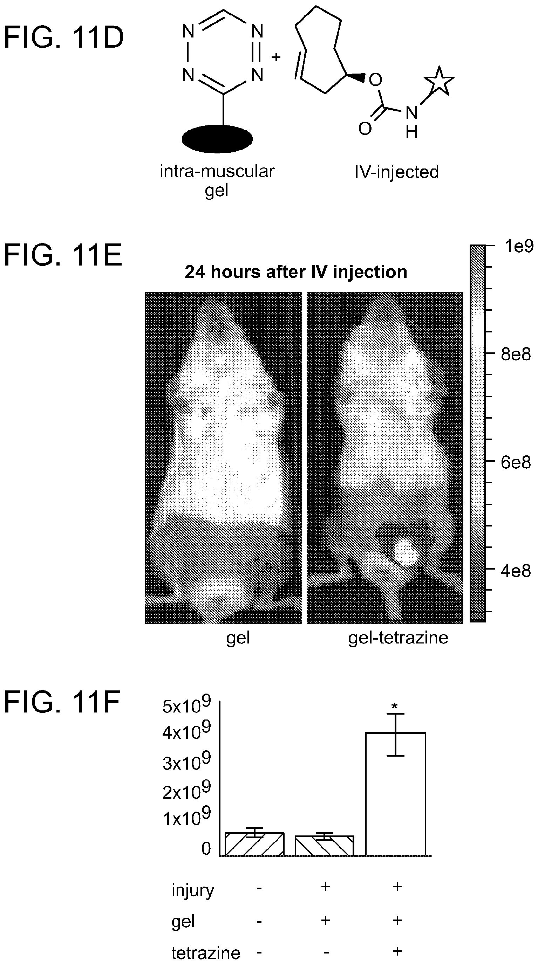

Figure 11A is a reaction scheme for alginate gels conjugated to azide or unconjugated, reacting with fluorescently labeled DBCO. Figure 11B is a set of images showing targeting of the IV-injected fluorescently labeled DBCO to the intra-muscular gel in the hindlimb of a hindlimb injury mouse model. Figure 11C is a graph quantifying the amount of fluorescence observed. Figure 11D is a reaction scheme for alginate gels conjugated to tetrazine or unconjugated, reacting with fluorescently labeled TCO. Figure 11E is a set of fluorescence images showing targeting of the IV-injected fluorescently labeled TCO to the intra-muscular gel in the hindlimb of a hindlimb injury mouse model. Figure 1 IF is a graph quantifying the amount of fluorescence observed. Values represent mean and S.E.M. n=3. * represents p<.01 vs. both controls by Student's t-test. Click chemistry mediated targeting of a drug surrogate to a hindlimb injury model.

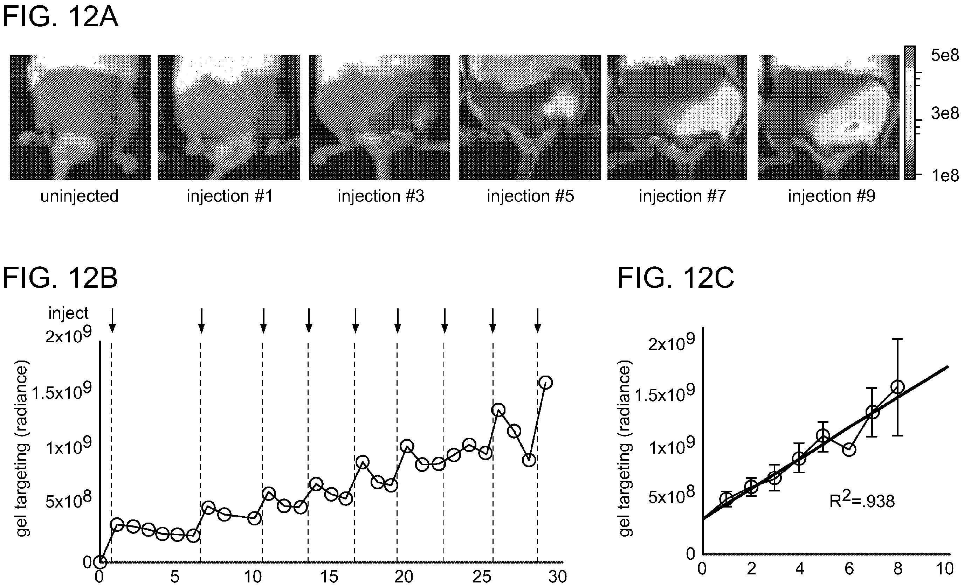

Figure 12A is a set of fluorescence images showing fluorescently-labeled DBCO that was repeatedly administered to mice (blue arrows) over the course of a one month. Figure 12B is a graph quantifying limb fluorescence 24 hours after repeat injections of targeting fluorescent DBCO. Figure 12C is a graph quantifying fluorescence 24 hours after fluorophore administration, showing a roughly linear increase in fluorescence with each injection. Repeat targeting of fluorescent drug surrogates to intra-muscular gel was achieved.

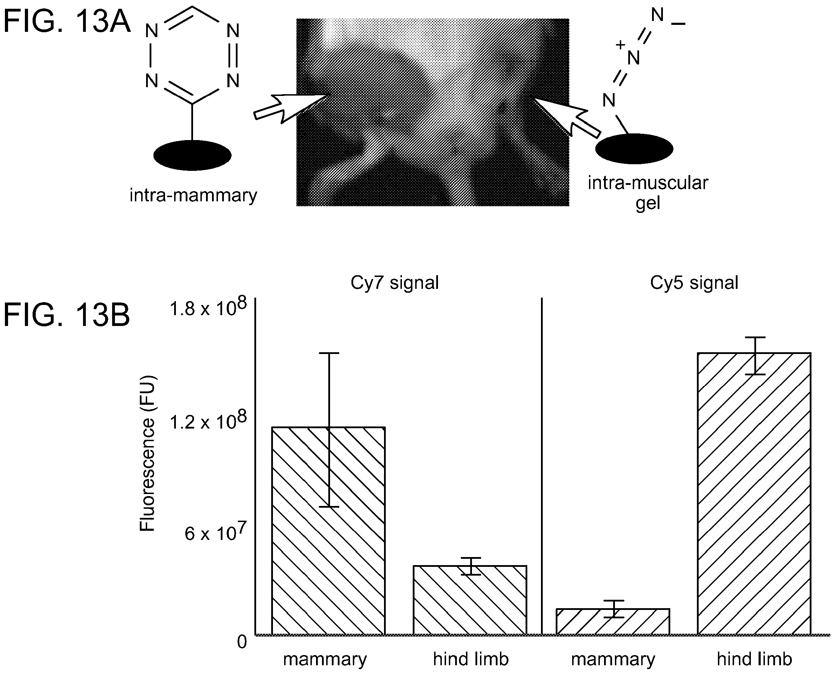

Figure 13A is an image plus schematic showing fluorescence in a mouse 1) in which alginate-Tz gel was implanted intra-muscularly in the left hind limb and alginate-Az was implanted into the right mammary pad of the same mouse, and 2) where a mixture of Cy5-TCO and Cy7-DBCO was injected IV, and 3) with the mouse imaged 48 hours later with fluorescence at both injection sites. Figure 13B is a set of graphs quantifying the fluorescence at each injection site. Spatial separation and specific targeting of two different small molecules was achieved using this system.

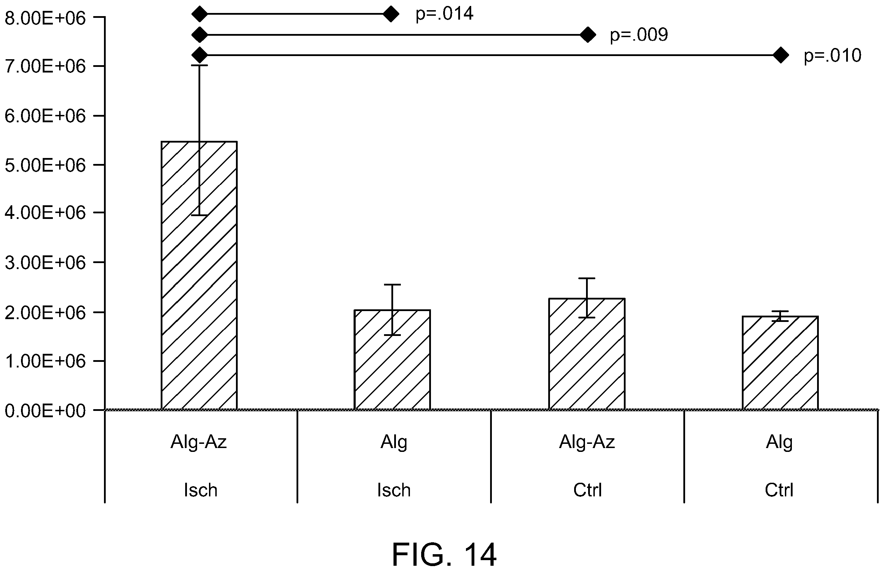

Figure 14 is a graph showing the targeting of oral delivery of fluorescently labeled DBCO to an alginate-azide gel implanted in mouse models of hind limb ischemia. Fluorescence (y-axis) at the site of alginate injection on mouse models of hind limb ischemia (Isch) and at a control site on the contralateral limb (Ctrl) is shown.

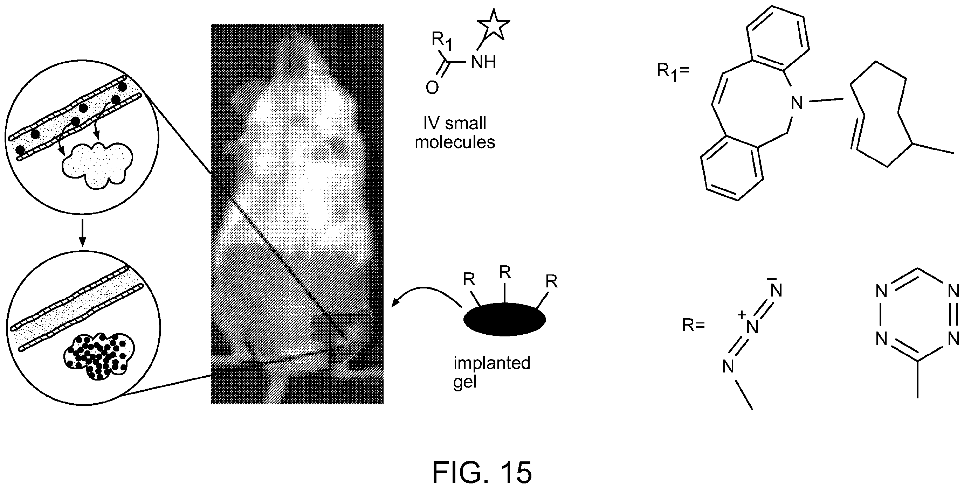

Figure 15 is an image and schematics illustrating the targeting of small molecules (e.g., via IV or oral) specifically to an implanted gel at a specific site in a body. The targeting can be mediated by click chemistry reactions.

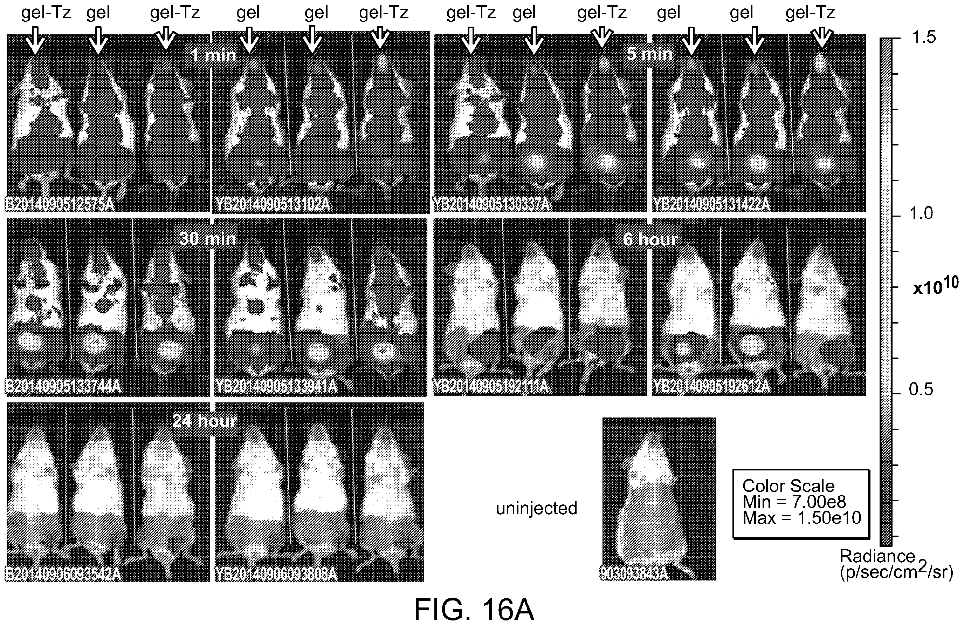

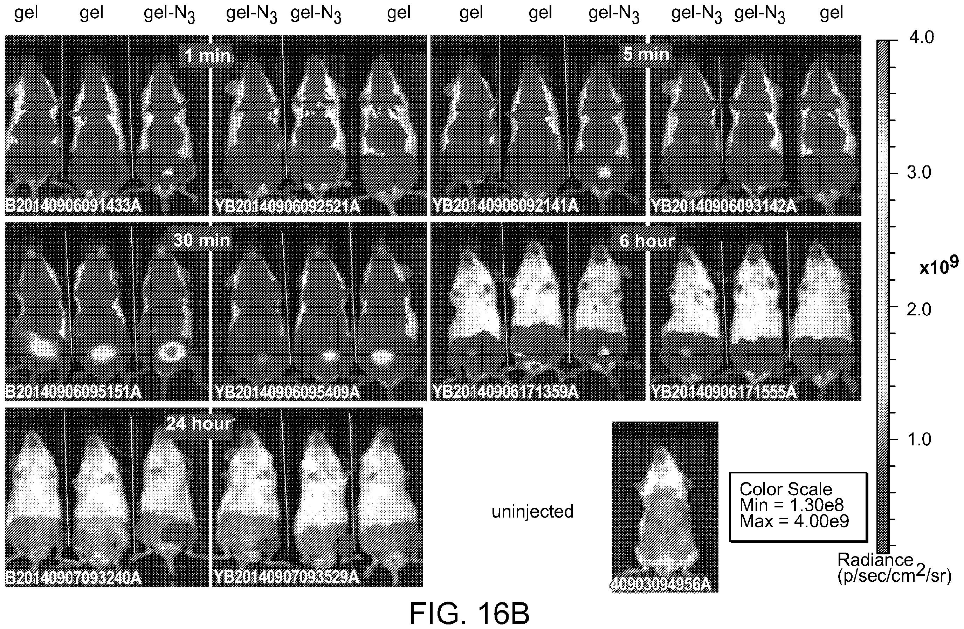

Figure 16A is a panel of live animal images of mice subjected to hind-limb ischemia and injected with unconjugated alginate gels or conjugated to tetrazine. 24 hours after surgery, fluorescent-TCO was injected IV. Animals were imaged at 1, 5, and 30 minutes, 6 hours, and 24 hours following TCO injection. Figure 16B is a panel of live cell images of mice subjected to hind-limb ischemia and injected with unconjugated alginate gels or conjugated to azide. 24 hours after surgery, fluorescent-DBCO was injected IV. Animals were imaged at 1, 5 and 30 minutes, 6 hours and 24 hours following DBCO injection. Images show mice (n=3 for each of the 2 groups) labeled with the alginate gel received intramuscularly at the different time points.

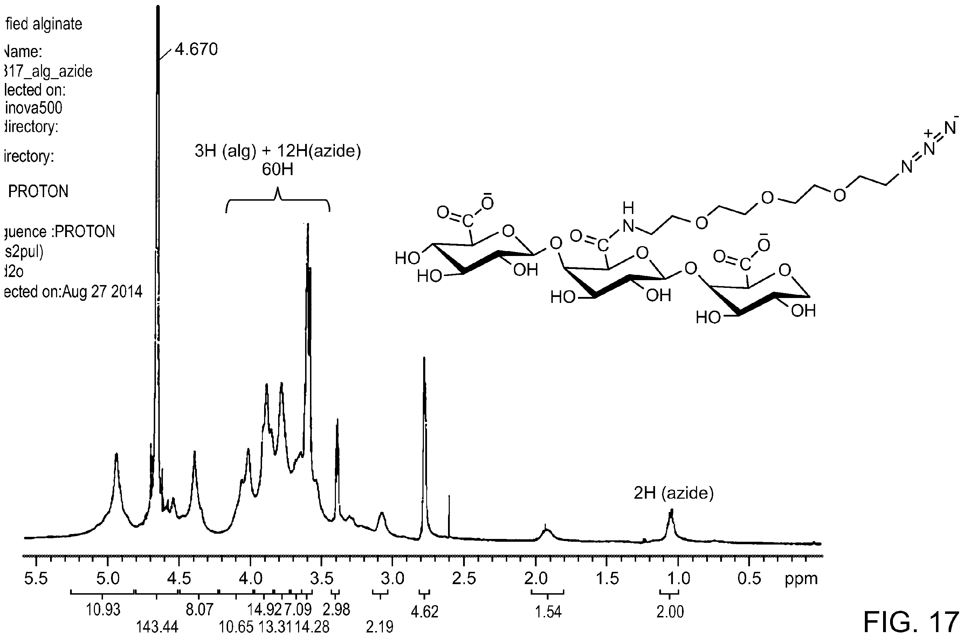

Figure 17 is a ¹H NMR spectrum of azide-modified alginate.

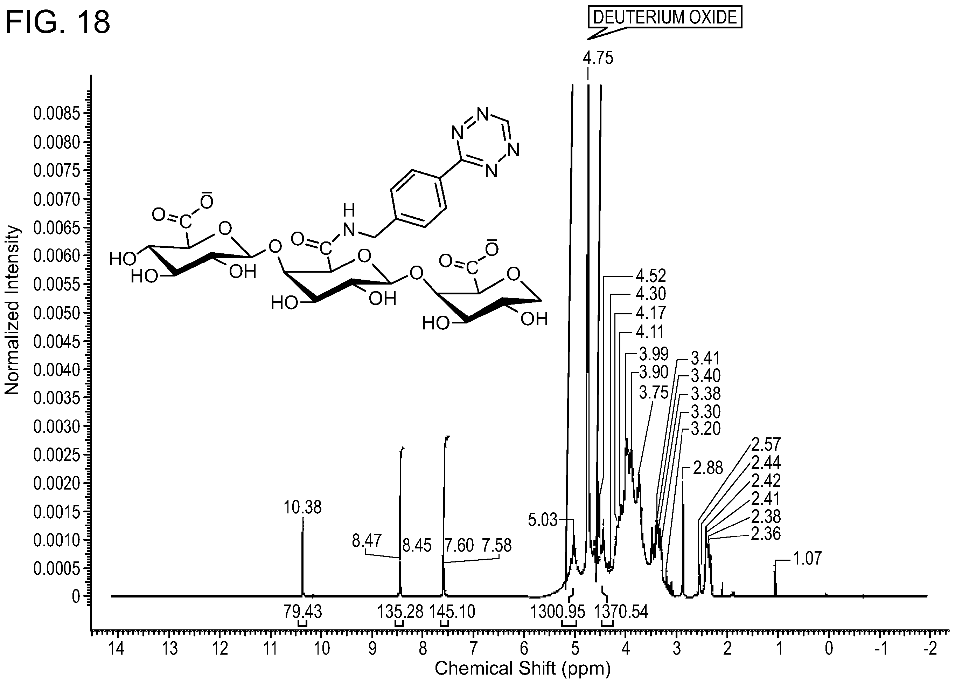

Figure 18 is a ¹H NMR spectrum of tetrazine-modified alginate.

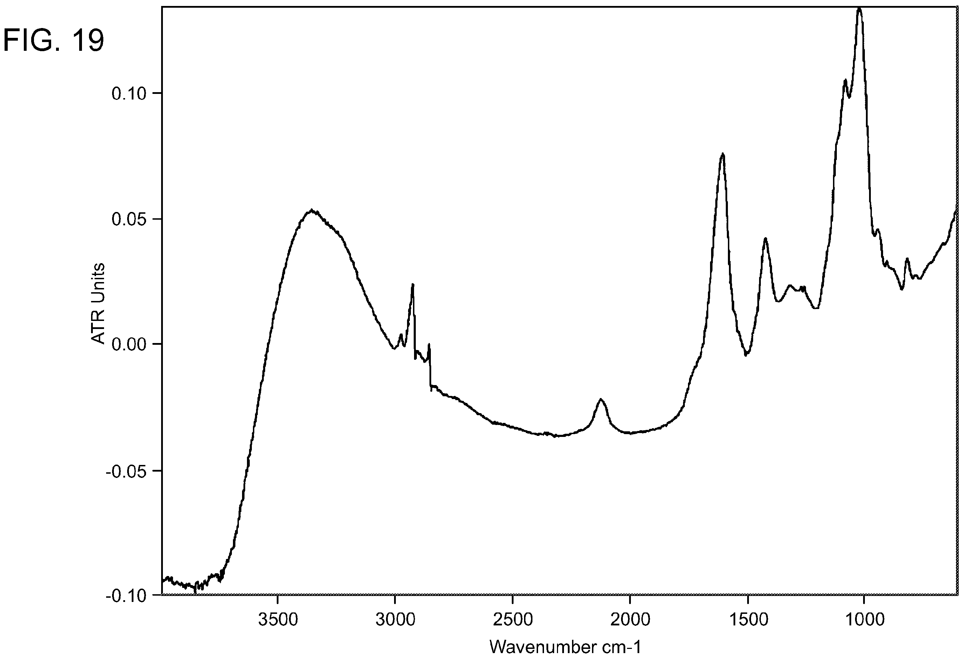

Figure 19 is an attenuated total reflectance (ATR)/infrared (IR) (ATIR) spectrum of azide-conjugated alginate.

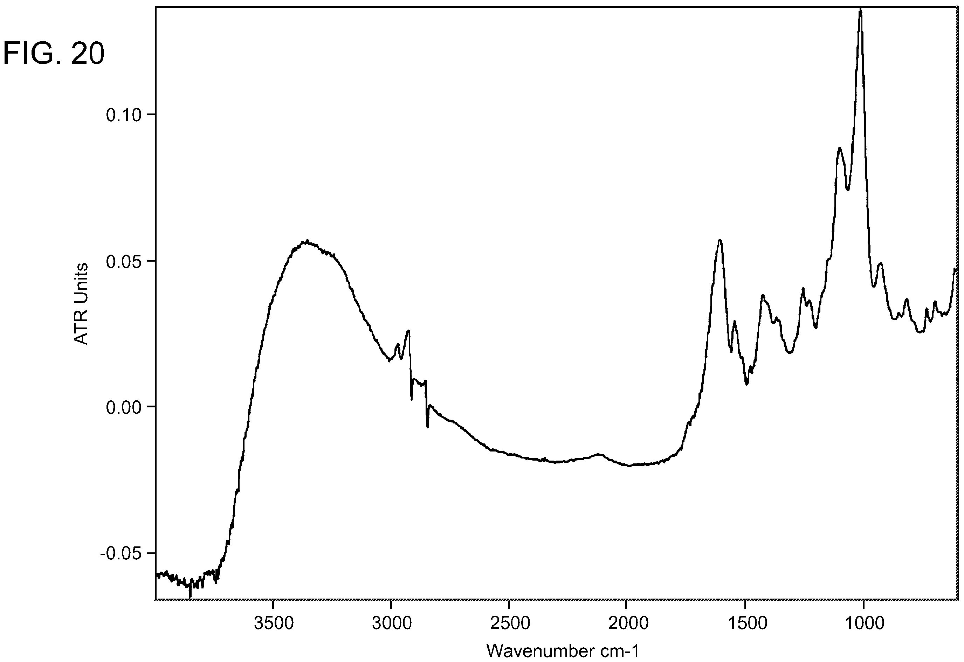

Figure 20 is an ATIR spectrum of azide-conjugated alginate reacted with 100 equivalents of DBCO-Cy7.

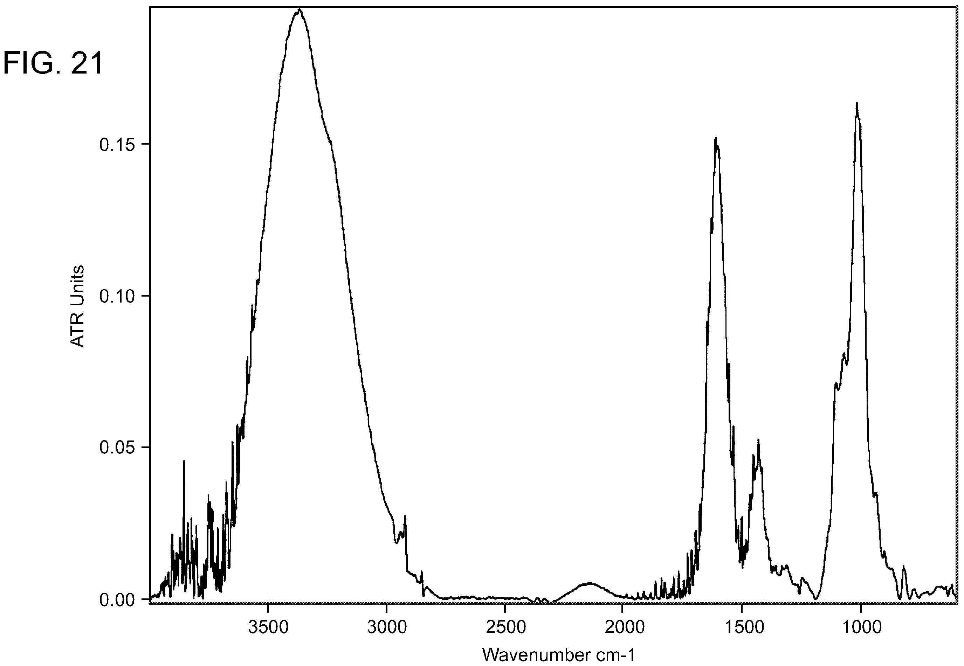

Figure 21 is an ATIR spectrum of tetrazine-conjugated alginate.

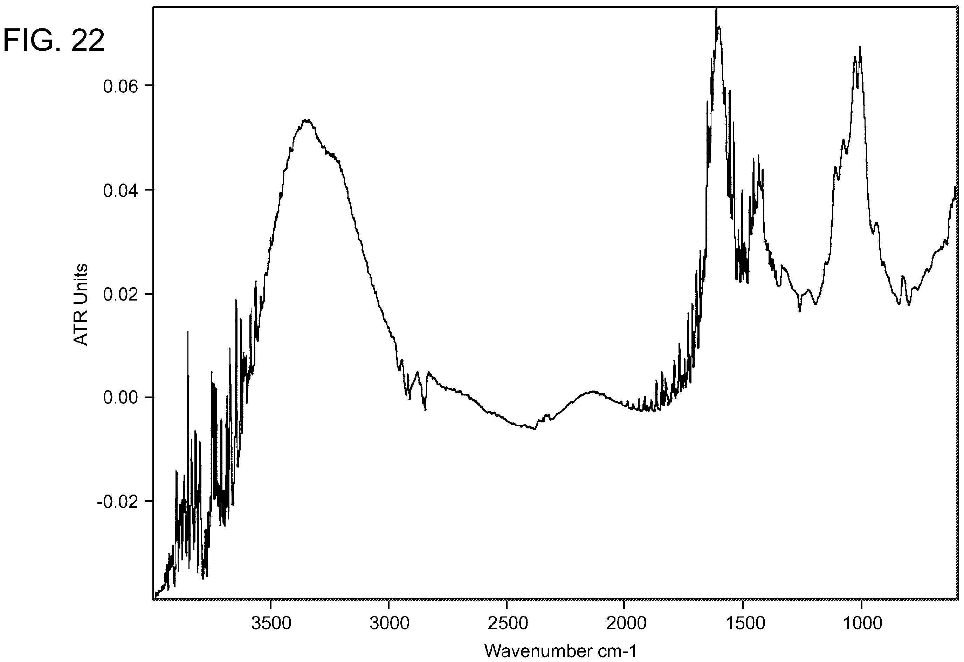

Figure 22 is an ATIR spectrum of tetrazine-conjugated alginate reacted with 100 equivalents of TCO-Cy5.

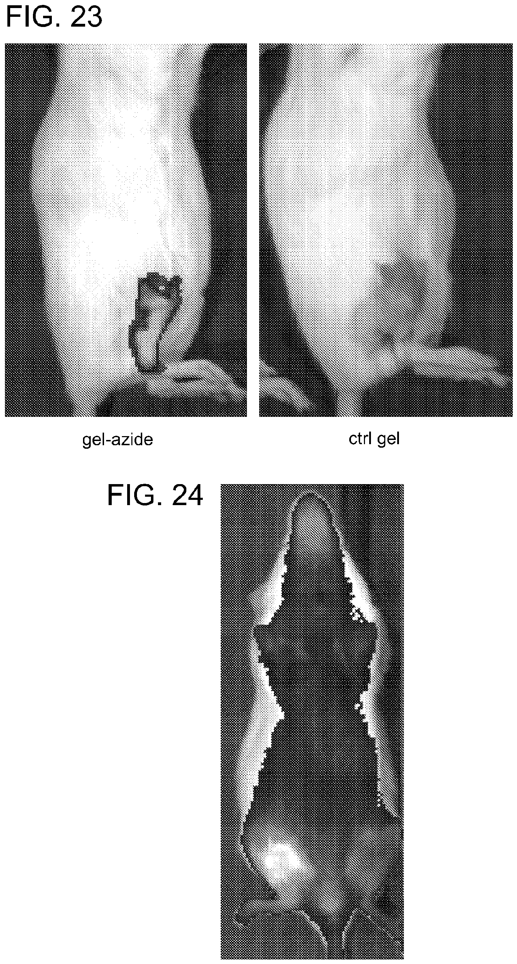

Figure 23 is a set of images showing targeting of the IV-injected fluorescently labeled DBCO to an intraosseous gel modified with azide or unmodified control gel in the femur of a mouse.
Figure 24 is an image showing targeting of the IV-injected fluorescently labeled DBCO to a gel modified with azide, injected into the right knee joint of a mouse.

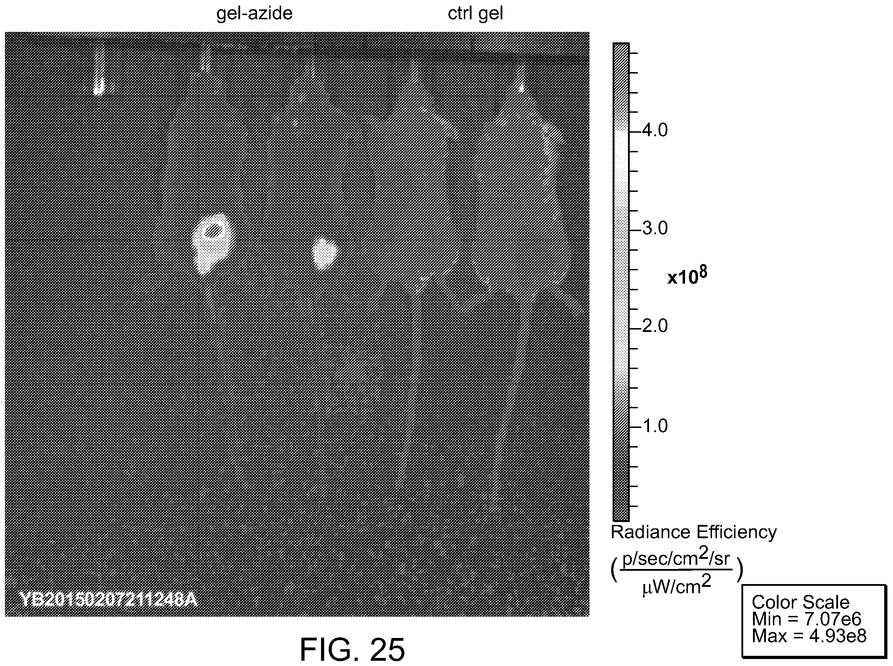

Figure 25 is an image showing targeting of the IV-injected fluorescently labeled DBCO to a gel modified with azide or control gel, injected into the tumor of a mouse. Imaged 24 hours after IV injection.

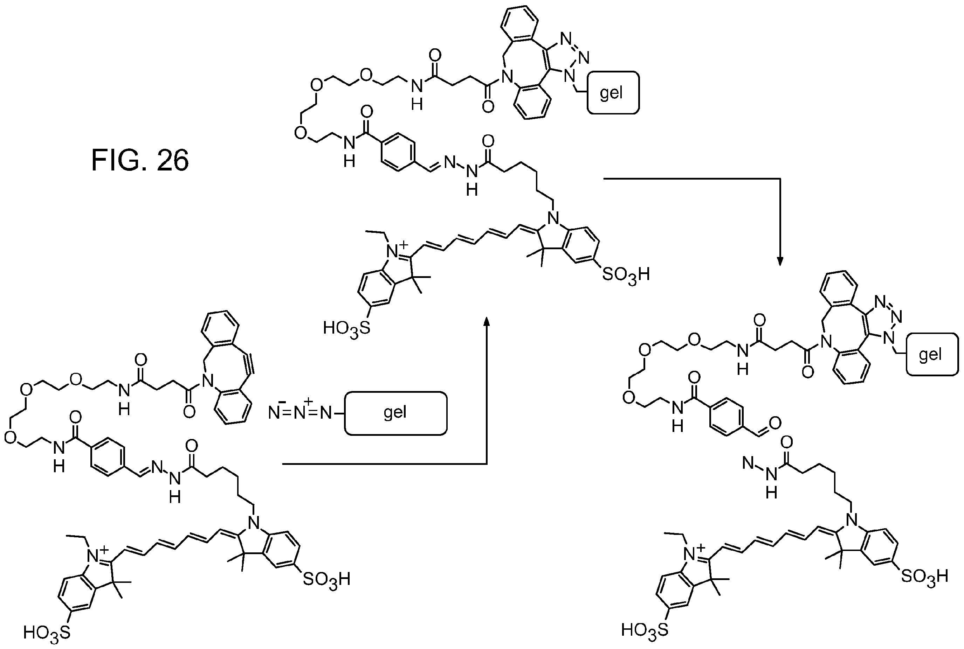

Figure 26 shows the capture of a small molecule (Cy7 linked to DBCO through a hydrolysable hydrazone linker) by an azide-modified gel and the subsequent release of a small molecule through hydrolysis after the capture.

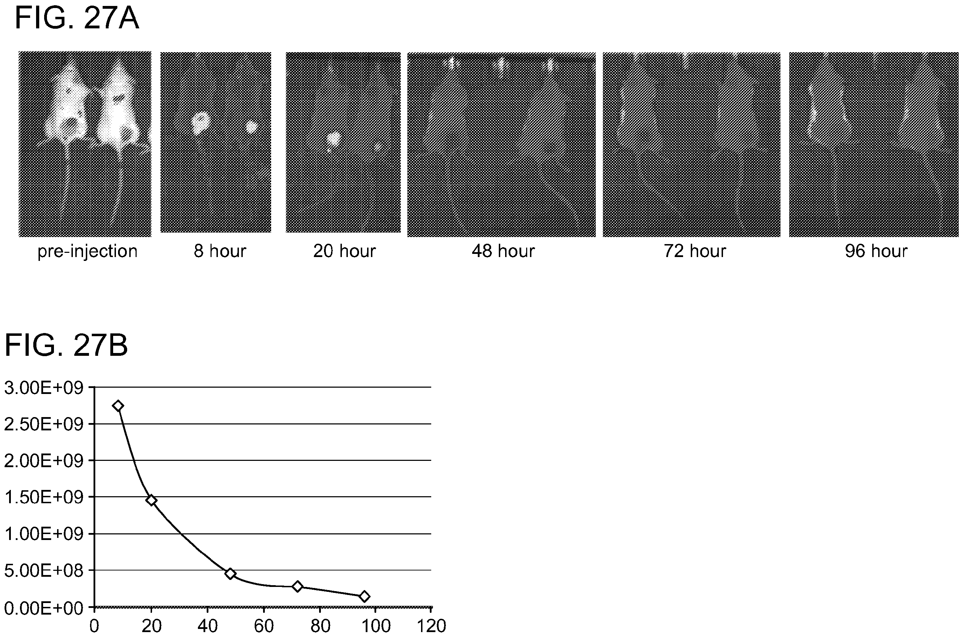

Figure 27A is a series of images showing targeting of the IV-injected small molecule by a gel modified with azide injected into the tumor of a mouse. The small molecule is subsequently released, leading to loss of the signal. Figure 27B is a line graph showing the quantification of the small molecule at the tumor site over time (hours) demonstrating release of the small molecule.

### DETAILED DESCRIPTION OF THE INVENTION

Systemic drug toxicity is a serious problem in current clinical interventions, including in cancer, arrthymia, immunosuppression, and ocular drug delivery. Local drug-delivering devices confer a substantial reduction in toxicity and thus have significant clinical utility, including in prevention of restenosis with stenting, cancer treatment, and enhanced wound healing. However, in applications where repeated local implantation of devices is not possible or desirable, e.g., for implantation of a drug-delivering scaffold during invasive surgery, there remains a need for drugs that can be targeted to implant sites through non-invasive means.

Also, targeting small molecules to diseased tissues as therapy or diagnosis is a significant challenge in drug delivery. Drug-eluting devices implanted during invasive surgery allow for controlled presentation of drugs at disease sites, but cannot be modified once surgery is complete.

The invention provides refillable drug delivery systems for use in replenishing or refilling a delivery device *in vivo* in a minimally invasive manner, e.g., via targeting with fresh drug payloads through the blood. For example, an injectable delivery device is manufactured to include moieties/molecules that bind refills infused into the blood (Fig 1A). Drug payloads infused into the blood of a patient extravasate into target tissues and are bound by the device (Fig 1B), subsequently allowing for sustained release of drug at the target site (Fig 1C). Efficient delivery of drug payload to the device in some cases requires more than one pass through the target site. Target sites with enhanced permeability are good candidates for sites of administration of drug delivery devices described herein. Materials with long blood circulation times and high stability are used in the devices of the systems of the invention.

Described herein are refillable drug delivery devices in which DNA recognition is used to target drug-carrying nanoparticle refills to a device placed within a tumor site. Drug payloads circulating in the blood of a patient can be bound by the device through specific chemical recognition, allowing for subsequent release through low-pH-mediated hydrolysis or enzymatic degradation at the target site. The results described herein demonstrate that DNA-conjugated alginate drug payloads can be utilized for refilling of drug-delivering hydrogels containing the complementary DNA sequence, and that this concept can be exploited for treatment of diseases, such as cancer. Free alginate has a long serum lifetime and can be chemically modified with DNA and drugs, making it a suitable carrier for refilling of a drug delivery depot/device. The results described herein show that free alginate strands conjugated to complementary DNA home to calcium cross-linked alginate gels *in vitro* and *in vivo.* For example, when DNA-alginate strands carrying coupled doxorubicin were injected into tumor-bearing mice, repeated drug refilling of the intra-tumor gels inhibited tumor growth.

The device/system described herein for localized drug delivery allows for minimally invasive refilling of drug depots/delivery devices for repeat drug dosing over the course of days, weeks, months, or longer. For example, the methods described herein include blood-based refilling of drug-delivering devices using DNA complementarity.

Also, as described in the results herein, gel homing using a bioorthogonal system was demonstrated in an animal model of ischemia. Repeated homing to a gel site in vivo via multiple administrations, e.g., over the period of one month, was achieved. Click chemistry-mediated targeting exhibited a high degree of specificity for the target site and the targeting was successfully repeated over a period of at least one month through nine administrations. Also, the devices/systems described herein permitted segregation of two different molecules (e.g., two different target molecules that bind to different target recognition motifs) through the use of two separate orthogonal chemistry systems. The spatial resolution of two different molecules targeting two different sites on the same animal demonstrates the utility of the device/systems described herein in combining incompatible therapies in patients.

The refilling methods described herein are applicable to the treatment of many diseases. As shown in the Examples, bioorthogonal click chemistry was used to target circulating small molecules to hydrogels resident intramuscularly in diseased tissues. Small molecules were repeatedly targeted to the diseased area over the course of at least one month. Two bioorthogonal reactions were used to segregate two small molecules injected as a mixture to two separate locations in a mouse disease model. Thus, click chemistry is useful for pharmacological drug delivery, e.g., in refilling drug depots in cancer therapy, wound healing, and/or drug-eluting vascular grafts and stents.

The ability to repeatedly target drug surrogates to a specific site within the body shows that implants within the body, such as hydrogels and medical devices, can be selectively targeted by small molecules circulating in the blood. The efficient and repeated targeting or refilling of drug depots (described in the Examples) permit long-lasting, local drug delivery at sites in the body, such as sites of intravascular stenting, intra-tumor chemotherapy delivery devices or surgical implants. The system allows for lowered toxicity, improved dosing and drug availability at disease site. Efficient homing of drug molecules to sites defined by an injected hydrogel or implant also permit controlled or on-demand release of drugs, e.g., by an external stimulus such as a magnetic field or focused light.

Also as shown in the Examples, in accordance with the drug delivery devices/refill systems and methods described herein, orally administered drugs were selectively targeted to the site of a device/depot. For example, the orally administered drugs accumulated in the intended site, e.g., that of the device/depot, and did not accumulate elsewhere in the body. Thus, the drug delivery devices/refill systems are suitable for the delivery of any orally available drug for which there is a desire to have it concentrate at a specific anatomic location(s). Exemplary orally available drugs include but are not limited to over the counter or prescription anti-inflammatory agents, steroids, immunosuppressive drugs, as well as other drugs/agents described herein.

When administered orally, the drugs described herein do not or only minimally dissolve or degrade in the stomach or intestines. For example, when administered orally, less than 50% dissolves/degrades in the stomach or intestine, e.g., less than 40%, less than 30%, less than 20%, less than 10%, less than 5%, or less than 1% dissolves/degrades in the stomach or intestine. The drugs/therapeutic agents retain their activity or therapeutic potential while traversing the gastrointestinal tract and absorption into the circulatory system. Suitable oral drugs include, but are not limited to, opioids (e.g., hydromorphone, hydrocodone, oxycodone, oxymorphone, ethylmorphine, and buprenorphine), NSAIDs (e.g., ibuprofen, naproxen, and acetaminophen), and antibiotics (e.g., beta lactams, linezolid, clindamycin, macrolines (e.g., erythromycin and clarithromycin), and quinolones). Moreover, degradation-resistant linkers are utilized to ensure that the linker between the drug and the target molecule does not dissolve/degrade in the stomach or intestines. Suitable degradation-resistant linkers include esters, oxyethyl, oxymethyl, oxypropyl, amides, and carbonyl. Degradation-resistant linkers stable at low or neutral pH and with a very slow cleavage rate at neutral pH (days to weeks) are utilized so that the linker slowly cleaves when attached to the device.

In some examples, drugs or diagnostic agents are conjugated to target motifs through cleavable linkers. For example, drugs are targeted to a specific location in vivo to be released by an external stimuli such as a magnetic field or light. See, e.g., Cezar et al. advanced healthcare materials (2014); Zhao et al. Proc. Natl. Acad. Sci. USA 108(2011):67; Mura et al. Nat. Materials 12(2013):991. Examples of cleavable linkers include disulfide linkers that are cleaved through reduction by free thiols and other reducing agents; peptide linkers that are cleaved through the action of proteases and peptidase; nucleic acid linkers cleaved through the action of nucleases; esters that are cleaved through hydrolysis either by enzymes or through the action of water in vivo; hydrazones, acetals, ketals, oximes, imine, aminals and similar groups that are cleaved through hydrolysis in the body; photo-cleavable linkers that are cleaved by the exposure to a specific wavelength of light; mechano-sensitive groups that are cleaved through the application of ultrasound or a mechanical strain (e.g., a mechanical strain created by a magnetic field on a magneto-responsive gel).

In some cases, the device comprises a stable structural material, e.g., a hydrogel (e.g., cryogel), or mesoporous silica (MPS) composition (e.g., MPS rod). See, e.g., WO 13/158673. For example, the hydrogel comprises shape memory properties. See, e.g., US 2014-0227327; US 2014-0112990.

In some embodiments, a refill comprises a pro-drug, e.g., a drug in an inactive form. After administration of the refill to a subject, the pro-drug remains in inactive form until it binds to the device. Only after binding to the device does the pro-drug become processed (e.g., cleaved) into an active drug form. The linker is cleaved very slowly, e.g., on a time frame much slower than the clearance rate of the drug from the body or the degradation rate of the drug itself. Thus, the drug-linker-target molecule binds to the device and the linker is cleaved slowly (through no action of the device itself) to release the drug from the device.

In some cases, the drug pre-filled in the device is directly connected to a target recognition moiety. For example, when the hydrogel is placed in a tumor site, in the spinal cord, or in bone, the target recognition moiety is present throughout the device. The targeted drugs penetrate inside the hydrogel, as diffusion through the device is relatively fast. In another example, such as click-modified bone cement, although the target recognition moiety is present throughout the device, the drug may only be able to target the surface of the device, as diffusion into a polymer is relatively slow.

Alternatively, the target recognition moiety is only on the surface of the device, e.g., in the pores of the device and/or on the external surface of the device. For example, the surfaces of orthopedic implants are coated with a polymer containing at least a target recognition moiety. In this case, the drug would target only the surface of the orthopedic implants.

In some cases, the bioorthogonal reaction that links a drug to a device is itself reversible, permitting the delivery of the drug through the reversal of the bond used to bind the drug to the device.

Provided herein is a system for selective targeting of drug (e.g., small molecule drug) surrogates (e.g., drug refills) to devices (e.g., hydrogels) for drug delivery at specific sites in a body, e.g., at ischemic and non-ischemic disease sites, such as muscular and mammary sites.

In some embodiments, the invention features a system including a stationary drug delivery device and a drug refill. Upon administration of the device to a subject, the amount of the pharmaceutical composition in the polymer decreases over time, such that the pharmaceutical composition is delivered to a surrounding tissue and/or bloodstream of the subject. The device is refillable by administration of a drug refill comprising a pharmaceutical composition through the bloodstream of the subject.

In some examples, the drug delivery device comprises a pharmaceutical composition, a target recognition molecule and a hydrogel. The drug refill comprises the pharmaceutical composition attached to a target molecule via a cleavable linker, and optionally, a nanocarrier (e.g., a polymer, such as alginate or PLG strand; liposome nanoparticle; metal, such as gold, nanoparticle). The target molecule and the target recognition molecule form a two-component binding pair. The drug refill is mobile until the target molecule on the drug refill binds to the target recognition molecule on the drug delivery device. Upon binding of the target molecule to the target recognition molecule, the drug refill delivers the pharmaceutical composition to the drug delivery device, thereby refilling the drug delivery device.

Exemplary polymers include alginate, cellulose, chitosan, polymethacrylic acid, poly(vinylpyridine), polyacrylamide, polystyrene, polyethyleneglycol, poly(D,L-lactide-co-glycolide), tragacanth, acacia, guar, gelatin, pectin, carrageenan, sodium alginate and dextrin, methyl cellulose, carboxymethylcellulose, polyvinylpyrrolidone, polyvinylpyrrolidone/vinyl alcohol copolymer, polymers of acrylic acid, carboxy vinyl polymers, polyvinyl alcohol, and vinyl pyrrolidone/vinyl acetate copolymer.

In some embodiments, a refill comprises components with the following connectivity (where "----" indicates a linkage, e.g., a bond or plurality of bonds):
Target molecule (e.g., TCO/DBCO/norbornene/DNA)----nanocarrier---- pharmaceutical composition
Target molecule----pharmaceutical composition---nanocarrier
Nanocarrier----target molecule---- pharmaceutical composition
Target molecule----pharmaceutical composition (with no nanocarrier).

In some examples, a target molecule linked directly to a pharmaceutical composition (without a nanocarrier) is useful as a refill to increase tissue penetration and is suitable for oral administration (e.g., in pill form). In other examples, a refill comprising a nanocarrier is useful for cancer treatment applications.

In some embodiments, a linkage between a target molecule, nanocarrier, and/or pharmaceutical composition comprises a covalent bond/linker such as an amide, ester, hydrazide, ether, thioether, alkyl, peg linker, ketone, or amine. In other cases, the linkage comprises a noncovalent or ionic bond. In the present invention, the linkage comprises a cleavable linker, such as one of the exemplary cleavable linkers described above.

In some embodiments, the target molecule and/or target recognition molecule comprises a nucleic acid, peptide, polysaccharide, lipid, small molecule, or combination thereof, and the homing agent comprises a nucleic acid, peptide, polysaccharide, lipid, small molecule, or combination thereof.

Described herein, the target and the target recognition moiety comprise a nucleic acid. For example, the target comprises a nucleic acid sequence that is complementary to the nucleic acid sequence of the target recognition moiety. For example, the nucleic acid comprises DNA, RNA, modified DNA, or modified RNA. In some examples, the nucleic acid comprises DNA or modified DNA.

DNA complementarity has been widely exploited to direct specific chemical interactions, including formation of complex nanostructures, surface functionalization, and nanoparticle and cell assemblies. See, e.g., Douglas S, et al. (2009) Nature 459(7245):414-418; Wei B, Dai M, & Yin P (2012) Nature 485(7400):623-626; Onoe H, et al. (2012) Langmuir : the ACS journal of surfaces and colloids 28(21):8120-8126; Gartner Z & Bertozzi C (2009) Proceedings of the National Academy of Sciences of the United States of America 106(12):4606-4610; Zhang Y, Lu F, Yager K, van der Lelie D, & Gang O (2013) Nature nanotechnology 8(11):865-872. The utility of DNA in these applications lies in the sequence specificity and strength of binding as well as the tunability of interaction strength. One challenge to this type of application is DNA stability. Alginate may be conjugated to the 3' end of DNA to overcome 3'-5' exonucleases (the major serum-based nuclease). See, e.g., Shaw J, Kent K, Bird J, Fishback J, & Froehler B (1991) Nucleic acids research 19(4):747-750; Floege J, et al. (1999) The American journal of pathology 154(1):169-179; Gamper H, et al. (1993) Nucleic acids research 21(1):145-150. In some embodiments, the backbone is modified with phosphorothioate groups for increased endonuclease and chemical stability. See Campbell J, Bacon T, & Wickstrom E (1990) Journal of biochemical and biophysical methods 20(3):259-267.

Described herein, the target may comprise one or more nucleotides that are complementary to one or more nucleotides of the target recognition moiety. For example, 50% or more (e.g., 50%, 60%, 70%, 80%, 90%, 95%, or 100%) of the nucleotides of the target may be complementary to 50% or more (e.g., 50%, 60%, 70%, 80%, 90%, 95%, or 100%) of the nucleotides of the target recognition moiety.

The melting temperature (Tm) of the target:target recognition moiety hybridization may be at least 40 °C, e.g., at least 40 °C, 45 °C, 50 °C, 55 °C, 60 °C, 65 °C, 70 °C, 75 °C, 80 °C, 85 °C, 90 °C, 95 °C, or 100 °C, or more.

Nucleic acids described herein include DNA, modified DNA, RNA, modified RNA, locked nucleic acids, peptide nucleic acids (PNA), threose nucleic acids (TNA), hexitol nucleic acids (HNA), bridge nucleic acids, cyclohexenyl nucleic acid, glycerol nucleic acids, morpholinos, phosphomorpholinos, as well as aptamers and catalytic nucleic acid versions thereof. For example, the nucleic acid contains a modified nitrogenous base. For example, modified DNA and RNA include 2'-o-methyl DNA, 2'-o-methyl RNA, 2'-fluoro-DNA, 2'-fluoro-RNA, 2'-methoxy-purine, 2'-fluoro-pyrimidine, 2'-methoxymethyl-DNA, 2'-methoxymethyl-RNA, 2'-acrylamido-DNA, 2'-acrylamido-RNA, 2'-ethanol-DNA, 2'-ethanol-RNA, 2'-methanol-DNA, 2'-methanol-RNA, and combinations thereof. The nucleic acid may contain a phosphate backbone. Also described herein, the nucleic acid contains a modified backbone, e.g., a phosphorothioate backbone, phosphoroborate backbone, methyl phosphonate backbone, phosphoroselenoate backbone, or phosphoroamidate backbone.

The nucleic acid described herein may be single-stranded. For example, the nucleic acid is partially single-stranded and partially double-stranded, e.g., due to secondary structures, such as hairpins.

Described herein, each nucleic acid molecule may comprise 8-50 nucleotides, e.g., 8, 10, 12, 14, 16, 18, 20, 22, 24, 26, 28, 30, 32, 34, 36, 38, 40, 42, 44, 46, 48, or 50 nucleotides e.g., 20 nucleotides.

Descibed herein, the target and/or the target recognition moiety may comprise a nucleic acid molecule comprising 8-50 nucleotides, e.g., 8, 10, 12, 14, 16, 18, 20, 22, 24, 26, 28, 30, 32, 34, 36, 38, 40, 42, 44, 46, 48, or 50 nucleotides, e.g., 20 nucleotides.

Described herein, the target may comprise a phosphorothioate nucleic acid molecule having the sequence of TTTTTTTTTTTTTTTTTTTT (SEQ ID NO: 1), also called (T)₂₀. Described herein, the target may comprise a phosphorothioate nucleic acid molecule having the sequence of AAAAAAAAAAAAAAAAAAAA (SEQ ID NO: 2), also called (A)₂₀.

Described herein, the target recognition moiety may comprise a phosphorothioate nucleic acid molecule having the sequence of TTTTTTTTTTTTTTTTTTTT (SEQ ID NO: 1), also called (T)₂₀. Described herein, the target recognition moiety may comprise a phosphorothioate nucleic acid molecule having the sequence of AAAAAAAAAAAAAAAAAAAA (SEQ ID NO: 2), also called (A)₂₀.

Described herein, the targeting agent and/or the homing agent may comprise a nucleic acid molecule, e.g., a phosphorothioate nucleic acid molecule, having the sequence of a nucleotide sequence in Table 1.

**Table 1: Exemplary Nucleic Acid Sequences, shown 3'-5'. Italicized stands for phosphorothioate linkages. FAM stands for 5' 6-fluorescein. Hex stands for 5' Hexachlorofluorescein. SH stands for C3 3' thiol.**

| Oligo Name | Oligo Sequence 3' to 5' | SEQ ID NO: |
|---|---|---|
| polyT -SH | SH-TTTTTTTTTTTTTTTTTTTT | 3 |
| polyA-SH | SH-AAAAAAAAAAAAAAAAAAAA | 4 |
| polyT -F | TTTTTTTTTTTTTTTTTTTT-FAM | 5 |
| polyA-F | AAAAAAAAAAAAAAAAAAAA-FAM | 6 |
| polyT -SH/Hex | SH-TTTTTTTTTTTTTTTTTTTT-HEX | 7 |
| thioT-SH | SH-*TTTTTTTTTTTTTTTTTTTT* | 8 |
| thioA-SH | SH*-AAAAAAAAAAAAAAAAAAAA* | 9 |

Other exemplary nucleic acid:complementary nucleic acid pairs are shown in the table below.

| Sequence (5'to 3') | SEQ ID NO: | Complement (5' to 3') | SEQ ID NO: |
|---|---|---|---|
| CATGGAGAGCGACGAGAGCGG C | 10 | GCCGCTCTCGTCGCTCTCCAT G | 11 |
| TGTAACTGAGGTAAGAGG | 12 | CCTCTTACCTCAGTTACA | 13 |
| CCCCCCCCCCCCCCCCCCCC | 14 | GGGGGGGGGGGGGGGGGG G | 15 |
| ACACACACACACACACACACACAC AC | 16 | GTGTGTGTGTGTGTGTGTGTG TGT | 17 |
| GAATGAATGAATGAATGAAT GAAT | 18 | ATTCATTCATTCATTCATT CATTCATTCATTC | 19 |
| GGATTGGATTGATTGGATTG ATTGGATT | 20 | AATCCAATCCAATCCAATC CAATCCAATCC | 21 |
| GTAAAACGACGGCCAGT | 22 | ACTGGCCGTCGTTTTAC | 23 |
| GCTAGTTATTGCTCAGCGG | 24 | CCGCTGAGCAATAACTAG C | 25 |

Described herein, the target may comprise biotin and the target recognition moiety may comprise avidin or streptavidin. Alternatively, the target comprises avidin or streptavidin and the target recognition moiety comprises biotin. Biotin and streptavidin bind with a high affinity, e.g., with a K_{D} of about 10⁻¹⁴ M.

According to the invention, the target molecule comprises a bioorthogonal functional group and the target recognition molecule comprises a complementary functional group, where the bioorthogonal functional group is capable of reacting by click chemistry with the complementary functional group to form a covalent bond, as described in the table below.

For example, copper(I)-catalyzed Azide-Alkyne Cycloaddition (CuAAC) comprises using a Copper (Cu) catalyst at room temperature. The Azide-Alkyne Cycloaddition is a 1,3-dipolar cycloaddition between an azide and a terminal or internal alkyne to give a 1,2,3-triazole.

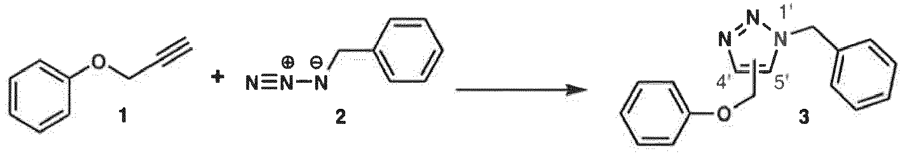

Another example of click chemistry includes Staudinger ligation, which is a reaction that is based on the classic Staudinger reaction of azides with triarylphosphines. It launched the field of bioorthogonal chemistry as the first reaction with completely abiotic functional. The azide acts as a soft electrophile that prefers soft nucleophiles such as phosphines. This is in contrast to most biological nucleophiles which are typically hard nucleophiles. The reaction proceeds selectively under water-tolerant conditions to produce a stable product. Phosphines are completely absent from living systems and do not reduce disulfide bonds despite mild reduction potential. Azides had been shown to be biocompatible in FDA-approved drugs such as azidothymidine and through other uses as cross linkers. Additionally, their small size allows them to be easily incorporated into biomolecules through cellular metabolic pathways

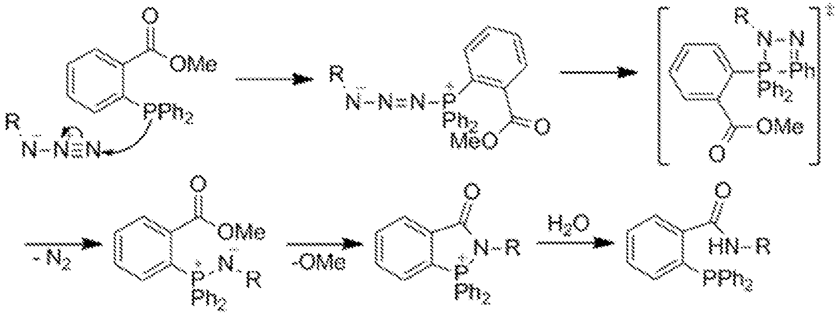

Copper-free click chemistry is a bioorthogonal reaction first developed by Carolyn Bertozzi as an activated variant of an azide alkyne cycloaddition. Unlike CuAAC, Cu-free click chemistry has been modified to be bioorthogonal by eliminating a cytotoxic copper catalyst, allowing reaction to proceed quickly and without live cell toxicity. Instead of copper, the reaction is a strain-promoted alkyne-azide cycloaddition (SPAAC). It was developed as a faster alternative to the Staudinger ligation, with the first generations reacting over sixty times faster. The incredible bioorthogonality of the reaction has allowed the Cu-free click reaction to be applied within cultured cells, live zebrafish, and mice. Cyclooctynes were selected as the smallest stable alkyne ring which increases reactivity through ring strain which has calculated to be 19.9 kcal/mol.

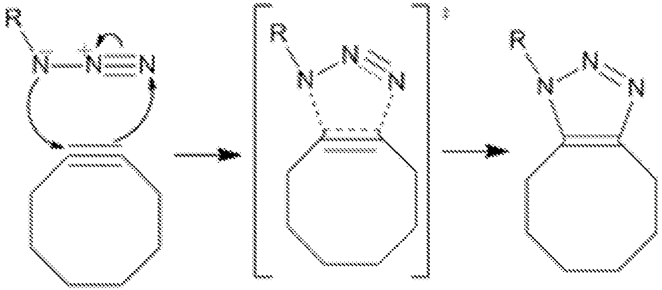

Copper-free click chemistry also includes nitrone dipole cycloaddition. Copper-free click chemistry has been adapted to use nitrones as the 1,3-dipole rather than azides and has been used in the modification of peptides.

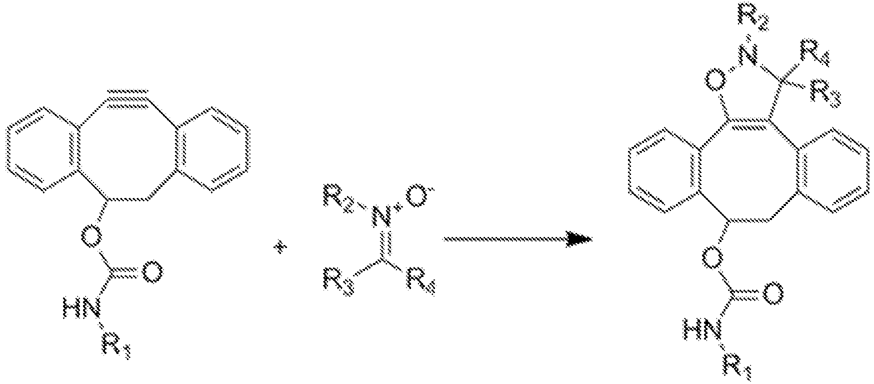

This cycloaddition between a nitrone and a cyclooctyne forms N-alkylated isoxazolines. The reaction rate is enhanced by water and is extremely fast with second order rate constants ranging from 12 to 32 M⁻¹•s⁻¹, depending on the substitution of the nitrone. Although the reaction is extremely fast, incorporating the nitrone into biomolecules through metabolic labeling has only been achieved through post-translational peptide modification.

Another example of click chemistry includes Norbornene Cycloaddition. 1,3 dipolar cycloadditions have been developed as a bioorthogonal reaction using a nitrile oxide as a 1,3-dipole and a norbornene as a dipolarophile. Its primary use has been in labeling DNA and RNA in automated oligonucleotide synthesizers.

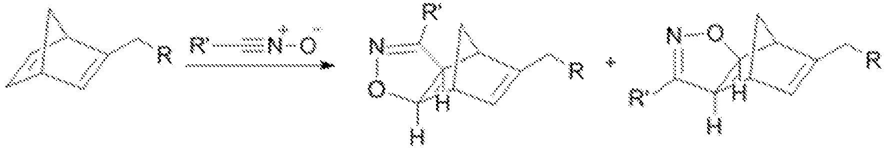

Norbornenes were selected as dipolarophiles due to their balance between strain-promoted reactivity and stability. The drawbacks of this reaction include the cross-reactivity of the nitrile oxide due to strong electrophilicity and slow reaction kinetics.

Another example of click chemistry includes oxanorbomadiene cycloaddition. The oxanorbomadiene cycloaddition is a 1,3-dipolar cycloaddition followed by a retro-Diels Alder reaction to generate a triazole-linked conjugate with the elimination of a furan molecule. This reaction is useful in peptide labeling experiments, and it has also been used in the generation of SPECT imaging compounds.

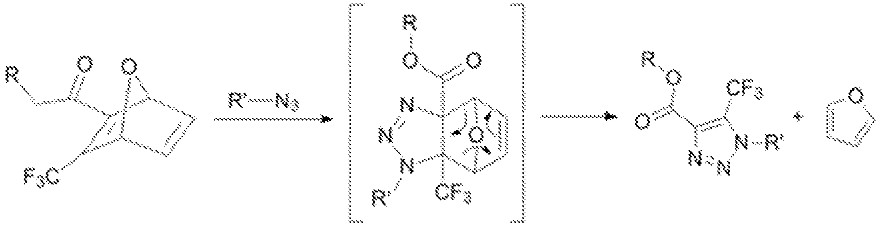

Ring strain and electron deficiency in the oxanorbomadiene increase reactivity towards the cycloaddition rate-limiting step. The retro-Diels Alder reaction occurs quickly afterwards to form the stable 1,2,3 triazole. Limitations of this reaction include poor tolerance for substituents which may change electronics of the oxanorbomadiene and low rates (second order rate constants on the order of 10⁻⁴).

Another example of click chemistry includes tetrazine ligation. The tetrazine ligation is the reaction of a trans-cyclooctene and an s-tetrazine in an inverse-demand Diels Alder reaction followed by a retro-Diels Alder reaction to eliminate nitrogen gas. The reaction is extremely rapid with a second order rate constant of 2000 M⁻¹-s⁻¹ (in 9:1 methanol/water) allowing modifications of biomolecules at extremely low concentrations.

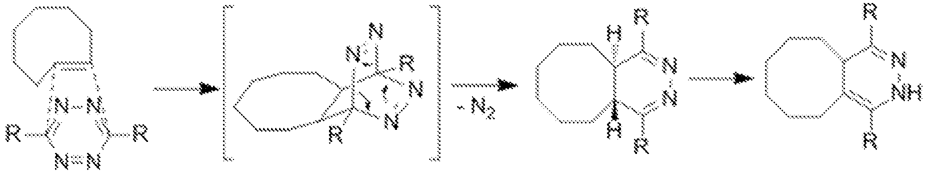

The highly strained trans-cyclooctene is used as a reactive dienophile. The diene is a 3,6-diaryl-s-tetrazine which has been substituted in order to resist immediate reaction with water. The reaction proceeds through an initial cycloaddition followed by a reverse Diels Alder to eliminate N₂ and prevent reversibility of the reaction.

Not only is the reaction tolerant of water, but it has been found that the rate increases in aqueous media. Reactions have also been performed using norbornenes as dienophiles at second order rates on the order of 1 M⁻¹•s⁻¹ in aqueous media. The reaction has been applied in labeling live cells and polymer coupling.

Another example of click chemistry includes is [4+1] cycloaddition. This isocyanide click reaction is a [4+1] cycloaddition followed by a retro-Diels Alder elimination of N₂.

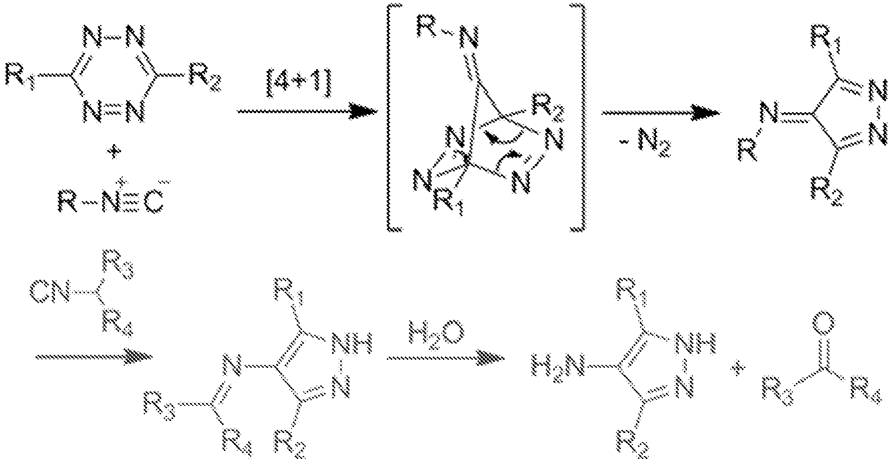

The reaction proceeds with an initial [4+1] cycloaddition followed by a reversion to eliminate a thermodynamic sink and prevent reversibility. This product is stable if a tertiary amine or isocyanopropanoate is used. If a secondary or primary isocyanide is used, the produce will form an imine which is quickly hydrolyzed.

Isocyanide is a favored chemical reporter due to its small size, stability, non-toxicity, and absence in mammalian systems. However, the reaction is slow, with second order rate constants on the order of 10⁻² M⁻¹•s⁻¹.

Another example of click chemistry includes quadricyclane ligation.
The quadricyclane ligation utilizes a highly strained quadricyclane to undergo [2+2+2] cycloaddition with π systems.

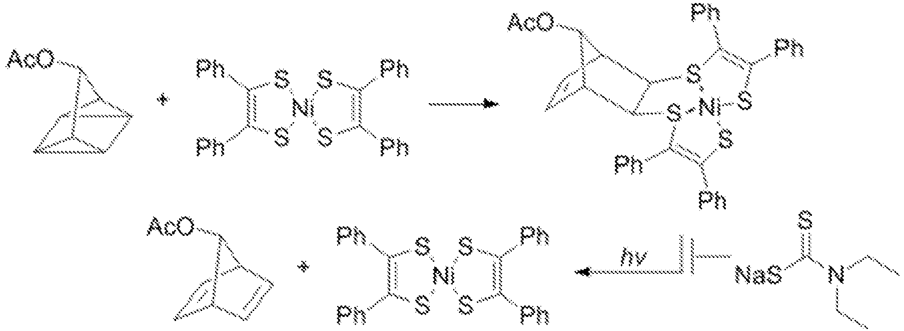

Quadricyclane is abiotic, unreactive with biomolecules (due to complete saturation), relatively small, and highly strained (-80 kcal/mol). However, it is highly stable at room temperature and in aqueous conditions at physiological pH. It is selectively able to react with electron-poor π systems but not simple alkenes, alkynes, or cyclooctynes.

Bis(dithiobenzil)nickel(II) was chosen as a reaction partner out of a candidate screen based on reactivity. To prevent light-induced reversion to norbornadiene, diethyldithiocarbamate is added to chelate the nickel in the product.

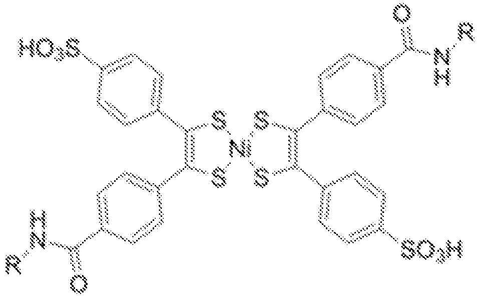

These reactions are enhanced by aqueous conditions with a second order rate constant of 0.25 M⁻¹•s⁻¹. Of particular interest is that it has been proven to be bioorthogonal to both oxime formation and copper-free click chemistry.

By bioorthogonal is meant a functional group or chemical reaction that can occur inside a living cell, tissue, or organism without interfering with native biological or biochemical processes. A bioorthogonal functional group or reaction is not toxic to cells. For example, a bioorthogonal reaction must function in biological conditions, e.g., biological pH, aqueous environments, and temperatures within living organisms or cells. For example, a bioorthogonal reaction must occur rapidly to ensure that covalent ligation between two functional groups occurs before metabolism and/or elimination of one or more of the functional groups from the organism. In other examples, the covalent bond formed between the two functional groups must be inert to biological reactions in living cells, tissues, and organisms.

Exemplary bioorthogonal functional group/complementary functional group pairs are shown in the table below.

| Functional group | Paired with | Functional group | Reaction type (Reference) |
|---|---|---|---|
| azide | | phosphine | Staudinger ligation (Saxon et al. Science 287(2000):2007-10) |
| azide | | Cyclooctyne, e.g., dibenzocyclooctyne, one of the cyclooctynes shown below, or other similar cyclooctynes: | Copper-free click chemistry (Jewett et al. J. Am. Chem. Soc. 132.11(2010):3688-90; Sletten et al. Organic Letters 10.14(2008):3097-9; Lutz. 47.12(2008):2182) |
| | | 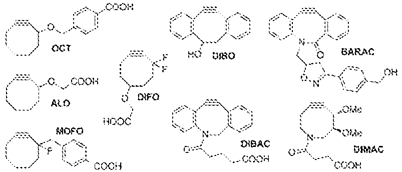 | |
| nitrone | | cyclooctyne | Nitrone Dipole Cycloaddition (Ning et al. 49.17(2010):3065) |
| Nitrile oxide | | norbornene | Norbornene Cycloaddition (Gutsmiedl et al. Organic Letters 11.11 (2009):2405-8) |
| oxanorbomadie ne | | azide | Oxanorbomadiene Cycloaddition (Van Berkel et al. 8.13(2007): 1504-8) |
| Trans-cyclooctene, norbornene, or other alkene | | s-tetrazine | Tetrazine ligation (Hansell et al. J. Am. Chem. Soc. 133.35(2011):13828-31) |
| nitrile | | 1,2,4,5-tetrazine | [4+1] cycloaddition (Stackman et al. Organic and Biomol. Chem. 9.21(2011):7303) |
| quadricyclane | | Bis (dithiobenzil)nickel(II) | Quadricyclane Ligation (Sletten et al. J. Am. Chem. Soc. 133.44(2011):17570-3) |
| Ketone or aldehyde | | Hydrazines, hydrazones, oximes, amines, ureas, thioureas, etc. | Non-aldol carbonyl chemistry (Khomyakova EA, et al. Nucleosides Nucleotides Nucleic Acids. 30(7-8) (2011) 577-84 |
| Thiol | | maleimide | Michael addition (Zhou et al. 2007 18(2):323-32.) |
| Dienes | | dienophiles | Diels Alder (Rossin et al. Nucl Med. (2013) 54(11):1989-95) |
| Tetrazine | | norbornene | Norbornene click chemistry (Knight et al. Org Biomol Chem. 2013 Jun 21;11(23):3817-25.) |

In some examples, a target molecule (e.g., on the refill) comprises a bioorthogonal functional group such as a trans-cyclooctene (TCO), dibenzycyclooctyne (DBCO), norbornene, tetrazine (Tz), or azide (Az). In other example, a target recognition moiety (e.g., on the device) comprises a bioorthogonal functional group such as a trans-cyclooctene (TCO), dibenzycyclooctyne (DBCO), norbornene, tetrazine (Tz), or azide (Az). TCO reacts specifically in a click chemistry reaction with a tetrazine (Tz) moiety. DBCO reacts specifically in a click chemistry reaction with an azide (Az) moiety. Norbornene reacts specifically in a click chemistry reaction with a tetrazine (Tz) moiety. For example, TCO is paired with a tetrazine moiety as target/target recognition moieties. For example, DBCO is paired with an azide moiety as target/target recognition moieties. For example, norbornene is paired with a tetrazine moiety as target/target recognition moieties.

The exemplary click chemistry reactions have high specificity, efficient kinetics, and occur in vivo under physiological conditions. See, e.g., Baskin et al. Proc. Natl. Acad. Sci. USA 104(2007):16793; Oneto et al. Acta biomaterilia (2014); Neves et al. Bioconjugate chemistry 24(2013):934; Koo et al. Angewandte Chemie 51(2012):11836; and Rossin et al. Angewandte Chemie 49(2010):3375. For a review of a wide variety of click chemistry reactions and their methodologies, *see,* e.g., Nwe K and Brechbiel MW, 2009 Cancer Biotherapy and Radiopharmaceuticals, 24(3): 289-302; Kolb HC et al., 2001 Angew. Chem. Int. Ed., 40: 2004-2021.

Described herein, the ratio of target molecules (e.g., nucleic acid molecules, biotin, streptavidin, avidin, or a bioorthogonal functional group described herein) to polymer molecules on the refill (e.g., alginate strands) maybe 1:1, 2:1, 3:1, 4:1, 5:1, 6:1, 7:1, 8:1, 9:1, 10:1, 15:1, 20:1, 30:1, 40:1, 50:1, 60:1, 70:1, 80:1, 90:1, 100:1, 120:1, 140:1, or higher.

Described herein, the ratio of the target recognition molecule (e.g., nucleic acid molecules, biotin, streptavidin, avidin, or a bioorthogonal functional group described herein) to the polymer molecule of the device (e.g., alginate hydrogel) may be at least 5:1, e.g., at least 5:1, 10:1, 20:1, 30:1, 40:1, 50:1, or greater. In some cases, at least 10 (e.g., at least 10, 20, 30, 40, 50, 60, 70, 80, 90, 100, 150, 200, 250, 300, or more) target recognition molecules are attached to each strand of the polymer (e.g., alginate). For example, where the target recognition molecule comprises a Tz or Az, at least 10 (e.g., at least 10, 20, 30, 40, 50, 60, 70, 80, 90, 100, 150, 200, 250, 300, or more) molecules of Tz or Az are attached to each strand of polymer (e.g., alginate).

For example, the binding affinity of the target molecule for the target recognition molecule is 500 µM or less. The binding affinity between the target molecule and target recognition molecule, e.g., measured by dissociation constant (K_{D}) may be 1 mM or less, e.g., 1 mM, 900 µM, 800 µM, 700 µM, 600 µM, 500 µM, 400 µM, 300 µM, 200 µM, 100 µM, 50 µM, 10 µM, 1 µM, 500 nM, 250 nM, 100 nM, 50 nM, 10 nM, 1 nM, 500 pM, 250 pM, 100 pM, 50 pM, 10 pM, 1 pM, 0.1 pM, 0.01 pM, or less. The K_{D} of the interaction between the homing agent and the targeting agent is measured using standard methods in the art.

For example, the hydrogel, e.g., alginate hydrogel, in the drug delivery device, e.g., when hydrated, has a volume of 1-500 µL, e.g., 10-250 µL, 20-100, µL, or 40-60 µL, e.g., about 50 µL).

The polymer of the device (e.g., alginate hydrogel) or the polymer of the refill (e.g., alginate strand) may be linked to a pharmaceutical composition. For example, the polymer may be linked to the pharmaceutical composition via a covalent bond, a noncovalent bond (e.g., hydrogen bond or van der Waals interaction), or an ionic bond.

The polymer (e.g., alginate) may be unoxidized or partially oxidized (e.g., by 1%, 2%, 3%, 4%, 5%, 6%, 7%, 8%, 9%, 10%, 15%, 20% or more). For example, the polymer (e.g., alginate) is oxidized in order to introduce functional groups, such as aldehyde functional groups for use in coupling of the polymer, e.g., alginate, to a pharmaceutical composition.

Described herein, drug refilling may be accomplished via binding of free alginate strands to calcium-crosslinked alginate gels mediated by complementary DNA interactions. As shown in the Examples herein, DNA-mediated binding of aginate outpaces non-specific interactions by five-fold. The significantly increased time for which infused alginate remains at the gel site in the *in vivo* studies supports the importance of the complementary DNA interaction to at least initially bind the free alginate to the gels.

The refillable drug delivery systems of the invention provide highly selective targeting of a refill to a specific site in the body, e.g., to a specific previously administered (e.g., stationary) device in the subject. For example, at least 2%, e.g., at least 2%, 4%, 6%, 8%, 10%, 15%, 20%, 30%, 40%, 50%,6 0%, 70%, 80%, 90%, or greater, of the refill molecules (e.g., comprising pharmaceutical composition(s)) administered to a subject travel to and are deposited at a specific drug delivery device, e.g., one that comprises a target recognition moiety that specifically binds to the target on the refill. Less than 98%, e.g., less than 98%, 95%, 90%, 85%, 80%, 70%, 60%, 50%, 40%, 30%, 20%, or fewer, of the refill molecules (e.g., comprising pharmaceutical composition(s)) administered to a subject are deposited at a site other than a device to which the target on the refill is targeted.

In some examples, an alginate strand of refill is coupled to a small molecule drug, such as doxorubicin, e.g., via an aldehyde functional group.

In some embodiments, a single set of target molecules (bioorthogonal functional groups) is used to refill the drug delivery device. Alternatively, multiple different target molecules (bioorthogonal functional groups) are used to deliver different, orthogonal payloads, e.g., to the same drug delivery device.

DNA nanotechnology may be used to create dynamic interactions. For instance, toe-hold-based DNA displacement can be used to remove bound refilling oligonucleotides from a target device to recover device binding sites and allow the re-use of device-bound target recognition moieties, such as oligonucleotides. Alternatively, device-bound target recognition moieties, e.g., oligonucleotides, can serve as aptamers or catalytic DNAzymes to bind a drug and/or drug-carrier for drug refilling.

In some examples, one drug delivery device is for administration to the subject. For example, the device comprises and/or delivers one or more kinds of pharmaceutical compositions. In this case, a refill comprises more than one kind of pharmaceutical composition such that one drug delivery device is refilled with more than one kind of pharmaceutical composition/drug.

In other examples, more than one device, e.g., 2, 3, 4, 5, 6, 7, 8, 9, 10, or more devices, is for administrationto the subject. Each device is capable of being refilled by a different drug/pharmaceutical composition. Alternatively, each device delivers the same drug/pharmaceutical composition.

In some cases, each implanted/injected device is capable of being refilled through multiple administrations of a drug (e.g., at least 2, 3, 4, 5, 6, 7, 8, 9, 10, 15, 20, 30 or more times) through the bloodstream without having to replace the device.

In one embodiment, the polymer of each device contains more than one kind of target recognition molecule. More than one refill is for administration to the subject. Each refill comprises a different target molecule, where each refill comprises a different kind of pharmaceutical composition/drug. In this way, each pharmaceutical composition/drug is associated with a unique target molecule. Thus, a single administration of a refill containing multiple different refills and pharmaceutical compositions targets the appropriate pharmaceutical compositions to specific devices, optionally located in different sites in the body. For example, one device is for administration to a subject intratumorally for delivery of an anti-cancer pharmaceutical composition and a separate device is for administration to a subject at a site of inflammation elsewhere in the body for delivery of an anti-inflammatory agent. Both devices can be refilled in a single administration of a mixture of refills-for example, one refill containing an anti-cancer drug and a target molecule specific for a target recognition molecule on the anti-cancer delivery device, and the other refill containing an anti-inflammatory agent and a target molecule specific for a target recognition molecule on the anti-inflammatory delivery device. In another example, a device comprising a polymer comprising a target recognition molecule (e.g., a Tz) is for administration to one site in a body. A device comprising a polymer comprising a different target recognition molecule (e.g., a Az) is for administration to a separate site in the same body. Administration of a drug refill comprising a target that specifically recognizes the first target recognition molecule (e.g., TCO, which recognizes Tz) and a drug refill comprising a target that specifically recognizes the second target recognition molecule (e.g., DBCO, which recognizes Az) leads to homing of each drug refill to the respective targeted device. The different refills may be for administration simultaneously or consecutively.

In another embodiment, e.g., where more than one drug delivery device is for administration to a subject, the polymer of each device comprises the same composition and same target recognition molecule. Each device delivers the same drug/pharmaceutical composition but at different sites in the body. A batch of refills for single administration comprising a particular target molecule and pharmaceutical composition refills each of the devices in the subject with the same pharmaceutical composition.

In some cases, devices, e.g., containing hydrogels, described herein comprise a non-biodegradable material. Exemplary non-biodegradable materials include, but are not limited to, metal, plastic polymer, or silk polymer. Devices, e.g., containing hydrogels, may be composed of a biocompatible material. This biocompatible material is non-toxic or non-immunogenic.

Devices, e.g., containing hydrogels, described herein may contain an external surface. Alternatively, or in addition, the devices, e.g., containing hydrogels, contain an internal surface. External or internal surfaces of the device, e.g., hydrogel, are solid or porous. Pore size is less than about 10 nm, in the range of about 100 nm-20 µm in diameter, or greater than about 20 µm, e.g., up to and including 1000 µm. For example, the pores are nanoporous, microporous, or macroporous. For example, the diameter of nanopores are less than about 10 nm; micropore are in the range of about 100 µm-20 µm in diameter; and, macropores are greater than about 20 µm (preferably greater than about 100 µm and even more preferably greater than about 400 µm, e.g., greater than 600 µm or greater than 800 µm). In one example, the device, e.g., hydrogel, is macroporous with open, interconnected pores of about 100-500 µm in diameter, e.g., 100-200, 200-400, or 400-500 µm.

A device, e.g., hydrogel, described herein may comprise one or more compartments.

The device, e.g., hydrogel, is biocompatible. The device, e.g., hydrogel, is biodegradable/erodable or resistant to breakdown in the body. Relatively permanent (degradation resistant) device materials include metals and some polymers such as silk. Preferably, one or more components of the device, e.g., hydrogel, degrades at a predetermined rate based on a physical parameter selected from the group consisting of temperature, pH, hydration status, and porosity, the cross-link density, type, and chemistry or the susceptibility of main chain linkages to degradation or it degrades at a predetermined rate based on a ratio of chemical polymers. For example, a high molecular weight polymer comprised of solely lactide degrades over a period of years, e.g., 1-2 years, while a low molecular weight polymer comprised of a 50:50 mixture of lactide and glycolide degrades in a matter of weeks, e.g., 1, 2, 3, 4, 6, 10 weeks. A calcium cross-linked gel composed of high molecular weight, high guluronic acid alginate degrade over several months (1, 2, 4, 6, 8, 10, 12 months) to years (1, 2, 5 years) *in vivo,* while a gel comprised of low molecular weight alginate, and/or alginate that has been partially oxidized, will degrade in a matter of weeks.

Exemplary materials used to form the device, e.g., hydrogel, include polylactic acid, polyglycolic acid, PLGA polymers, alginates and alginate derivatives, gelatin, collagen, fibrin, hyaluronic acid, laminin rich gels, agarose, natural and synthetic polysaccharides, polyamino acids, polypeptides, polyesters, polyanhydrides, polyphosphazines, poly(vinyl alcohols), poly(alkylene oxides), poly(allylamines)(PAM), poly(acrylates), modified styrene polymers, pluronic polyols, polyoxamers, poly(uronic acids), poly(vinylpyrrolidone) and copolymers or graft copolymers of any of the above. One preferred hydrogel includes an RGD-modified alginate. In other examples, the hydrogel includes crosslinked polymers, e.g., crosslinked alginates, gelatins, or derivatives thereof, such as those that are methacrylated.

Another preferred device, e.g., hydrogel, comprises a macroporous poly-lactide-co-glycolide (PLG). PLG matrices release an encapsulated pharmaceutical composition from the device, e.g., hydrogel, e.g., gradually over the next days or weeks after administration to the site in or on the subject to be treated. For example, release is approximately 60% of the pharmaceutical composition load within the first 5 days, followed by slow and sustained release of the pharmaceutical composition over the next 10 days. This release profile mediates a rate of diffusion of the pharmaceutical composition to and/or through the surrounding tissue.

A method of making a device, e.g., hydrogel, is carried out by providing a hydrogel material and covalently linking or noncovalently associating the hydrogel material with a pharmaceutical composition. Exemplary devices and methods of making them are described in US 2012/0100182, PCT/US2010/057630, and PCT/US2012/35505.

In some cases, components of the device, e.g., containing hydrogel, are organized in a variety of geometric shapes *(e.g.,* discs, beads, pellets), niches, planar layers *(e.g.,* thin sheets). For example, discs of about 0.1-200 millimeters in diameter, *e.g.,* 5, 10, 20, 40, 50 millimeters are implanted subcutaneously. The disc may have a thickness of 0.1 to 10 millimeters, e.g., 1, 2, 5 millimeters. The discs are readily compressed or lyophilized for administration to a patient. An exemplary disc for subcutaneous administration has the following dimensions: 8 millimeters in diameter and 1 millimeter in thickness. Multicomponent devices, e.g., containing hydrogels, are optionally constructed in concentric layers each of which is characterized by different physical qualities (% polymer, % crosslinking of polymer, chemical composition of the hydrogel, pore size, porosity, and pore architecture, stiffness, toughness, ductility, viscoelasticity, and/or pharmaceutical composition.

The device, e.g., hydrogel, is placed or transplanted on or next to a target tissue, in a protected location in the body, next to blood vessels, or outside the body as in the case of an external wound dressing. Devices are introduced into or onto a bodily tissue using a variety of known methods and tools, e.g., spoon, tweezers or graspers, hypodermic needle, endoscopic manipulator, endo- or trans-vascular- catheter, stereotaxic needle, snake device, organ-surface-crawling robot (United States Patent Application 20050154376; Ota et al., 2006, Innovations 1:227-231), minimally invasive surgical devices, surgical implantation tools, and transdermal patches. Devices can also be assembled in place, for example by senquentially injecting or inserting matrix materials.

In some examples, hydrogel materials include biodegradable or permanent materials such as those listed below. The mechanical characteristics of the hydrogel vary according to the application or tissue type for which regeneration is sought. It is biodegradable (*e.g*., collagen, alginates, polysaccharides, polyethylene glycol (PEG), poly(glycolide) (PGA), poly(L-lactide) (PLA), or poly(lactide-co-glycolide) (PLGA), poly lactic-coglycolic acid, or permanent (e.g., silk). In the case of biodegradable structures, the hydrogel is degraded by physical or chemical action, *e.g.,* level of hydration, heat or ion exchange or by cellular action, e.g., elaboration of enzyme, peptides, or other compounds by nearby or resident cells. The consistency varies from a soft/pliable (*e.g.*, a gel) to glassy, rubbery, brittle, tough, elastic, stiff. The hydrogel structures contain pores, which are nanoporous, microporous, or macroporous, and the pattern of the pores is optionally homogeneous, heterogenous, aligned, repeating, or random.

Alginates are versatile polysaccharide based polymers that may be formulated for specific applications by controlling the molecular weight, rate of degradation and method of scaffold formation. Coupling reactions can be used to covalently attach bioactive epitopes, such as the cell adhesion sequence RGD to the polymer backbone. Alginate polymers are formed into a variety of hydrogel types. Injectable hydrogels can be formed from low molecular weight (MW) alginate solutions upon addition of a cross-linking agents, such as calcium ions, while macroporous hydrogels are formed by lyophilization of high MW alginate discs. Differences in hydrogel formulation control the kinetics of hydrogel degradation. Release rates of pharmaceutical compositions, e.g., small molecules, morphogens, or other bioactive substances, from alginate hydrogels is controlled by hydrogel formulation to present the pharmaceutical compositions in a spatially and temporally controlled manner. This controlled release eliminates systemic side effects and the need for multiple injections. 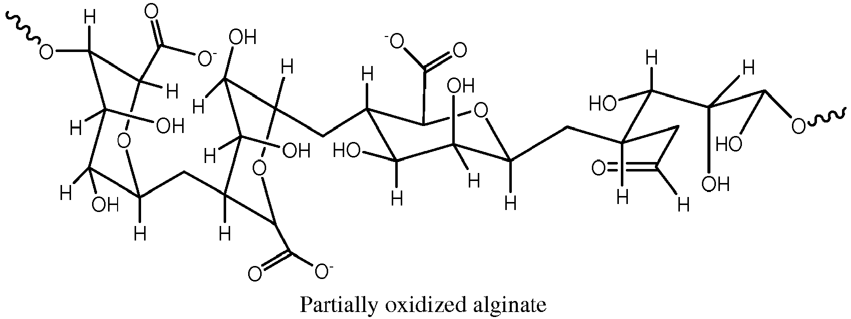 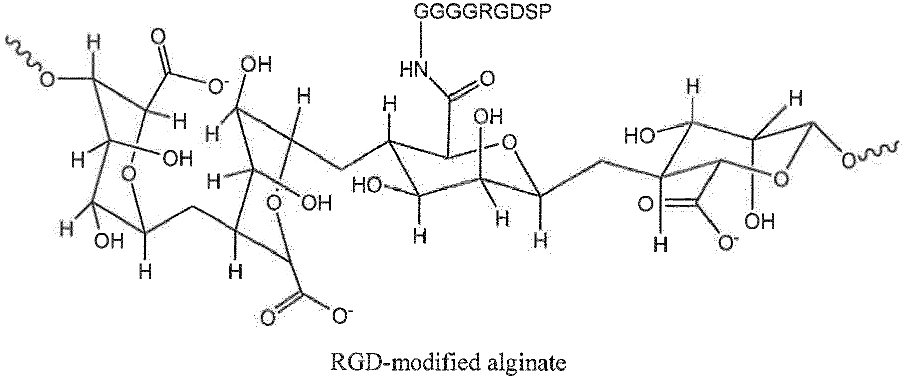 (The peptide sequence GGGGRGDSP corresponds to SEQ ID NO: 26)

The devices, e.g., containing hydrogels, are fabricated from a variety of synthetic polymers and naturally-occurring polymers such as, but not limited to, collagen, fibrin, hyaluronic acid, agarose, self-assembling synthetic peptides, and laminin-rich gels. One preferred material for the hydrogel is alginate or modified alginate material. Alginate molecules are comprised of (1-4)-linked β-D-mannuronic acid (M units) and α L-guluronic acid (G units) monomers, which can vary in proportion and sequential distribution along the polymer chain. Alginate polysaccharides are polyelectrolyte systems which have a strong affinity for divalent cations (*e.g.,* Ca⁺², Mg⁺², Ba⁺²) and form stable hydrogels when exposed to these molecules. *See* Martinsen A., et al., Biotech. & Bioeng., 33 (1989) 79-89.) For example, calcium cross-linked alginate hydrogels are useful for the methods described herein. For example, the polymers, e.g., alginates, of the hydrogel are 0-100% crosslinked, e.g., at least 1%, 5%, 10%, 20%, 30%, 40%, 50%, 60%, 70%, 80%, 90%, or more, crosslinked. In other embodiments, the polymers, e.g., alginates, of the hydrogel are not crosslinked. In some examples, the polymers, e.g., alginates, of the hydrogel contain less than 50%, e.g., less than 50%, 40%, 30%, 20%, 10%, 50%, 2%, 1%, or less, crosslinking.

In some embodiments, the hydrogel comprises polymers that are modified, e.g., sites on the polymer molecule are modified with a methacrylic acid group (methacrylate (MA)) or an acrylic acid group (acrylate). Exemplary modified hydrogels/cryogels are MA-alginate (methacrylated alginate) or MA-gelatin. In the case of MA-alginate or MA-gelatin, 50% corresponds to the degree of methacrylation of alginate or gelatin. This means that every other repeat unit contains a methacrylated group. The degree of methacrylation can be varied from 1% to 90%. Above 90%, the chemical modification may reduce solubility of the polymer water-solubility.

Polymers can also be modified with acrylated groups instead of methacrylated groups. The product would then be referred to as an acrylated-polymer. The degree of methacrylation (or acrylation) can be varied for most polymers. However, some polymers (*e.g.* PEG) maintain their water-solubility properties even at 100% chemical modification. After crosslinking, polymers normally reach near complete methacrylate group conversion indicating approximately 100% of cross-linking efficiency. For example, the polymers in the hydrogel are 50-100% crosslinked (covalent bonds). The extent of crosslinking correlates with the durability of the hydrogel. Thus, a high level of crosslinking (90-100%) of the modified polymers is desirable.

An exemplary device utilizes an alginate or other polysaccharide of a relatively low molecular weight, preferably of size which, after dissolution, is at the renal threshold for clearance by humans, *e.g*., the alginate or polysaccharide is reduced to a molecular weight of 1000 to 80,000 daltons. Prefereably, the molecular mass is 1000 to 60,000 daltons, particularly preferably 1000 to 50,000 daltons. It is also useful to use an alginate material of high guluronate content since the guluronate units, as opposed to the mannuronate units, provide sites for ionic crosslinking through divalent cations to gel the polymer. U.S. Patent Number 6,642,363 discloses methods for making and using polymers containing polysachharides such as alginates or modified alginates.

Useful polysaccharides other than alginates include agarose and microbial polysaccharides such as those listed in the table below.

### Polysaccharide Hydrogel Materials

| Polymers^{a} | Structure |
|---|---|
| Fungal | |
| Pullulan (N) | 1,4-; 1,6-α-D-Glucan |
| Scleroglucan (N) | 1,3;1,6- α -D-Glucan |
| Chitin (N) | 1,4-β-D-Acetyl Glucosamine |
| Chitosan (C) | 1,4- β.-D-N-Glucosamine |
| Elsinan (N) | 1,4-;1,3- α -D-Glucan |
| Bacterial | |
| Xanthan gum (A) | 1,4- β.-D-Glucan with D-mannose; D-glucuronic Acid as side groups |
| Curdlan (N) | 1,3- β.-D-Glucan (with branching) |
| Dextran (N) | 1,6- α -D-Glucan with some 1,2;1,3-; |
| | 1,4-α- linkages |
| Gellan (A) | 1,4- β.-D-Glucan with rhamose, D-glucuronic acid |
| Levan (N) | 2,6- β -D-Fructan with some β -2,1-branching |
| Emulsan (A) | Lipoheteropolysaccharide |
| Cellulose (N) | 1,4- β-D-Glucan |

| | |
|---|---|
| ^{a} N-neutral, A = anionic and C=cationic. | |

The hydrogels used in the refillable drug delivery systems of the invention are porous or non-porous. For example, the hydrogels are nanoporous having a diameter of less than about 10 nm; microporous wherein the diameter of the pores are preferably in the range of about 100 nm-20 µm; or macroporous wherein the diameter of the pores are greater than about 20 µm, more preferably greater than about 100 µm and even more preferably greater than about 400 µm. In one example, the hydrogel is macroporous with aligned pores of about 400-500 µm in diameter. Methods of preparing porous hydrogel products are known in the art. (See, e.g., U.S. Pat. No. 6,511,650.

The device, e.g., hydrogel, structure is constructed out of a number of different rigid, semi-rigid, flexible, gel, self-assembling, liquid crystalline, or fluid compositions such as peptide polymers, proteins, polysaccharides, synthetic polymers, hydrogel materials, organogel materials, ceramics (*e.g*., calcium phosphate or hydroxyapatite), proteins, glycoproteins, proteoglycans, metals and metal alloys. The compositions are assembled into hydrogels using methods known in the art, e.g., injection molding, lyophillization of preformed structures, printing, self-assembly, phase inversion, solvent casting, melt processing, gas foaming, fiber forming/processing, particulate leaching or a combination thereof. The assembled devices are then implanted or administered to the body of an individual to be treated.

The device is assembled *in vivo* in several ways. The hydrogel is made from a gelling material, which is introduced into the body in its ungelled form where it gels *in situ.* Exemplary methods of delivering device components to a site at which assembly occurs include injection through a needle or other extrusion tool, spraying, painting, or methods of deposit at a tissue site, *e.g*., delivery using an application device inserted through a cannula. In one example, the ungelled or unformed hydrogel material is mixed with pharmaceutical compositions prior to introduction into the body or while it is introduced. The resultant *in vivo*/*in situ* assembled device, e.g., hydrogel, contains a mixture of these pharmaceutical composition(s).

*In situ* assembly of the device, e.g., hydrogel, occurs as a result of spontaneous association of polymers or from synergistically or chemically catalyzed polymerization. Synergistic or chemical catalysis is initiated by a number of endogenous factors or conditions at or near the assembly site, *e.g.,* body temperature, ions or pH in the body, or by exogenous factors or conditions supplied by the operator to the assembly site, e.g., photons, heat, electrical, sound, or other radiation directed at the ungelled material after it has been introduced. The energy is directed at the hydrogel material by a radiation beam or through a heat or light conductor, such as a wire or fiber optic cable or an ultrasonic transducer. Alternatively, a shear-thinning material, such as an ampliphile, is used which re-cross links after the shear force exerted upon it, for example by its passage through a needle, has been relieved.

In some embodiments, the hydrogel is injectable. For example, the hydrogels are created outside of the body as macroporous scaffolds. The hydrogels can be injected into the body because the pores collapse and the gel becomes very small and can fit through a needle. See, e.g., WO 12/149358; and Bencherif et al. Proc. Natl. Acad. Sci. USA 109.48(2012):19590-5.

Suitable hydrogels for both *in vivo* and *ex vivo* assembly of hydrogel devices are well known in the art and described, e.g., in Lee et al., 2001, Chem. Rev. 7:1869-1879. The peptide amphiphile approach to self-assembly assembly is described, e.g., in Hartgerink et al., 2002, Proc. Natl. Acad. Sci. U. S. A. 99:5133-5138. A method for reversible gellation following shear thinning is exemplied in Lee et al., 2003, Adv. Mat. 15:1828-1832.

A multiple compartment device is assembled *in vivo* by applying sequential layers of similarly or differentially doped gel or other scaffold material to the target site. For example, the device is formed by sequentially injecting the next, inner layer into the center of the previously injected material using a needle, forming concentric spheroids. Non-concentric compartments are formed by injecting material into different locations in a previously injected layer. A multi-headed injection device extrudes compartments in parallel and simultaneously. The layers are made of similar or different hydrogel compositions differentially doped with pharmaceutical compositions. Alternatively, compartments self-organize based on their hydro-philic/phobic characteristics or on secondary interactions within each compartment.

In certain situations, a device containing compartments with distinct chemical and/or physical properties is useful. A compartmentalized device is designed and fabricated using different compositions or concentrations of compositions for each compartment.

Alternatively, the compartments are fabricated individually, and then adhered to each other *(e.g.,* a "sandwich" with an inner compartment surrounded on one or all sides with the second compartment). This latter construction approach is accomplished using the intrinsic adhesiveness of each layer for the other, diffusion and interpenetration of polymer chains in each layer, polymerization or cross-linking of the second layer to the first, use of an adhesive *(e.g.,* fibrin glue), or physical entrapment of one compartment in the other. The compartments self-assemble and interface appropriately, either *in vitro* or *in vivo,* depending on the presence of appropriate precursors (*e.g*., temperature sensitive oligopeptides, ionic strength sensitive oligopeptides, block polymers, cross-linkers and polymer chains (or combinations thereof).

Alternatively, the compartmentalized device is formed using a printing technology. Successive layers of a hydrogel precursor doped with pharmaceutical compositions is placed on a substrate then cross linked, for example by self-assembling chemistries. When the cross linking is controlled by chemical-, photo- or heat-catalyzed polymerization, the thickness and pattern of each layer is controlled by a masque, allowing complex three dimensional patterns to be built up when un-cross-linked precursor material is washed away after each catalyzation. (WT Brinkman et al., Photo-cross-linking of type 1 collagen gels in the presence of smooth muscle cells: mechanical properties, cell viability, and function. Biomacromolecules, 2003 Jul-Aug;4(4): 890-895.; W. Ryu et al., The construction of three-dimensional micro-fluidic scaffolds of biodegradable polymers by solvent vapor based bonding of micro-molded layers. Biomaterials, 2007 Feb;28(6): 1174-1184; Wright, Paul K. (2001). 21st Century manufacturing. New Jersey: Prentice-Hall Inc.) Complex, multicompartment layers are also built up using an inkjet device which "paints" different doped-scaffold precursors on different areas of the substrate. Julie Phillippi (Carnegie Mellon University) presentation at the annual meeting of the American Society for Cell Biology on December 10, 2006; Print me a heart and a set of arteries, Aldhouse P., New Scientist 13 April 2006 Issue 2547 p 19.; Replacement organs, hot off the press, C. Choi, New Scientist, 25 Jan 2003, v2379. These layers are built-up into complex, three dimensional compartments. The device is also built using any of the following methods: Jetted Photopolymer, Selective Laser Sintering, Laminated Object Manufacturing, Fused Deposition Modeling, Single Jet Inkjet, Three Dimensional Printing, or Laminated Object Manufacturing.

In some embodiments, a stationary device described herein comprises a cancer chemotherapeutic-delivering gel or wafer; a growth factor releasing gel (e.g., to enhance wound healing); or a drug-eluting stent (*e.g*., that prevents thrombosis or restenosis). In other embodiments, a hydrogel device described herein is incorporated into or onto (e.g., adhered to the surface of, imbedded in, mixed with, or coated with) a stationary device, such as a gel, wafer, stent, or other implant. In some cases, a device (e.g., implanted or injected device) is modified with a target recognition molecule capable of recognizing and binding target molecules (e.g., small molecules) circulating in the body. Accumulated target molecules (e.g., small molecules) bound by the device (e.g., implant) can function in bound form or be released over time, permitting controlled drug dosing, e.g., at the device site. In one example, bioorthogonal click chemistry, as described herein, is utilized to accumulate small molecules at a specific site in a subject, e.g., a site of injury.

The drug payloads or refills described herein comprise systemic drugs, such as chemotherapeutic agents, antibiotics (e.g., vancomycin). In some examples, when the systemic drug is not used in combination with a drug eluting device described herein, the systemic drug (e.g., small molecule) leads to systemic toxicity. For example, toxic molecules may be intended to target a tumor, but end up targeting an area of wound healing. The devices and systems herein allow for more efficient targeting of disease sites. When used in combination with a drug eluting device described herein, the systemic drug is targeted to a localized specific site in the body and general systemic toxicity is avoided. The refill process is achieved without the need for a second or subsequent invasive intervention, e.g., implanting a device into a body.

For example, in addition to direct treatment of tumors, the devices and systems described herein are useful to target tumor sites after biopsy or after tumor resection. Alternatively, the devices and systems are useful to treat other disease sites, such as wound healing or inflammatory sites. The drug targeting capabilities of the invention are also useful to deliver drugs to drug-eluting vascular stents and vascular grafts, ocular drug delivery, or to target antibiotics to sites where an implant is infected. The devices and systems are useful for numerous applications, e.g., in which a device is implanted/injected at a local site in a body and systemic dosing of a toxic drug, e.g., small molecule, is needed.

The release profiles of pharmaceutical compositions from hydrogel devices is controlled by both factor diffusion and polymer degradation, the dose of the pharmaceutical composition loaded in the system, and the composition of the polymer. Similarly, the range of action (tissue distribution) and duration of action, or spatiotemporal gradients of the released pharmaceutical compositions are regulated by these variables. The diffusion and degradation of the pharmaceutical composition in the tissue of interest is optionally regulated by chemically modifying the pharmaceutical compositions (*e.g*., PEGylating polypeptides). In both cases, the time frame of release determines the time over which effective delivery by the device is desired.

The pharmaceutical compositions are added to the drug delivery devices using known methods including surface absorption, physical immobilization, *e.g*., using a phase change to entrap the substance in the hydrogel material. For example, a pharmaceutical compositions, such as a polypeptide, *e.g*., growth factor, is mixed with the hydrogel material while it is in an aqueous or liquid phase, and after a change in environmental conditions (*e.g*., pH, temperature, ion concentration), the liquid gels or solidifies thereby entrapping the pharmaceutical composition. Alternatively, covalent coupling, *e.g*., using alkylating or acylating agents, is used to provide a stable, long term presentation of a pharmaceutical composition on the hydrogel in a defined conformation. Exemplary reagents for covalent coupling of pharmaceutical compositions, such as peptide/proteins, are provided in the table below.

### Methods to covalently couple peptides/proteins to polymers

| Functional Group of Polymer | Coupling reagents and cross-linker | Reacting groups on proteins/peptides |
|---|---|---|
| -OH | Cyanogen bromide (CNBr) Cyanuric chloride 4-(4,6-Dimethoxy-1,3,5-triazin-2-yl)-4-methyl-morpholinium chloride (DMT-MM) | -NH₂ |
| -NH₂ | Diisocyanate compounds Diisothoncyanate compounds Glutaraldehyde Succinic anhydride | -NH₂ _-OH |
| -NH₂ | Nitrous Acid Hydrazine + nitrous acid | -NH₂ -SH -Ph-OH |
| -NH₂ | Carbodiimide compounds (*e.g*., EDC, | -COOH |
| | DCC)[a] DMT-MM | |
| Azide | Copper catalyst | -alkyne |
| Azide | None | -DBCO |
| Tetrazine | none | -TCO |
| -NH₂ | Sortase enzyme | peptide |
| -O-NH₂ | Pyridoxamine | N-terminus |
| -COOH | Thionyl chloride N-hydroxysuccinimide N-hydroxysulfosuccinimide + EDC | -NH₂ |
| -SH | Disulfide compound | -SH |
| Maleimide | None | -SH |
| iodoacetate | none | SH |

| | | |
|---|---|---|
| [a] EDC: 1-ethyl-3-(3-dimethylaminopropyl) carbodiimide hydrochloride; DCC: dicyclohexylcarbodiimide | | |

For efficient homing to target areas, drug payloads must circulate in the blood for sufficiently long periods of time. One polymer capable of both local controlled drug release and blood-based targeting is alginate, a naturally occurring polysaccharide comprised of α-L-guluronic and β-D-mannuronic acid sugar residues. See Augst A, Kong H, & Mooney D (2006) Macromolecular bioscience 6(8):623-633. Alginate is widely applied in the pharmaceutical industry as an excipient for drugs and as a wound dressing. See, e.g., Liew C, Chan L, Ching A, & Heng P (2006) International journal of pharmaceutics 309(1-2):25-37; and Matthew I, Browne R, Frame J, & Millar B (1995) Biomaterials 16(4):275-278. Alginate is biocompatible and non-immunogenic and can be gelled under gentle conditions, allowing encapsulation of drugs or biological factors with minimal trauma. Additionally, alginate is readily chemically modified for cell adhesion or as a drug carrier, has tunable degradation rates, and chemically modified forms of alginate are currently used clinically as a drug delivery vehicle for proteins that promote regeneration of mineralized tissue and have been used as a carrier for transplanted cells. See, e.g., Silva E, Kim E-S, Kong H, & Mooney D (2008) Proceedings of the National Academy of Sciences of the United States of America 105(38):14347-14352; Bouhadir K, et al. (2001) Biotechnology progress 17(5):945-950; Bratthall G, et al. (2001) Journal of clinical periodontology 28(10):923-929; Bent A, et al. (2001) Neurourology and urodynamics 20(2):157-165; Yu J, et al. (2010) Biomaterials 31(27):7012-7020; and Zhao L, Weir M, & Xu H (2010) Biomaterials 31(25):6502-6510.

Alginate has previously been reported to have long circulation times in the blood, being detectable in serum for at least one week as well as having PEG-like properties in its ability to increase the circulation time of nanoparticles following conjugation. Al-Shamkhani A & Duncan R (1995) Journal of bioactive and compatible polymers 10(1):4-13; and Kodiyan A, Silva E, Kim J, Aizenberg M, & Mooney D (2012) ACS nano 6(6):4796-4805. The circulation lifetime primarily depends on the rate of excretion into the urine (small molecules), uptake by the mononuclear phagocyte system (MPS) in the liver and spleen (particles), and drug stability. For example, the alginate used in the studies herein had a MW of 280 kDa (unoxidized) or 200 KDa (5% oxidized) and a hydrodynamic radius of 44 nm and 17 nm, respectively. Because of these properties, alginate will behave in these systems similarly to nanoparticles, and will enhance drug circulation time and reduce drug permeability in off-target tissues. The results of these studies indicate that the free alginate strands have a blood circulation lifetime of about two weeks. Imaging of individual organs revealed accumulation in the lungs, consistent with many nanoparticles, as well as a tendency to undergo significant clearance into the MPS-related organs, and to a lesser extent in the kidneys (Fig 6D), demonstrating that all of these organs contribute to alginate clearance.

The results presented herein demonstrate that DNA-mediated doxorubicin refilling of alginate gels dramatically inhibited tumor growth in a xenograft tumor model. The homing aspect of the devices and methods of the invention can overcome toxicity related to the non-specific targeting of all permeable tissues by nanoparticles and advanced drug delivery systems that take advantage of the EPR effect. See, e.g., Park J-H, et al. (2010) Advanced materials (Deerfield Beach, Fla.) 22(8):880-885; Park J-H, et al. (2010) Proceedings of the National Academy of Sciences of the United States of America 107(3):981-986; Ruoslahti E, Bhatia S, & Sailor M (2010) The Journal of cell biology 188(6):759-768; Simberg D, et al. (2007) Proceedings of the National Academy of Sciences of the United States of America 104(3):932-936; von Maltzahn G, et al. (2011) Nature materials 10(7):545-552; Perrault S & Chan W (2010) Proceedings of the National Academy of Sciences of the United States of America 107(25):11194-11199.

In addition to direct treatment of tumors, the devices and methods described herein are used to target tumor sites after biopsy or tumor resection.

For example, the invention includes the refillable drug delivery system according to the invention for use in (1) maintaining or reducing the size of a tumor in a subject in need thereof, or (2) reducing cancer progression in the subject,
wherein the pharmaceutical composition comprises an anti-cancer drug; optionally wherein the drug refill is refilled a plurality of times by administering the drug refill; optionally wherein the drug refill is administered to the subject orally, intraperitoneally, intravenously, or intra-arterially;
thereby maintaining or reducing the size of the tumor in the subject or reducing cancer progression in the subject.

In some embodiments, the size of the tumor is maintained, i.e., the size of the tumor after administration of the anti-cancer drug remains within 10% of the size of the tumor prior to administration of the anti-cancer drug. In some examples, the size of the tumor is reduced by at least 1.5-fold, e.g., at least 1.5-fold, 2-fold, 3-fold, 4-fold, 5-fold, 6-fold, 7-fold, 8-fold, 9-fold, 10-fold, 20-fold, 50-fold, 100-fold, or more compared to the size of the tumor prior to administration of the device and/or a refill. In some cases, the systems of the invention completely eliminate a tumor in the subject. In some examples, the size of a tumor is measured by the area or diameter of the tumor, e.g., on an X-ray, PET scan, MRI, or CAT scan.

In other examples, the methods described herein are effective to slow cancer progression and/or tumor growth. For example, a tumor growth rate is reduced by at least 1.5-fold, e.g., at least 1.5-fold, 2-fold, 3-fold, 4-fold, 5-fold, 6-fold, 7-fold, 8-fold, 9-fold, 10-fold, 20-fold, 50-fold, 100-fold, or more, compared to the growth rate prior to administration of a device or refill described herein. In some cases, the methods described herein stop cancer progression and/or tumor growth completely.

In some cases, an anti-cancer drug includes a small molecule, a peptide or polypeptide, a protein or fragment thereof (e.g., an antibody or fragment thereof), or a nucleic acid.

Exemplary anti-cancer drugs include but are not limited to Abiraterone Acetate, Abitrexate (Methotrexate), Abraxane (Paclitaxel Albumin-stabilized Nanoparticle Formulation), ABVD, ABVE, ABVE-PC, AC, AC-T, Adcetris (Brentuximab Vedotin), ADE, Ado-Trastuzumab Emtansine, Adriamycin (Doxorubicin Hydrochloride), Adrucil (Fluorouracil), Afatinib Dimaleate, Afinitor (Everolimus), Aldara (Imiquimod), Aldesleukin, Alemtuzumab, Alimta (Pemetrexed Disodium), Aloxi (Palonosetron Hydrochloride), Ambochlorin (Chlorambucil), Amboclorin (Chlorambucil), Aminolevulinic Acid, Anastrozole, Aprepitant, Aredia (Pamidronate Disodium), Arimidex (Anastrozole), Aromasin (Exemestane), Arranon (Nelarabine), Arsenic Trioxide, Arzerra (Ofatumumab), Asparaginase Erwinia chrysanthemi, Avastin (Bevacizumab), Axitinib, Azacitidine, BEACOPP, Bendamustine Hydrochloride, BEP, Bevacizumab, Bexarotene, Bexxar (Tositumomab and I 131 Iodine Tositumomab), Bicalutamide, Bleomycin, Bortezomib, Bosulif (Bosutinib), Bosutinib, Brentuximab Vedotin, Busulfan, Busulfex (Busulfan), Cabazitaxel, Cabozantinib-S-Malate, CAF, Campath (Alemtuzumab), Camptosar (Irinotecan Hydrochloride), Capecitabine, CAPOX, Carboplatin, Carboplatin-Taxol, Carfilzomib, Casodex (Bicalutamide), CeeNU (Lomustine), Cerubidine (Daunorubicin Hydrochloride), Cervarix (Recombinant HPV Bivalent Vaccine), Cetuximab, Chlorambucil, Chlorambucil-Prednisone, CHOP, Cisplatin, Clafen (Cyclophosphamide), Clofarabine, Clofarex (Clofarabine), Clolar (Clofarabine), CMF, Cometriq (Cabozantinib-S-Malate), COPP, COPP-ABV, Cosmegen (Dactinomycin), Crizotinib, CVP, Cyclophosphamide, Cyfos (Ifosfamide), Cytarabine, Cytarabine, Liposomal, Cytosar-U (Cytarabine), Cytoxan (Cyclophosphamide), Dabrafenib, Dacarbazine, Dacogen (Decitabine), Dactinomycin, Dasatinib, Daunorubicin Hydrochloride, Decitabine, Degarelix, Denileukin Diftitox, Denosumab, DepoCyt (Liposomal Cytarabine), DepoFoam (Liposomal Cytarabine), Dexrazoxane Hydrochloride, Docetaxel, Doxil (Doxorubicin Hydrochloride Liposome), Doxorubicin Hydrochloride, Doxorubicin Hydrochloride Liposome, Dox-SL (Doxorubicin Hydrochloride Liposome), DTIC-Dome (Dacarbazine), Efudex (Fluorouracil), Elitek (Rasburicase), Ellence (Epirubicin Hydrochloride), Eloxatin (Oxaliplatin), Eltrombopag Olamine, Emend (Aprepitant), Enzalutamide, Epirubicin Hydrochloride, EPOCH, Erbitux (Cetuximab), Eribulin Mesylate, Erivedge (Vismodegib), Erlotinib Hydrochloride, Erwinaze (Asparaginase Erwinia chrysanthemi), Etopophos (Etoposide Phosphate), Etoposide, Etoposide Phosphate, Evacet (Doxorubicin Hydrochloride Liposome), Everolimus, Evista (Raloxifene Hydrochloride), Exemestane, Fareston (Toremifene), Faslodex (Fulvestrant), FEC, Femara (Letrozole), Filgrastim, Fludara (Fludarabine Phosphate), Fludarabine Phosphate, Fluoroplex (Fluorouracil), Fluorouracil, Folex (Methotrexate), Folex PFS (Methotrexate), Folfiri, Folfiri-Bevacizumab, Folfiri-Cetuximab, Folfirinox, Folfox, Folotyn (Pralatrexate), FU-LV, Fulvestrant, Gardasil (Recombinant HPV Quadrivalent Vaccine), Gazyva (Obinutuzumab), Gefitinib, Gemcitabine Hydrochloride, Gemcitabine-Cisplatin, Gemcitabine-Oxaliplatin, Gemtuzumab Ozogamicin, Gemzar (Gemcitabine Hydrochloride), Gilotrif (Afatinib Dimaleate), Gleevec (Imatinib Mesylate), Glucarpidase, Goserelin Acetate, Halaven (Eribulin Mesylate), Herceptin (Trastuzumab), HPV Bivalent Vaccine, Recombinant, HPV Quadrivalent Vaccine, Recombinant, Hycamtin (Topotecan Hydrochloride), Hyper-CVAD, Ibritumomab Tiuxetan, Ibrutinib, ICE, Iclusig (Ponatinib Hydrochloride), Ifex (Ifosfamide), Ifosfamide, Ifosfamidum (Ifosfamide), Imatinib Mesylate, Imbruvica (Ibrutinib), Imiquimod, Inlyta (Axitinib), Intron A (Recombinant Interferon Alfa-2b), Iodine 131 Tositumomab and Tositumomab, Ipilimumab, Iressa (Gefitinib), Irinotecan Hydrochloride, Istodax (Romidepsin), Ixabepilone, Ixempra (Ixabepilone), Jakafi (Ruxolitinib Phosphate), Jevtana (Cabazitaxel), Kadcyla (Ado-Trastuzumab Emtansine), Keoxifene (Raloxifene Hydrochloride), Kepivance (Palifermin), Kyprolis (Carfilzomib), Lapatinib Ditosylate, Lenalidomide, Letrozole, Leucovorin Calcium, Leukeran (Chlorambucil), Leuprolide Acetate, Levulan (Aminolevulinic Acid), Linfolizin (Chlorambucil), LipoDox (Doxorubicin Hydrochloride Liposome), Liposomal Cytarabine, Lomustine, Lupron (Leuprolide Acetate), Lupron Depot (Leuprolide Acetate), Lupron Depot-Ped (Leuprolide Acetate), Lupron Depot-3 Month (Leuprolide Acetate), Lupron Depot-4 Month (Leuprolide Acetate), Marqibo (Vincristine Sulfate Liposome), Matulane (Procarbazine Hydrochloride), Mechlorethamine Hydrochloride, Megace (Megestrol Acetate), Megestrol Acetate, Mekinist (Trametinib), Mercaptopurine, Mesna, Mesnex (Mesna), Methazolastone (Temozolomide), Methotrexate, Methotrexate LPF (Methotrexate), Mexate (Methotrexate), Mexate-AQ (Methotrexate), Mitomycin C, Mitozytrex (Mitomycin C), MOPP, Mozobil (Plerixafor), Mustargen (Mechlorethamine Hydrochloride), Mutamycin (Mitomycin C), Myleran (Busulfan), Mylosar (Azacitidine), Mylotarg (Gemtuzumab Ozogamicin), Nanoparticle Paclitaxel (Paclitaxel Albumin-stabilized Nanoparticle Formulation), Navelbine (Vinorelbine Tartrate), Nelarabine, Neosar (Cyclophosphamide), Neupogen (Filgrastim), Nexavar (Sorafenib Tosylate), Nilotinib, Nolvadex (Tamoxifen Citrate), Nplate (Romiplostim), Obinutuzumab, Ofatumumab, Omacetaxine Mepesuccinate, Oncaspar (Pegaspargase), Ontak (Denileukin Diftitox), OEPA, OPPA, Oxaliplatin, Paclitaxel, Paclitaxel Albumin-stabilized Nanoparticle Formulation, Palifermin, Palonosetron Hydrochloride, Pamidronate Disodium, Panitumumab, Paraplat (Carboplatin), Paraplatin (Carboplatin), Pazopanib Hydrochloride, Pegaspargase, Peginterferon Alfa-2b, PEG-Intron (Peginterferon Alfa-2b), Pemetrexed Disodium, Perjeta (Pertuzumab), Pertuzumab, Platinol (Cisplatin), Platinol-AQ (Cisplatin), Plerixafor, Pomalidomide, Pomalyst (Pomalidomide), Ponatinib Hydrochloride, Pralatrexate, Prednisone, Procarbazine Hydrochloride, Proleukin (Aldesleukin), Prolia (Denosumab), Promacta (Eltrombopag Olamine), Provenge (Sipuleucel-T), Purinethol (Mercaptopurine), Radium 223 Dichloride, Raloxifene Hydrochloride, Rasburicase, R-CHOP, R-CVP, Recombinant HPV Bivalent Vaccine, Recombinant HPV Quadrivalent Vaccine, Recombinant Interferon Alfa-2b, Regorafenib, Revlimid (Lenalidomide), Rheumatrex (Methotrexate), Rituxan (Rituximab), Rituximab, Romidepsin, Romiplostim, Rubidomycin (Daunorubicin Hydrochloride), Ruxolitinib Phosphate, Sclerosol Intrapleural Aerosol (Talc), Sipuleucel-T, Sorafenib Tosylate, Sprycel (Dasatinib), Stanford V, Sterile Talc Powder (Talc), Steritalc (Talc), Stivarga (Regorafenib), Sunitinib Malate, Sutent (Sunitinib Malate), Sylatron (Peginterferon Alfa-2b), Synovir (Thalidomide), Synribo (Omacetaxine Mepesuccinate), Tafinlar (Dabrafenib), Talc, Tamoxifen Citrate, Tarabine PFS (Cytarabine), Tarceva (Erlotinib Hydrochloride), Targretin (Bexarotene), Tasigna (Nilotinib), Taxol (Paclitaxel), Taxotere (Docetaxel), Temodar (Temozolomide), Temozolomide, Temsirolimus, Thalidomide, Thalomid (Thalidomide), Toposar (Etoposide), Topotecan Hydrochloride, Toremifene, Torisel (Temsirolimus), Tositumomab and 1131 Iodine Tositumomab, Totect (Dexrazoxane Hydrochloride), Trametinib, Trastuzumab, Treanda (Bendamustine Hydrochloride), Trisenox (Arsenic Trioxide), Tykerb (Lapatinib Ditosylate), Vandetanib, VAMP, Vectibix (Panitumumab), VeIP, Velban (Vinblastine Sulfate), Velcade (Bortezomib), Velsar (Vinblastine Sulfate), Vemurafenib, VePesid (Etoposide), Viadur (Leuprolide Acetate), Vidaza (Azacitidine), Vinblastine Sulfate, Vincasar PFS (Vincristine Sulfate), Vincristine Sulfate, Vincristine Sulfate Liposome, Vinorelbine Tartrate, Vismodegib, Voraxaze (Glucarpidase), Vorinostat, Votrient (Pazopanib Hydrochloride), Wellcovorin (Leucovorin Calcium), Xalkori (Crizotinib), Xeloda (Capecitabine), Xelox, Xgeva (Denosumab), Xofigo (Radium 223 Dichloride), Xtandi (Enzalutamide), Yervoy (Ipilimumab), Zaltrap (Ziv-Aflibercept), Zelboraf (Vemurafenib), Zevalin (Ibritumomab Tiuxetan), Zinecard (Dexrazoxane Hydrochloride), Ziv-Aflibercept, Zoladex (Goserelin Acetate), Zoledronic Acid, Zolinza (Vorinostat), Zometa (Zoledronic Acid), and Zytiga (Abiraterone Acetate).

In some embodiments, the refill is administered to the subject at a site located away from the administered device, e.g., at a site that is located at least 5 cm (e.g., at least 5 cm, 10 cm, 15 cm, 20 cm, 30 cm, 40 cm, 50 cm, 1 m, 1.5 m, 2 m, 2.5 m, 3 m, or more) away from the center of the device.

For example a tumor is derived from one or more cancers described below.

In some examples, the size of the tumor is reduced by at least 1.5-fold, e.g., at least 1.5-fold, 2-fold, 3-fold, 4-fold, 5-fold, 6-fold, 7-fold, 8-fold, 9-fold, 10-fold, 20-fold, 50-fold, 100-fold, or more compared to the size of the tumor prior to administration of the device and/or a refill. In some cases, the systems for use of the invention completely eliminate a tumor in the subject.

In other examples, the methods described herein are effective to slow cancer progression and/or tumor growth. For example, a tumor growth rate is reduced by at least 1.5-fold, e.g., at least 1.5-fold, 2-fold, 3-fold, 4-fold, 5-fold, 6-fold, 7-fold, 8-fold, 9-fold, 10-fold, 20-fold, 50-fold, 100-fold, or more, compared to the growth rate prior to administration of a device or refill described herein. In some cases, the methods described herein stop cancer progression and/or tumor growth completely.

In some embodiments, the subject suffers from a cancer. Exemplary cancers include melanoma, a central nervous system (CNS) cancer, a CNS germ cell tumor, a lung cancer, leukemia, multiple myeloma, a renal cancer, a malignant glioma, a medulloblatoma, a breast cancer, an ovarian cancer, a prostate cancer, a bladder cancer, a fibrosarcoma, a pancreatic cancer, a gastric cancer, a head and neck cancer, or a colorectal cancer. For example, a cancer cell is derived from a solid cancer or hematological cancer. The hematological cancer is, e.g., a leukemia or a lymphoma. A leukemia is acute lymphoblastic leukemia (ALL), acute myelogenous leukemia (AML), chronic lymphocytic leukemia (CLL), small lymphocytic lymphoma (SLL), chronic myelogenous leukemia (CML), or acute monocytic leukemia (AMoL). A lymphoma is follicular lymphoma, Hodgkin's lymphoma (e.g., Nodular sclerosing subtype, mixed-cellularity subtype, lymphocyte-rich subtype, or lymphocyte depleted subtype), or Non-Hodgkin's lymphoma. Exemplary solid cancers include but are not limited to melanoma (*e.g*., unresectable, metastatic melanoma), renal cancer (e.g., renal cell carcinoma), prostate cancer (e.g., metastatic castration resistant prostate cancer), ovarian cancer (e.g., epithelial ovarian cancer, such as metastatic epithelial ovarian cancer), breast cancer (e.g., triple negative breast cancer), and lung cancer (e.g., non-small cell lung cancer).

For example, an anti-cancer drug for use in the devices/methods described herein is doxorubicin.

In some cases, refilling of drug delivery devices is used in a permeable tissue, e.g., at/near the site of a wound, or at/near an inflammatory site. For example, the methods described herein are used to refill drug delivery devices placed in areas of ischemia, such as tissues affected by peripheral artery disease (PAD) or myocardial tissues after a myocardial infarction. Drugs used for refilling the delivery device include but are not limited to polypeptides/peptides, proteins or fragments thereof (e.g., antibodies or fragments thereof), nucleic acids, polysaccharides, small molecules, or lipids, e.g., growth factors. For example, a drug used herein promotes angiogenesis and blood vessel growth, promotes maturation and/or remodeling of existing vessels, and/or promotes muscle regeneration. In some exmaples, a drug used herein that promotes muscle regeneration includes hepatocyte growth factor (HGF), basic fibroblast growth factor (bFGF), insulin-like growth factor-1 (IGF-1), nerve growth factor (NGF), leukemia inhibitor factor (LIF), and/or platelet-derived growth factor (PDGF-BB), or combinations thereof.

In some examples, the invention provides a refillable drug delivery system according to the invention for use in promoting wound healing in a subject in need thereof, wherein the pharmaceutical composition promotes angiogenesis and/or maturation or remodeling of an existing blood vessel; wherein
i) the drug delivery device is refilled by administering the drug refill to the subject orally, intraperitoneally, intravenously, or intra-arterially;
ii) optionally repeating step i);
thereby promoting wound healing in the subject.

In some embodiments, the pharmaceutical composition comprises a protein or fragment thereof (e.g., antibody or fragment thereof), peptide/polypeptide, nucleic acid, polysaccharide, or small molecule. In some examples, the agent comprises a protein or fragment thereof, e.g., a growth factor or angiogenic factor, such as vascular endothelial growth factor (VEGF), e.g., VEGFA, VEGFB, VEGFC, or VEGFD, and/or IGF, e.g., IGF-1, fibroblast growth factor (FGF), angiopoietin (ANG) (e.g., Ang1 or Ang2), matrix metalloproteinase (MMP), delta-like ligand 4 (DLL4), or combinations thereof. In other examples, the agent comprises a protein or fragment thereof that muscle regeneration, e.g., hepatocyte growth factor (HGF), basic fibroblast growth factor (bFGF), insulin-like growth factor-1 (IGF-1), nerve growth factor (NGF), leukemia inhibitor factor (LIF), and/or platelet-derived growth factor (PDGF-BB), or combinations thereof.

In some cases, the device is administered at a site in the subject within or in proximity to (e.g., 10 cm or less from a perimeter of, e.g., 10 cm, 5 cm, 2.5 cm, 1 cm, 5 mm, 2.5 mm, 1 mm, or less) an ischemic tissue.

In some examples, the ischemic tissue is caused by peripheral artery disease (PAD). In other examples, the ischemic tissue is a myocardial tissue affected by a myocardial infarction.

The invention also provides a refillable drug delivery system according to the invention for use in reducing or controlling inflammation in a subject in need thereof, where the pharmaceutical composition comprises an anti-inflammatory agent; wherein
(i) the drug delivery device is refilled by
(ii) administering the drug refill to the subjectorally, intraperitoneally, intravenously, or intra-arterially;
(ii) optionally repeating step (i);
thereby reducing or controlling inflammation in the subject.

In some examples, the device is administered to an inflammatory site in the subject or to a site in proximity to (e.g., 10 cm or less from a perimeter of, e.g., 10 cm, 5 cm, 2.5 cm, 1 cm, 5 mm, 2.5 mm, 1 mm, or less) an inflamed tissue.

In some cases, the inflammation is chronic inflammation, e.g., caused by rheumatoid arthritis.

By controlling inflammation, the use prevents an increase in severity of inflammation in a tissue or prevents an increase in the amount of tissue that is inflamed.

Additionally, devices and methods described herein deliver drugs to blood-based medical devices and refill such medical devices in a non-invasive manner. Exemplary medical devices include but are not limited to drug-eluting vascular stents and vascular grafts.

Local delivery of drugs via catheters and/or stents has been used for treatment of cardiovascular tissues as well as other tissues characterized such as urinary tissues. In such cases, catheters or stents are deployed into bodily lumens. Vascular grafts, stents, and catheters are used to deliver drugs to inhibit blood clotting/coagulation, restenosis of vascular lumens, as well as other drugs to treat adjacent tissues. Catheters, e.g., catheters coated with drug-containing hydrogels, are inserted into a tissue, expanded to make contact with the tissue and to deposit the drug-containing hydrogel, and subsequently removed from the tissue. Stents that are coated with drug-loaded hydrogels are administered to a bodily lumen and deployed to reside in situ. In each case, drug (or cells) exit the hydrogel to confer a clinical benefit. In some cases, the benefit is reduction or inhibition of restenosis, in other cases, other medicaments such as anti-microbial agents diffuse out of the hydrogel (USPN 8,221,783; 8,182,481; 8,157,854;78,133,501;7,794,490; 7.767,219; 7,601,382; 7,517,342; 7,462,165; 7,371,257; 7,066,904; 6, 364,856; 5,954,706; 5,868,719; 5,674,192; 5,588,962; 5,304,121 to treat adjacent or nearby tissues. In addition to depositing hydrogels in vascular lumens, hydrogels are administered subcutaneously, intramuscularly, and into other tissues of the body for reducing tumor burden/cancer treatment (see, e.g., US 8,067,237; US 2013-0202707; and WO 12/167230 wound healing (US 2013-0177536; and US 2011-0117170 and for treatment of ischemic tissue (as described above as well as for peripheral artery disease affecting extremities such as feet, hands, legs and arms).

Over time, the active agent in the initially administered hydrogel is depleted. The systems of the invention are used to recharge, refill, and therefore extend the useful life of the deployed treatment modality.

Devices, e.g., hydrogels, described herein are administered or implanted orally, intraperitoneally, systemically, sub- or trans-cutaneously, as an arterial stent, or surgically. Alternatively, devices, e.g., hydrogels, are administered via injection, e.g., intra-arterially intraperitoneally, or intravenously.

In some examples, the drug delivery system described herein comprises nanoparticles, e.g., calcium phosphate polymer nano-formulations, condensed nucleic acids, or liposomal nucleic acids. For example, bloodborne nanoparticles home to and accumulate in disease tissue, e.g., tumor tissue, and serve as the refillable device. Alternatively, nanoparticles are delivered directly to the disease tissue. Subsequently, drug refills are localized to the nanoparticles in the disease tissue as needed.

A refill and/or pharmaceutical composition described herein is formulated to be compatible with its intended route of administration. Examples of routes of administration include parenteral, *e.g.,* intravenous, intraperitoneal, intradermal, subcutaneous, oral (*e.g.,* inhalation), transdermal (*i.e.,* topical), transmucosal, and rectal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid (EDTA); buffers such as acetates, citrates or phosphates, and agents for the adjustment of tonicity such as sodium chloride or dextrose. The pH can be adjusted with acids or bases, such as hydrochloric acid or sodium hydroxide. The parenteral preparation can be enclosed in ampoules, disposable syringes or multiple dose vials made of glass or plastic.
Devices, e.g., hydrogels, refills, and/or pharmaceutical compositions described herein are suitable for injectable use and include sterile aqueous solutions (where water soluble) or dispersions and sterile powders for the extemporaneous preparation of sterile injectable solutions or dispersion. For intravenous administration, suitable carriers include physiological saline, bacteriostatic water, CREMOPHOR EL™ (BASF, Parsippany, N.J.) or phosphate buffered saline (PBS). In all cases, the composition must be sterile and should be fluid to the extent that easy syringeability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as manitol, sorbitol, sodium chloride in the composition. Prolonged absorption of the injectable compositions can be brought about by including in the composition an agent which delays absorption, for example, aluminum monostearate and gelatin.

Sterile injectable solutions can be prepared by incorporating a pharmaceutical composition in the required amount in an appropriate solvent with one or a combination of ingredients enumerated above, as required, followed by filtered sterilization. Generally, dispersions are prepared by incorporating the active compound into a sterile vehicle that contains a basic dispersion medium and the required other ingredients from those enumerated above. In the case of sterile powders for the preparation of sterile injectable solutions, methods of preparation are vacuum drying and freeze-drying that yields a powder of the active ingredient plus any additional desired ingredient from a previously sterile-filtered solution thereof.

In one embodiment, the pharmaceutical compositions are prepared with carriers that will protect the compound against rapid elimination from the body, such as sustained/controlled release formulations, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art.

For example, the pharmaceutical compositions can be entrapped in microcapsules prepared, for example, by coacervation techniques or by interfacial polymerization, for example, hydroxymethylcellulose or gelatin-microcapsules and poly-(methylmethacrylate) microcapsules, respectively, in colloidal drug delivery systems (for example, liposomes, albumin microspheres, microemulsions, nano-particles, and nanocapsules) or in macroemulsions.

Sustained-release preparations can be prepared. Suitable examples of sustained-release preparations include semipermeable matrices of solid hydrophobic polymers containing the antibody, which matrices are in the form of shaped articles, e.g., films, or microcapsules. Examples of sustained-release matrices include polyesters, hydrogels (for example, poly(2-hydroxyethyl-methacrylate), or poly(vinylalcohol)), polylactides (U.S. Pat. No. 3,773,919), copolymers of L-glutamic acid and γ ethyl-L-glutamate, non-degradable ethylene-vinyl acetate, degradable lactic acid-glycolic acid copolymers such as the LUPRON DEPOT™ (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods.

The materials can also be obtained commercially from Alza Corporation and Nova Pharmaceuticals, Inc. Liposomal suspensions (including liposomes targeted to infected cells with monoclonal antibodies to viral antigens) and can also be used as pharmaceutically acceptable carriers. These can be prepared according to methods known to those skilled in the art, for example, as described in U.S. Patent No. 4,522,811.

It is especially advantageous to formulate oral or parenteral compositions in dosage unit form for ease of administration and uniformity of dosage. Dosage unit form as used herein refers to physically discrete units suited as unitary dosages for the subject to be treated; each unit containing a predetermined quantity of active compound calculated to produce the desired therapeutic effect in association with the required pharmaceutical carrier. The specification for the dosage unit forms are dictated by and directly dependent on the unique characteristics of the active compound and the particular therapeutic effect to be achieved, and the limitations inherent in the art of compounding such an active compound for the treatment of individuals.

In some examples, a pharmaceutical composition described herein is incorporated into or onto the device, e.g., onto the polymer (e.g., hydrogel, such as alginate) of the device. In such cases, 0-100 mg (e.g., 5-100 mg, 10-100 mg, 20-100 mg, 30-100 mg, 40-100 mg, 50-100 mg, 60-100 mg, 70-100 mg, 80-100 mg, 90-100 mg, 1-95 mg, 1-90 mg, 5-95 mg, 5-90 mg, 5-80 mg, 5-70 mg, 5-60 mg, 5-50 mg, 5-40 mg, 5-30 mg, or 5-20 mg) of the pharmaceutical composition is present in the device. For example, the pharmaceutical composition is present in the device at a weight/weight concentration of at least 5% (e.g., at least 5%, 10%, 15%, 20%, 25%, 30%, 35%, 40%, 45%, 50%, 55%, 60%, 65%, 70%, 75%, 80%, 85%, 90%, 95%, 98%, 99%, or more).

In some embodiments, the device, e.g., hydrogel, comprises 0.1 mg to 25 mg pharmaceutical composition per mL of hydrogel, e.g., 0.5 mg to 15 mg, 1 mg to 10 mg, 1 mg to 5 mg, 5 mg to 10 mg, or 1 mg to 3 mg per mL, e.g., about 1.6 mg per mL.

In other examples, a pharmaceutical composition described herein is conjugated to a refill of the invention. For example, the pharmaceutical composition is conjugated to the refill polymer, e.g., alginate strand, at a weight/weight ratio of 1:100 to 100:1, e.g., 1:100, 5:100, 1:10, 1:5, 3:10, 4:10, 1:2, 6:10, 7:10, 8:10, 9:10, 1:1, 2:1, 3:1, 4:1, 5:1, 6:1, 10:1, 20:1, 30:1, 40:1, 50:1, 60:1, 70:1, 80:1, 90:1, or 100:1. In other examples, the pharmaceutical composition is conjugated to the target molecule of the refill, e.g., at a molar ratio of 10:1 to 1:10, eg., 10:1, 9:1, 8:1, 7:1, 6:1, 5:1, 4:1, 3:1, 2:1, 1:1, 1:2, 1:3, 1:4, 1:5, 1:6, 1:7, 1:8, 1:9, or 1:10 pharmaceutical composition:target molecule.

The device is administered to a subject in need thereof. For example, the subject is a mammal, e.g., human, monkey, primate, dog, cat, horse, cow, pig, sheep, or goat. For example, the subject is a human.

In accordance with the methods described herein, a device and/or refill is administered orally, intraperitoneally, intravenously, intraarterially, rectally, buccally, sublingually, intraocularly, intranasally, transdermally, transmucosally, subcutaneously, intramuscularly, via injection, via aerosol-based delivery, or via implantation.

A small molecule is a low molecular weight compound of less than 1000 Daltons, less than 800 Daltons, or less than 500 Daltons.

In some embodiments, a fragment of a protein, antibody, or polypeptide contains 1500 or less, 1250 of less, 1000 or less, 900 or less, 800 or less, 700 or less, 600 or less, 500 or less, 400 or less, 300 or less, 200 or less amino acids. For example, a protein or peptide contains 1500 or less, 1250 of less, 1000 or less, 900 or less, 800 or less, 700 or less, 600 or less, 500 or less, 400 or less, 300 or less, 200 or less, 100 or less, 80 or less, 70 or less, 60 or less, 50 or less, 40 or less, 30 or less, 25 or less, 20 or less, 10 or less amino acids. For example, a nucleic acid of the invention contains 400 or less, 300 or less, 200 or less, 150 or less, 100 or less, 90 or less, 80 or less, 70 or less, 60 or less, 50 or less, 40 or less, 35 or less, 30 or less, 28 or less, 26 or less, 24 or less, 22 or less, 20 or less, 18 or less, 16 or less, 14 or less, 12 or less, 10 or less nucleotides.

In some cases, a compound *(e.g.,* small molecule) or macromolecule *(e.g.,* nucleic acid, polypeptide, or protein) of the invention is purified and/or isolated. As used herein, an "isolated" or "purified" small molecule, nucleic acid molecule, polynucleotide, polypeptide, or protein (e.g., antibody or fragment thereof), is substantially free of other cellular material, or culture medium when produced by recombinant techniques, or chemical precursors or other chemicals when chemically synthesized. Purified compounds are at least 60% by weight (dry weight) the compound of interest. Preferably, the preparation is at least 75%, more preferably at least 90%, and most preferably at least 99%, by weight the compound of interest. For example, a purified compound is one that is at least 90%, 91%, 92%, 93%, 94%, 95%, 98%, 99%, or 100% (w/w) of the desired compound by weight. Purity is measured by any appropriate standard method, for example, by column chromatography, thin layer chromatography, or high-performance liquid chromatography (HPLC) analysis. A purified or isolated polynucleotide (ribonucleic acid (RNA) or deoxyribonucleic acid (DNA)) is free of the genes or sequences that flank it in its naturally occurring state. Purified also defines a degree of sterility that is safe for administration to a human subject, e.g., lacking infectious or toxic agents.

By "substantially pure" is meant a nucleotide or polypeptide that has been separated from the components that naturally accompany it. Typically, the nucleotides and polypeptides are substantially pure when they are at least 60%, 70%, 80%, 90%, 95%, or even 99%, by weight, free from the proteins and naturally-occurring organic molecules with they are naturally associated.

Polynucleotides, polypeptides, or other agents are purified and/or isolated. Specifically, as used herein, an "isolated" or "purified" nucleic acid molecule, polynucleotide, polypeptide, or protein, is substantially free of other cellular material, or culture medium when produced by recombinant techniques, or chemical precursors or other chemicals when chemically synthesized. Purified compounds are at least 60% by weight (dry weight) the compound of interest. Preferably, the preparation is at least 75%, more preferably at least 90%, and most preferably at least 99%, by weight the compound of interest. For example, a purified compound is one that is at least 90%, 91%, 92%, 93%, 94%, 95%, 98%, 99%, or 100% (w/w) of the desired compound by weight. Purity is measured by any appropriate standard method, for example, by column chromatography, thin layer chromatography, or high-performance liquid chromatography (HPLC) analysis. A purified or isolated polynucleotide (ribonucleic acid (RNA) or deoxyribonucleic acid (DNA)) is free of the genes or sequences that flank it in its naturally-occurring state. A purified or isolated polypeptide is free of the amino acids or sequences that flank it in its naturally-occurring state. Purified also defines a degree of sterility that is safe for administration to a human subject, *e.g*., lacking infectious or toxic agents.

Similarly, by "substantially pure" is meant a nucleotide or polypeptide that has been separated from the components that naturally accompany it. Typically, the nucleotides and polypeptides are substantially pure when they are at least 60%, 70%, 80%, 90%, 95%, or even 99%, by weight, free from the proteins and naturally-occurring organic molecules with they are naturally associated.

By "isolated nucleic acid" is meant a nucleic acid that is free of the genes which flank it in the naturally-occurring genome of the organism from which the nucleic acid is derived. The term covers, for example: (a) a DNA which is part of a naturally occurring genomic DNA molecule, but is not flanked by both of the nucleic acid sequences that flank that part of the molecule in the genome of the organism in which it naturally occurs; (b) a nucleic acid incorporated into a vector or into the genomic DNA of a prokaryote or eukaryote in a manner, such that the resulting molecule is not identical to any naturally occurring vector or genomic DNA; (c) a separate molecule such as a cDNA, a genomic fragment, a fragment produced by polymerase chain reaction (PCR), or a restriction fragment; and (d) a recombinant nucleotide sequence that is part of a hybrid gene, *i.e.,* a gene encoding a fusion protein. Isolated nucleic acid molecules according to the present invention further include molecules produced synthetically, as well as any nucleic acids that have been altered chemically and/or that have modified backbones. For example, the isolated nucleic acid is a purified cDNA or RNA polynucleotide. Isolated nucleic acid molecules also include messenger ribonucleic acid (mRNA) molecules.

The transitional term "comprising," which is synonymous with "including," "containing," or "characterized by," is inclusive or open-ended and does not exclude additional, unrecited elements or method steps. By contrast, the transitional phrase "consisting of' excludes any element, step, or ingredient not specified in the claim. The transitional phrase "consisting essentially of' limits the scope of a claim to the specified materials or steps "and those that do not materially affect the basic and novel characteristic(s)" of the claimed invention.

As used herein, the term, "about", is plus or minus 1%, 2%, 3%, 4%, 5%, 6%, 7%, 8%, 9%, 10%, 12%, or 15%.

A drug refill is a composition that contains the first moiety (target molecule) of a binding pair, where the second moiety (target recognition moiety, e.g., molecule) of the binding pair is present on a stationary device described herein.

Genbank accession numbers for proteins and peptides/polypeptides described herein are provided below.

The amino acid sequence of streptavidin from *Streptomyces avidinii,* provided by GenBank Accession No. P22629.1, is shown below:

1 mrkivvaaia vslttvsita sasadpskds kaqvsaaeag itgtwynqlg stfivtagad
61 galtgtyesa vgnaesryvl tgrydsapat dgsgtalgwt vawknnyrna hsattwsgqy
121 vggaearint qwlltsgtte anawkstlvg hdtftkvkps aasidaakka gvnngnplda
181 vqq  (SEQ ID NO: 27)

The nucleic acid sequence of streptavidin from *Streptomyces avidinii,* provided by GenBank Accession No. X03591.1, is shown below:

1 ccctccgtcc ccgccgggca acaactaggg agtatttttc gtgtctcaca tgcgcaagat
61 cgtcgttgca gccatcgccg tttccctgac cacggtctcg attacggcca gcgcttcggc
121 agacccctcc aaggactcga aggcccaggt ctcggccgcc gaggccggca tcaccggcac
181 ctggtacaac cagctcggct cgaccttcat cgtgaccgcg ggcgccgacg gcgccctgac
241 cggaacctac gagtcggccg tcggcaacgc cgagagccgc tacgtcctga ccggtcgtta
301 cgacagcgcc ccggccaccg acggcagcgg caccgccctc ggttggacgg tggcctggaa
361 gaataactac cgcaacgccc actccgcgac cacgtggagc ggccagtacg tcggcggcgc
421 cgaggcgagg atcaacaccc agtggctgct gacctccggc accaccgagg ccaacgcctg
481 gaagtccacg ctggtcggcc acgacacctt caccaaggtg aagccgtccg ccgcctccat
541 cgacgcggcg aagaaggccg gcgtcaacaa cggcaacccg ctcgacgccg ttcagcagta
601 gtcgcgtccc ggcaccggcg ggtgccggga cctcggcc (SEQ ID NO: 28)

IGF-1 is a single chain polypeptide of 70 amino acids crosslinked by three disulfide bridges. (Rinderknecht et al., 1978, J. Biol. Chem. 253:2768-2776; sequence on p. 2771, hereby incorporated by reference). Human IGF-1 comprises the following amino acid sequence (GenBank: CAA01954.1 and SEQ ID NO:29). Human IGF-1 can be purchased from R&D Systems (614 McKinley Place NE. Minneapolis, MN 55413)

1 mgpetlcgae lvdalqfvcg drgfyfnkpt gygsssrrap qtgmvdeccf rscdlrrlem
61 ycaplkpaks a (SEQ ID NO: 29)

Human IGF-1B isoform comprises the following sequence (GenBank: CAA40093.1; SEQ ID NO:30). The mature peptide comprises residues 49-118.

1 mgkisslptq lfkccfcdfl kvkmhtmsss hlfylalcll tftssatagp etlcgaelvd
61 alqfvcgdrg fyfnkptgyg sssrrapqtg ivdeccfrsc dlrrlemyca plkpaksars
121 vraqrhtdmp ktqkyqppst nkntksqrrk gwpkthpgge qkegteaslq irgkkkeqrr
181 eigsrnaecr gkkgk (SEQ ID NO: 30)

Exemplary GenBank Accession Nos. of VEGFB include (nucleic acid) NM 003377.4 and (amino acid) NP_003368.1. Exemplary GenBank Accession Nos. of VEGFC include (nucleic acid) NM 005429.3 and (amino acid) NP 005420.1. Exemplary GenBank Accession Nos. of VEGFD include (nucleic acid) NM 004469.4 and (amino acid) NP_004460.1.

Exemplary GenBank Accession Nos. of basic fibroblast growth factor (amino acid) AAB21432.2 (GI:8250666) and (nucleic acid) A32848.1 (GI:23957592).

Exemplary GenBank Accession Nos. of FGF include (nucleic acid) U76381.2 and (amino acid) AAB18786.3.

Exemplary GenBank Accession Nos. of HGF include (nucleic acid) M73239.1 and (amino acid) AAA64239.1.

Exemplary GenBank Accession Nos. of NGF include (nucleic acid) M57399.1 and (amino acid) AAA35961.1.

Exemplary GenBank Accession Nos. of LIF include (nucleic acid) NM 002309.4 and (amino acid) NP_002300.1.

Exemplary GenBank Accession Nos. of PDGF include (nucleic acid) NM002608.2, NM_002607.5, NM_016205.2, and NM 025208.4, and (amino acid) NP_002599.1, NP_002598.4, NP 057289.1, NP_079484.1.

Exemplary GenBank Accession Nos. of Ang1 include (nucleic acid) AY124380.1 and (amino acid) AAM92271.1. Exemplary GenBank Accession Nos. of Ang2 include (nucleic acid) AF024631.2 and (amino acid) AAF21627.2.

Exemplary GenBank Accession Nos. of MMP include (nucleic acid) D83646.1 and D83647.1, and (amino acid) BAA12022.1 and BAA12023.1.

Exemplary GenBank Accession Nos. of Delta-Like Ligand 4 (DLL4) include (nucleic acid) NM_019074.3 and (amino acid) NP_061947.1.

The following materials and methods were used in the examples described herein.

### DNA synthesis

DNA oligonucleotides were either synthesized using standard automated solid-phase phosphoramidite coupling methods on a PerSeptive Biosystems Expedite 8909 DNA synthesizer or purchased from Integrated DNA Technologies. All reagents and phosphoramidites for DNA synthesis were purchased from Glen Research. Oligonucleotides were purified by reverse-phase HPLC using a C18 stationary phase and an acetonitrile/100 mM triethyl ammonium acetate (TEAA) gradient and quantitated using UV spectroscopy carried out on a Nanodrop ND1000 Spectrophotometer. 3'-thiol DNA was synthesized using 3'-Thiol-Modifier C3 S-S CPG columns. Phosphorothioates were synthesized using 0.05 M Sulfurizing Reagent II in pyridine/acetonitrile. DNA sequences used are summarized in Table 1.

### Alginate Oxidation

Medical grade, high guluronic acid content, high M_{w} alginate (MVG) was purchased from FMC Biopolymers (Princeton, NJ). A 1% solution of sodium alginate (1.0 g, 4 micromoles) in water was mixed with sodium periodate (54 mg, 252 micromoles) at room temperature and stirred. The reaction was stopped after 24 h by the addition of ethylene glycol (30 mg, 28.5 µL). Solution was precipitated with an excess amount of isopropyl alcohol. The precipitates, collected by centrifugation, were redissolved in distilled water and precipitated again with isopropanol. The oxidized alginate was freeze-dried under reduced pressure to yield a white product (0.9 g, 90% yield). The molecular size and hydrodynamic radius were analyzed with gel permeation chromatography (Viscotek) comprised of a laser refractometer, a differential viscometer, and a right angle laser light scattering detector.

### BMPH, Dye-750 and DNA conjugation to alginate

100 mg of alginate (0.4 micromoles, 1 eq.) was dissolved overnight in 100 mL 2-(N-morpholino)ethanesulfonic acid (MES) buffer (100 mM MES, 300 mM NaCl, pH=5.5). 1-ethyl-3-(3-dimethylaminopropyl)carbodiimide (EDC) (7.7 mg, 40 micromoles, Sigma E7750) was added and stirred for 5 minutes. N-beta-Maleimidopropionic acid hydrazide-TFA (BMPH) (5.94 mg, 20 micromoles, Thermo Scientific 22297) and/or HiLyte Fluor™ 750 hydrazide (3.76 mg, 3.6 micromoles, Anaspec 81268) were added and the reaction was stirred for 20 hours. Samples were precipitated in 80% isopropanol, resuspended in water and precipitated again. Samples were dried under high vacuum.

¹H NMR spectra were recorded on a Varian Inova-500 (500 MHz) at ambient temperature in 99.9% D2O. All NMR solvents were purchased from Cambridge Isotope Laboratories.

Alginate-BMPH (10 mg, -0.04 micromoles) was dissolved in Tris buffer (100 mM, pH 8.0) overnight. 3'-thiol DNA (0.4 umoles) was dissolved in 200 µL Tris (100 mM, pH 8.0) and incubated with 20 µL (tris(2-carboxyethyl)phosphine) (TCEP) (.5M) for 30 minutes. DNA was centrifuged on a 3K MW cut-off (MWCO) centrifugal filter device (Nanosep 3K Omera filter, PALL OD003C33) to remove TCEP and thiol small molecules and mixed with alginate. The mixture was incubated for 20 hours and then transferred to a 100K MWCO centrifugal filter device (Nanosep 100K Omega Filter, PALL OD100C33), centrifuged to remove DNA. Supplemented with 500 µL distilled water and centrifuged again. Samples were diluted to 0.5% alginate, sterile filtered and freeze-dried to dryness. Final weight: ∼9 mg.

### Doxorubixin-alginate coupling

Adipic acid dihydrazide (587 mg, 3.3 mmoles, Sigma A0638) was dissolved in aqueous calcium chloride (1 M) at a concentration of 0.5M. Doxorubicin hydrochloride (10 mg, 17 µmols, Sigma D1515, 10 mg/mL in DMSO) was added to the solution and stirred for 48 hours at 37 °C. Solutions were directly purified on a Agilent 1200 Series Prep-Scale HPLC. Product fractions were freeze-dried. Yield: 40% Observed Product Mass: 700.3 Daltons.
BMPH-conjugated alginate (5 mg) was dissolved in 5 mL Tris-HCl (100 mM, pH 8.0) overnight. To this solution was added 160 mg adipic-doxorubicin (228 µmoles) and stirred overnight. The solution was buffer exchanged into water using a 3K MWCO centrifugal filter device (Nanosep 3K Omera filter, PALL OD003C33). Solution was sterile filtered and freeze-dried. Overall yield: 80%.

### Doxorubicin release from alginate

Doxorubicin or doxorubicin-hydrazde was combined with alginate (oxidized and unoxidized) and gelled with calcium chloride and incubated in PBS at 37 °C. At different time points, solution was changed and doxorubicin concentrations were measured on a molecular devices spectramax plate reader (ex: 530, em: 590).

### In vitro cytotoxicity of alginate-coupled doxorubicin

The cytotoxic effects of hydrazone-linked doxorubicin-alginate and doxorubicin alone were evaluated using the Alamar Blue assay. MDA-MB-231 cells were seeded at a density of 60,000 cells/well in 24-well flat-bottomed plates and incubated for 24 h. Cells were washed twice with PBS and incubated in the culture medium with various concentrations of doxorubicin or alginate-linked doxorubicin for 24 h at 37 °C. Cell viability was evaluated by Alamar blue assay according to vendor instructions and read on a molecular devices spectramax plate reader (ex: 530, em: 590).

### Alginate circulation measurements:

C57/B6 mice were injected retro-orbitally with 100 µL 0.5% fluorescent alginate (unoxidized alginate, Dye-750). Mice were shaved on the front and back and imaged with an IVIS Spectrum in vivo imager (ex: 745, em: 820) over 30 days. Shaving was repeated before every image collection. Fluorescence in the blood was measured through quantification of fluorescence in the snout area.

### In vitro interaction studies (Figure 2)

15-20 µL. droplets of 0.5% unoxidized alginate conjugated to polyA-SH were immersed in a calcium chloride solution (100 mM) for 60 minutes. Calcium-alginate gels were washed twice with PBS containing 1mM calcium chloride. 500 µL of 0.8 µM fluorescent (Fl) oligonucleotides (polyT-Fl or polyA-Fl) were added and incubated for 1, 5, 30 minutes or 16 hours on an orbital shaker. Samples were washed twice with 500 µL PBS (1 mM CaCl2) and imaged on a fluorescence microscope under the green channel. For quantification, gels were de-crosslinked in 100 µL 100 mM EDTA for 30 minutes and fluorescence read on a molecular devices spectramax plate reader (excitation 485, emission 538, cutoff 530).

### In vitro interaction studies (Figure 3)

15-20 µL. droplets of DNA-conjugated alginate (0.5% unoxidized alginate conjugated to polyA-SH, polyT-SH or unconjugated) were immersed in a calcium chloride solution (100 mM) for 60 minutes. Calcium-alginate gels were washed twice with PBS containing 1 mM calcium chloride. 500 µL of 0.05% alginate conjugated to polyT-SH/HEX (Figs. 3A-C) or to polyA-SH and Dye-750 was added and incubated for 5, 10, 30, 60 minutes (Figs. 3A-C) or 30 mins (Figs. 3D-F) on an orbital shaker. Samples were washed twice with 500 µL PBS (1 mM CaCl₂) and imaged on a fluorescence microscope (green channel) or on an IVIS Spectrum CT in vivo imager (ex: 745, em: 820). For quantification, gels for Figs. 3A-C were de-crosslinked in 100 µL. 100 mM Ethylenediaminetetraacetic acid (EDTA) for 30 minutes and fluorescence read on a molecular devices spectramax plate reader.

### In vivo Hydrogel Homing

All animal experiments were performed according to established animal protocols. Tumors were created by injecting C57/B6 mice subcutaneously with 100,000 B16-F10 melanoma cells (American Type Culture Collection, VA) in 100 µL PBS in the back of the neck. Animals were monitored for the onset of tumour growth (approximately 2 weeks). 20 mg/mL alginate conjugated to DNA (polyT-SH or polyA-SH) was crosslinked with 4% wt/v CaSO₄ (1.22 M) and 50 µL was injected into the center of tumors with a 23-gauge needle. Any covering hair on the tumors was shaved.

One day later, mice were injected retro-orbitally with 100 µL 0.5% DNA-conjugated fluorescent alginate (unoxidized alginate, polyA-SH, Dye-750). Mice were imaged every 24 hours for six days on an IVIS Spectrum *in vivo* imager (ex: 745, em: 820). Mice were euthanized for humane reasons when tumours grew to > 20 mm in one direction or when the tumor became ulcerated through skin with a non-healing open wound present. Mice that died or had to be euthanized prior to completion of experiment were not included in the analysis.

### Xenograft Tumor Studies

All animal experiments were performed according to established animal protocols. Tumors were created by injecting 10⁶MDA-MB-231 cells (American Type Culture Collection, VA) combined with Matrigel (BD Biosciences, CA) to a total volume of 200 µL (100 µL. PBS and 100 µL. Matrigel) into the hind limbs of 8 week old J:Nu (*Foxn1^{nu}*/*Foxn1^{nu}*) mice (Jackson Labs, ME). 35 days following tumor inoculation tumors were injected with alginate gels. 20 mg/mL alginate conjugated to DNA (thioT-SH or unconjugated) was mixed with doxorubicin (1.6 mg drug per mL of gel), then crosslinked with 4% wt/v CaSO₄ (1.22 M) and then 50 µL of gel were injected into tumors with a 23-gauge needle. 2, 3, 4, and 5 weeks after initial tumoral gel injection, mice were injected retro-orbitally with 100 µL 0.5% DNA-conjugated alginate carrying doxorubicin (5% oxidized alginate, thioA-SH, Dye-750, 160ug Dox-hydrazide = 120 µg Dox). Throughout the study, tumor area was measured twice per week with digital calipers in two dimensions and the product of the two dimensional values was used to approximate tumor area. Mice were sacrificed on day 49. Mice that died or had to be euthanized prior to completion of experiment were not included in the analysis.

### 3-(p-Benzylamino)-l,2,4,5 Tetrazine synthesis

3-(p-Benzylamino)-1,2,4,5-tetrazine was synthesized according to standard methods. For example, 50 mmol of 4-(Aminomethyl)benzonitrile hydrochloride and 150 mmol formamidine acetate were mixed while adding 1 mol of anhydrous hydrazine. The reaction was stirred at 80 °C for 45 minutes and then cooled to room temperature, followed by addition of 0.5 mol of sodium nitrite in water. 10% HCl was then added dropwise to acidify the reaction to form the desired product. The oxidized acidic crude mixture was then extracted with DCM, basified with NaHCO₃, and immediately extracted again with DCM. The final product was recovered by rotary evaporation, and purified by HPLC. Chemicals were purchased from Sigma-Aldrich.

### Azide and Tetrazine conjugation to alginate

200 mg of alginate (0.8 micromoles, 1 eq.) was dissolved overnight in 200 mL MES buffer (100 mM MES, 300 mM NaCl, pH=5.5). 11-Azido-3,6,9-trioxaundecan-1-amine (349 mg, 1.6 mmole, 2000 eq, Sigma-Aldrich 17758) or tetrazine-amine (300 mg, 1.6 mmole, 2000 eq) was added to the solution and stirred for an additional 1 hour at room temperature. A mixture of 1-Ethyl-3-[3-dimethylaminopropyl]carbodiimide hydrochloride (306.7 mg, 1.6 mmole, 2000 eq, Sigma-Aldrich E7750) and sulfo-N-hydroxysuccinimide (173 mg, 800 µmoles, 1000 eq, Thermo Fisher 24510) was added in three equal doses eight hours apart and stirred for an additional eight hours. Samples were dialyzed against 4 L of water with successively lower NaCl content, changing solution 2-3 times per day. NaCl per 4 L of water: 30 g, 25 g, 20 g, 15 g, 10 g, 5 g, 0 g, 0 g, 0 g, 0 g, 0 g. Samples were lyophilized under high vacuum. Final weight: 150-160 mg. Yield: 75-80%. ¹H NMR spectra were recorded on a Varian Inova-500 (500 MHz) at ambient temperature in 99.9% D₂O. Deuterium oxide was purchased from Cambridge Isotope Laboratories. For estimation of substitution, azide peak at 1-1.2 ppm and tetrazine peak at 7.5, 8.5 and 10.4 ppm were compared to the three urinate protons between 3 and 4.2 ppm. See Figures 17-18.

### In vitro interaction studies azide-DBCO

15-20 µL. droplets of 2% unoxidized alginate conjugated to azide or unconjugated controls were immersed in a calcium chloride solution (100 mM) for 60 minutes. Calcium-alginate gels were washed twice with PBS containing 1 mM calcium chloride. 500 µL of 100 µM Cy7-DBCO (Click Chemistry Tools) were added and incubated for four hours on an orbital shaker. Samples were washed twice with 500 µL PBS (1 mM CaCl₂). For quantification, gels were digested with 3.5 mg/mL alginate lyase (Aldrich A1603) in 100 µL PBS overnight and fluorescence read on a molecular devices spectramax plate reader (excitation 745, emission 780). Reaction with click partner was also confirmed through incubation of calcium-crosslinked gels with 100 equivalents of DBCO-Cy7 (alginate-azide) followed by attenuated total reflectance (ATR)/infrared (IR) spectroscopy (ATIR) analysis. ATIR was measured on a Vertex70 machine with atmospheric adjustment. See Figures 19-20.

### In vitro interaction studies tetrazine-TCO

15-20 µL. droplets of 2% unoxidized alginate conjugated to tetrazine or unconjugated controls were immersed in a calcium chloride solution (100 mM) for 60 minutes. Calcium-alginate gels were washed twice with PBS containing 1 mM calcium chloride. 500 µL of 100 µM Cy7-TCO (Click Chemistry Tools) were added and incubated for four hours on an orbital shaker. Samples were washed twice with 500 µL PBS (1mM CaCl₂). For quantification, gels were digested with 3.5 mg/mL alginate lyase (Aldrich A1603) in 100 µL PBS overnight and fluorescence read on a molecular devices spectramax plate reader (excitation 745, emission 780). Reaction with click partner was also confirmed through incubation of calcium-crosslinked gels with 100 equivalents of TCO-Cy5 (alginate-tetrazine) followed by ATIR analysis. ATIR was measured on a Vertex70 machine with atmospheric adjustment. See Figures 21-22.

### Mouse Model of Hind-limb Ischemia and Alginate Gel Implantation

8-week old, female CD-1 background outbred strains (Charles River Laboratories, MA). Animals were anesthetized by intra-peritoneal injections of ketamine (80 mg/kg) and xylazine (5 mg/kg). Hindlimb ischemia was induced by unilateral external iliac artery and vein ligation. At the time of surgery, mice were randomized to one of two groups (n = 3 per group). 20 mg/mL alginate conjugated to azide or tetrazine or unconjugated control was crosslinked with 4% wt/v CaSO₄ (1.22 M) in PBS and 50µL alginate-azide/tetrazine hydrogel was injected through a 25 gauge needle near the distal end of the ligation site (n=3 each group). In control animals following surgery, unconjugated alginate was injected intramuscularly. Incisions were closed by 5-0 Ethilon sutures (Johnson & Johnson, NJ). Ischemia in the hindlimb was confirmed by laser Doppler perfusion imaging (LDPI) system (Perimed AB, Sweden).

### In Vivo Hydrogel Targeting

24 hours after surgery and gel implantation, mice were injected retro-orbital with 100 µL. 20ug/mL Cy7-TCO or Cy7-DBCO (Click Chemistry Tools). Mice were imaged at 1, 5, 30 minutes, 6 and 24 hours after IV-injection on an IVIS Spectrum in vivo imager (ex: 745, em:820). For repeated gel targeting, mice bearing alginate-Az were re-injected with 20ug/mL Cy7-TCO and imaged on the IVIS Spectrum daily. Mice did not show adverse effects from these administrations and no mouse had to be euthanized prior to completion of experiment. No mice were excluded from the analysis for any reason.

### Spatial Segregation of Molecular Targeting

Hind-limb ischemia was induced as described above, and alginate-tetrazine gels implanted intra-muscularly (n=3). Alginate-azide gels (n=3) were injected into the mammary fat pad of mice on the opposite side from the ischemic limb. A mixture of 100 µL Cy5-TCO (Click Chemistry Tools, 20 µg/mL) and Cy7-DBCO (Click Chemistry Tools, 20ug/mL) was injected retro-orbital. Mice were imaged 48 hours after IV injection in the Cy5 (Ex: 640, Em:680) and Cy7 (Ex: 740, Em: 820) regions and an optical image. Regions of Interest (ROIs) were placed blind based on the optical image based on visible gel swelling in mammary fat pad and sutures in the hind-limb. Fluorescence was quantified based on "Radiance" in the areas.

### Muscle Isolation and Fluorescence Quantitation

24-hours after IV injection of Cy7-DBCO, mice (n=3) were sacrificed. Both alginate-azide-injected and control hindlimb muscles were isolated. Samples were digested for 2 hours in 2 mL of 250 units/ml collagenase II, 1 unit/mL dispase II and 1 mg/mL alginate lyase. Samples were centrifuged (5 mins, 500 g) supernatant removed and pellet was resuspended and digestion repeated. The two digests were combined together and quantified on a molecular devices spectramax plate reader (excitation 745, emission 780) using a Cy7-DBCO dilution series for quantitation.

### Statistical Testing

All data was compared based on pair-wise comparison using the two-tailed, homoscedastic Students' t-test.

### Oral Delivery and Hydrogel Targeting to Alginate-Azide

24 hours after surgery and gel implantation, mice were administered through oral gavage 100 µL 1 mg/mL Cy7-DBCO (Click Chemistry Tools). Mice were imaged at 1, 30 minutes, 6 and 24 hours and every day for 1 week after the oral administration on an IVIS Spectrum in vivo imager (ex: 745, em:820). Fluorescence was quantified at the alginate injection site and on the mirror location in the contralateral (ctrl) limb. Mean fluorescence six days after IV-injection is shown in Figure 14. Error bars show SEM. p-value represents a one-tailed t-test either paired (against Alg-Az Ctrl conditions) or unpaired (the other conditions).

The invention will be further described in the following examples, which do not limit the scope of the invention described in the claims. Examples with complementary DNA molecules in the drug delivery device and drug refill are reference examples.

### EXAMPLES

### Example 1: Alginate circulation time in the blood

Efficient blood-based refilling of drug payloads relies on sufficient circulation lifetimes that allow payloads to encounter and bind to the primary device. The circulation time of alginate (285 KDa, 44nm hydrodynamic radius (Rh)) conjugated to a near-IR probe (Fig. 6A) was analyzed following intravenous (IV) administration to mice. Quantification of fluorescence (Fig. 6B) demonstrated that this alginate remained in circulation for at least 14 days, with a circulatory half-life of about seven days (Fig. 6C). Imaging of individual organs revealed accumulation in the lungs, liver, spleen, and to a lesser extent in the kidneys (Fig. 6D), demonstrating that all of these organs contribute to removal of the circulating alginate from the bloodstream. With a long circulation time, alginate can serve as an efficient intravascular drug carrier with the capability of extravasating and interacting with the primary drug delivery device.

### Example 2: DNA-mediated binding of drug-surrogates to alginate gels in vitro

Experiments were performed to determine whether device refilling with drug payloads could be mediated by complementary DNA binding between target calcium-alginate gel and free alginate strands conjugated to a drug payload. DNA (see Table 1 for a list of DNA used) was conjugated by its 3' end to alginate strands at a ratio of two molecules of DNA coupled per molecule of alginate. The ability of alginate-conjugated DNA to retain nucleic acid binding-activity was then tested. Alginate conjugated to (T)₂₀ oligonucleotides was ionically crosslinked with calcium to form a gel and then was incubated with fluorescently-labeled complementary (A)₂₀ or non-complementary (T)₂₀ oligonucleotides (Fig. 2A) in phosphate buffer with 1mM calcium chloride. Complementary oligonucleotides bound to the gel surface in a sequence-specific manner while non-complementary oligonucleotides showed little binding (Figs. 2B-C).

The ability of DNA-conjugated alginate gels to bind soluble, DNA-conjugated alginate strands was next tested *in vitro,* as a model for drug refilling *in vivo.* DNA-conjugated or unconjugated alginate was gelled and incubated with free alginate strands coupled to fluorescently-labeled complementary DNA for variable times in a buffer with physiological calcium concentration (Fig. 3A). Alginate strands bearing complementary DNA specifically bound to DNA-bearing gel surfaces, with about 5-fold selectivity as compared to control gel surfaces unconjugated to DNA; this selectivity was constant over time (Figs. 3B-C). To more completely represent a drug-bearing alginate-DNA conjugate, alginate conjugated to DNA was modified along its backbone with a near-IR dye to mimic drug loading, and tested for association with complementary or non-complementary DNA-bearing alginate gels. With one hour of incubation, the fluorescently-labeled alginate strands selectively bound to complementary-DNA-bearing gels, as compared to control gels bearing non-complementary DNA (Figs. 3D-F).

Due to the possibility for non-DNA mediated interactions in the presence of calcium that could crosslink free alginate strands to the gels, *in vitro* alginate binding studies were preformed under a physiologically-relevant soluble calcium concentration of 1 mM. Under these conditions, a small amount of alginate does bind to the gels in a DNA-independent manner *in vitro* (Figs. 3C and 3F). However, DNA-mediated binding of the alginates outpaced non-specific interactions by five-fold. This data show that DNA-conjugated alginate strands bind to complementary DNA-conjugated alginate gels.

### Example 3: In Vivo DNA-mediated alginate homing

Experiments were performed to determine whether fluorescently-labeled free alginate strands could home *in vivo* to a target gel through DNA-mediated targeting. DNA was conjugated to alginate through the 3' end to increase serum exonuclease stability. See, e.g., Shaw J, Kent K, Bird J, Fishback J, & Froehler B (1991) Nucleic acids research 19(4):747-750; Floege J, et al. (1999) The American journal of pathology 154(1):169-179; and Gamper H, et al. (1993) Nucleic acids research 21(1):145-150. A melanoma cancer model was chosen for these studies due to the well established enhanced permeability and retention effect in these tumors, which provides a means for passive accumulation of bloodborne nanoparticles in tumor tissue. See, e.g., Maeda H, Wu J, Sawa T, Matsumura Y, & Hori K (2000) Journal of controlled release: official journal of the Controlled Release Society 65(1-2):271-284. Mice bearing tumors between 10-20 mm³ in size received intra-tumor injections of DNA-conjugated calcium-crosslinked alginate gels. At 24 hours, fluorescently-labeled free alginate strands conjugated to complementary-DNA were administered intravenously. Imaging of mice first revealed that IV-administered strands collected in the tumors of all mice at 24 hours post-administration, likely through the EPR effect (Figs. 4A-B). In mice receiving free alginate strands conjugated to complementary DNA, the strands continued to aggregate in the tumors over the next few days in a statistically significant manner relative to controls. Over the course of one week, mice receiving non-complementary DNA-conjugated gels, or those not injected with gel showed a progressive loss of fluorescence in the tumor, reaching background level of fluorescence by day 5. In contrast, mice receiving complementary DNA-conjugated gels showed continued accumulation of fluorescence on days 2 and 3 after injection and significant retention of fluorescent alginate in the tumor. Total alginate retention is quantified as the area under the curve (Fig. 4C); DNA-mediated alginate homing produced significantly higher free strand retention in the tumor as compared to controls. This data indicate that alginate strands containing a DNA target molecule accumulated at the site of a previously administered alginate drug delivery device containing the complementary DNA as a target recognition moiety.

### Example 4: Drug refilling slows tumor growth

The therapeutic efficacy of the refillable drug delivery device described herein was analyzed by examining the ability of the targeting technology to inhibit tumor growth over several weeks. Drug-delivering alginate hydrogels destined for intra-tumor injection demonstrated sustained release of doxorubicin over a period of weeks (Fig. 8). To carry IV-administered drug payloads, alginate strands were partially oxidized to introduce aldehyde functional groups, allowing coupling of hydrazine-doxorubicin through a hydrolyzable hydrazone linker. See, e.g., Bouhadir K, et al. (2001) Biotechnology progress 17(5):945-950; and Bouhadir K, Alsberg E, & Mooney D (2001) Biomaterials 22(19):2625-2633. Oxidized alginate demonstrated sustained release of doxorubicin (Fig. 9B) without inhibiting the cytotoxicity of released doxorubicin (Fig. 9C).

The breast cancer MDA-MB-231 model was chosen because it is a slow-growing tumor, widely used to test chemotherapeutic strategies. See, e.g., Choi K, et al. (2011) ACS nano 5(11):8591-8599; and Tien J, Truslow J, & Nelson C (2012) PloS one 7(9). Immunocompromised mice bearing MDA-MB-231 xenograft tumors were injected intra-tumor with phosphorothioate DNA-conjugated alginate gels releasing doxorubicin (80 ug per animal) or bolus doxorubicin in PBS. After two weeks, mice received weekly IV administrations of free alginate strands conjugated to complementary phosphorothioate DNA and doxorubicin (120 ug per animal), or bolus doxorubicin (120 ug per animal) as a control (Fig. 5A). Targeted drug refilling inhibited tumor growth significantly, as compared to treatment with bolus doxorubicin control (Fig. 5B). The ability of targeted therapy to reduce tumor size was assessed by monitoring the change in tumor size in the three days following each IV administration. Tumors shrank after the first two drug targeted treatments, but continued to grow in mice receiving bolus doxorubicin controls (Fig. 5C).

Strikingly, the first two refillings of the gel appeared to yield the greatest impact, with a less pronounced effect for the second two refillings. This may have been due to saturation of DNA strands on the targeted gel or degradation of the phosphorothioate oligonucleotides on the gel over the course of the experiment. Alternatively, the drug delivery system may have shrunk the tumor to a size in which enhanced permeability was no longer prominent, leading to inefficient targeting.

These results demonstrated that drug reloading using these methods had a significant clinical benefit. In addition, experiments were performed to further confirm that the effect was specific to the interaction of complementary DNA strands *in vivo* and that differences in doxorubicin pharmacokinetics or release could not account for the effects seen. Specifically, the following additional controls were tested (see Table 2): 1) intratumoral injections of alginate with encapsulated doxorubicin but no conjugated DNA, coupled with IV administration of DNA-conjugated alginate and hydrazone-linked doxorubicin, 2) intratumoral injections of alginate gels with encapsulated doxorubicin but no bound DNA, coupled with IV administration of free doxorubicin.

**Table 2: Tumor therapy experimental groups**

| Group | Intratumor Injection | Retro-orbital Injections |
|---|---|---|
| Targeted therapy | 50µL of alginate gel (2% w/v in PBS; ionically crosslinked) conjugated to thioT-DNA and mixed with doxorubicin (0.08mg/animal) | 100µL of alginate (0.5% w/v in PBS, 5% oxidized) conjugated to thioA-DNA and dox-hydrazide (0.12mg dox/animal) |
| No DNA control | 50µL of alginate gel(2% w/v in PBS; ionically crosslinked) mixed with doxorubicin (0.08mg/animal) | 100µL of alginate (0.5% w/v in PBS, 5% oxidized) conjugated to thioA-DNA and dox-hydrazide (0.12mg dox/animal) |
| Bolus IV control | 50µL of alginate gel (2% w/v in PBS; ionically crosslinked) mixed with doxorubicin (0.08mg/animal) | 100µL doxorubicin (0.12mg/animal) in PBS |
| All bolus ctrl | 50µL of PBS with doxorubicin (.08mg/animal) | 100µL doxorubicin (0.12mg/animal) in PBS |

The total dose of drug injected directly into the tumor and delivered via IV administration was kept constant across all groups, as was the frequency of IV administration. After 7 weeks of tumor growth, tumors treated with the drug reloading technology were markedly smaller than tumors in any of the control conditions (Fig. 5D). Quantification of the tumor sizes confirmed the smaller size of the tumors treated with the drug reloading technology compared to the controls (p<0.05 targeted compared to first two controls, Fig. 5E). This data demonstrate that systemic administration of a cancer drug refill to recharge a drug delivery device previously administered intra-tumorally reduced tumor size in a breast cancer mouse model.Example 5: Specific binding of bioorthogonal functional groups to hydrogels

The binding specificity of fluorescently-labeled bioorthogonal functional groups (e.g., trans-cyclooctene (TCO) and dibenzycyclooctyne (DBCO) molecules) to hydrogels was evaluated *in vitro.* Polymer, e.g., alginate, was modified with either tetrazine (Tz) or azide (Az) through carbodiimide chemistry. Nuclear Magnetic Resonance (NMR) analysis demonstrated that, on average, 100 molecules of Tz or Az were attached to each polymer (e.g., alginate) strand, constituting 400 nanomoles of target (e.g., bioorthogonal functional group, e.g., Tz or Az) per mg of polymer. The functional groups, e.g., Tz or Az, were labeled with a fluorescent moiety, e.g., Cy7, for detection.

Cy7-labeled trans-cyclooctene specifically bound to tetrazine-modified alginate, but not to unmodified alginate (Figures 10A,C). Similarly, fluorescent DBCO bound alginate-azide gels but had little interaction with unmodified alginate gels (Figures 10B,D). This data indicate that bioorthogonal functional groups bind specifically to hydrogels modified with the corresponding binding partner of the functional group.

### Example 6: Bioorthogonal functional groups target gels in vivo

Experiments were performed to determine whether bioorthogonal functional groups labeled with near-IR (NIR) fluorophores could target gels *in vivo. In vivo* gels were resident at a disease site in an animal model of lower limb ischemia. 50 µL of tetrazine-modified, azide-modified, or unmodified alginate gels were implanted intra-muscularly in the limbs of mice subjected to femoral artery ligation, in a similar manner as described in Chen et al. J. Pharmaceutical research 24(2006):258; and Silva et al. J. Biomaterials 31(2010):1235. Twenty-four hours post-surgery, NIR-labeled DBCO or TCO-molecules were administered intravenously (IV), and the animals were monitored over 24 hours through live animal fluorescence imaging. The NIR-labled small molecules circulated in the animal's system and were eliminated mainly through the bladder in the first 24 hours (Figures 16A-B) After 24-hours, fluorescence was observed in the limbs implanted with modified gels (e.g., Tz- or Zamodified gels), but not in control gels, which lacked the target recognition motifs (e.g., Tz or Az) (Figure 11).

To quantify the dose of circulating molecules delivered to intra-muscular gels, azide-modified gels were isolated from mouse muscles and digested. The amount of targeted molecules on the gels was quantified by fluorescence. An average of 106 picomoles of targeting compound, corresponding to 6.5% of the initially injected dose, localized to the intra-muscular disease area, as shown in the table below.

**Quantitation of small molecule targeting in muscles**

| Site | Moles SM | St. Dev |
|---|---|---|
| Ischemic Limb with Alginate-Az gel | 6.54% | 3% |
| Control Limb (no gel) | 0.12% | 0.1% |

This number of molecules that were targeted to the gels constitutes 0.03% of the total available azide sites on the gel, demonstrating that multiple gel fillings/refillings are possible for each gel due to the excess target recognition sites. This data show that systemically administered bioorthogonal functional groups accumulate at the site of a previously administered/implanted drug delivery device. Because a single injected dose targets about .03% of the available azide sites on the gel, the device may be refilled up to 3,300 times. For example, the gel in Fig. 5A is refilled four times, while the gel in Fig. 12A is refilled nine times over the period of one month. In some cases, with respect to reversible/cleavable chemistry, toehold exchange (WO 2012058488 A1 is utilized to reverse binding of DNA-conjugated nanoparticles to the gel.

### Example 7: Repeated administrations of bioorthogonal functional groups to target and refill gels in vivo

Multiple intravascular administrations were performed to repeatedly target and fill an intramuscular gel in injured mice. Mice with hind limb ischemia carrying azide-modified gels (e.g., alginate-Az gels) were repeatedly administered NIR-labeled DBCO, approximately once every three days for one month (Figure 12A). Over the one-month time course, limb fluorescence increased in a step-wise fashion, corresponding to fluorophore administrations (Figure 12B), with each dose increasing fluorescence to a similar extent (Figure 12C). Thus, gel homing using the methods described herein was achieved in an animal model of ischemia. A small but not significant decrease in fluorescence in the limb was observed between 24 and 48 hours after IV injection, likely due to residual unbound fluorophores removed from the gel. This data demonstrate that systemically administered drug refills accumulate at the site of the drug delivery device each time the refill is administered. The same device can be recharged multiple times by systemically administed refills.

### Example 8: Targeting of two different bioorthogonal functional groups to two separate gel sites in vivo

Experiments were conducted to determine whether two different bioorthogonal functional groups, e.g., DBCO and TCO, could selectively home to their respective binding partners (i.e., target recognition moieties, e.g., molecules) to achieve spatial separation of drug-devices in the same animal. Two sites on a mouse were used. Tetrazine-modified gels were implanted intra-muscularly in the hind limb of mice subjected to hind-limb ischemia as a first target site. Azide-modified gels were implanted into the mammary fat pad of the same mice as a second target site. A mixture of Cy7-labeled DBCO and Cy5-labeled TCO was administered intravenously 24 hours after gel administration. The two bioorthogonal functional groups were efficiently and specifically targeted to their respective disease sites (Figure 13). This data indicate that systemic administration of two different types of drug refills leads to accumulation of each type of drug refill at its respective drug delivery device site.

### Example 9: Oral delivery and targeting to alginate-azide hydrogel

Mouse models of hind limb ischemia were generated as described above. Hydrogels containing alginate modified with azide were implanted intramuscularly into the ischemic site in the mice. 24 hours after surgery and gel implantation, mice were orally administered Cy7-DBCO, e.g., through oral gavage. Mice were imaged 1, 30 minutes, 6, and 24 hours, and every day for 1 week after the oral administration. Fluorescence was quantified at the alginate implantation site and on the mirror location in the contralateral limb (control limb). Orally administered Cy7-DBCO was targeted to the azide-modified alginate hydrogel. See Figure 14. These data indicate that oral delivery of a drug refill leads to accumulation of the orally administered drug at the site of the alginate drug delivery device.

### Example 10: Targeting DBCO To Gels Modified With Azide

A set of images showing targeting of the IV-injected fluorescently labeled DBCO to an intraosseous gel modified with azide or unmodified control gel in the femur of a mouse is shown in Fig. 23. 50 µL of azide-modified or unmodified alginate gels were injected intraosseus into the bones of mice. Twenty-four hours post-surgery, NIR-labeled DBCO molecules were administered intravenously (IV), and the animals were monitored over 24 hours through live animal fluorescence imaging. After 24-hours, fluorescence was observed in the limbs implanted with modified gels (e.g., Az-modified gels), but not in control gels, which lacked the target recognition motifs (e.g., Az).

As shown in Fig. 24, IV-injected fluorescently labeled DBCO was targeted to a gel modified with azide injected into the right knee joint of a mouse. 50 µL of azide-modified alginate gels were injected into knee joint of mice. Twenty-four hours post-surgery, NIR-labeled DBCO molecules were administered intravenously (IV), and the animals were monitored over 24 hours through live animal fluorescence imaging. After 24-hours, fluorescence was observed in the limbs implanted with modified gels (e.g., Az-modified gels).

As shown in Fig. 25, IV-injected fluorescently-labeled DBCO was targeted to a gel modified with azide compared to control gel injected into the tumor of a mouse. The images in Figure 25 were taken 24 hours after IV injection. Lewis Lung Carcinoma (LLC) tumors were induced in C57B6 mice. Once the tumors were 5-8 mm in diameter, 50 µL of azide-modified alginate gels were injected into the tumors. Twenty-four hours post tumoral injection, NIR-labeled DBCO molecules (see, Fig. 26) were administered intravenously (IV), and the animals were monitored over 24 hours through live animal fluorescence imaging. After 8-hours, fluorescence was observed in the tumors implanted with modified gels (e.g., Az-modified gels), but not in control gels, which lacked the target recognition motifs (e.g., Az).

An illustration showing capture of a small molecule by an azide-modified gel and the subsequent release of the small molecule through hydrolysis after the capture is shown in Fig. 26. The molecule in this example is a Cy7 fluorophore conjugated to DBCO through a hydrolyzable hydrazone linker.

As shown in Fig. 27A, IV-injected small molecule was targeted to a gel modified with azide injected into the tumor of a mouse. The small molecule, a Cy7 fluorophore conjugated to DBCO through a hydrolyzable hydrazone linker, is subsequently released, leading to loss of the Cy7 fluorescence signal. A line graph showing the quantification of the small molecule at the tumor site over time (hours) demonstrating release of the small molecule is illustrated in Fig. 27B. Lewis Lung Carcinoma (LLC) tumors were induced in mice. Once the tumors were 5-8 mm in diameter, 50 µL of azide-modified alginate gels were injected into the tumors. Twenty-four hours post tumoral injection, NIR-labeled DBCO molecules (see, Fig. 26) were administered intravenously (IV), and the animals were monitored over four days through live animal fluorescence imaging. Fig. 27B shows quantitation of tumor fluorescence 24, 48, 72 and 96 hours after IV fluorophore administration showing that the fluorescence decreases over time as Cy7 fluorophore is cleaved from the gel and is cleared from the tumor.

### OTHER EMBODIMENTS

While the invention has been described in conjunction with the detailed description thereof, the foregoing description is intended to illustrate and not limit the scope of the invention, which is defined by the scope of the appended claims.

The patent and scientific literature referred to herein establishes the knowledge that is available to those with skill in the art.

While this invention has been particularly shown and described with references to preferred embodiments thereof, it will be understood by those skilled in the art that various changes in form and details may be made therein without departing from the scope of the invention encompassed by the appended claims.

## Claims

1. A refillable drug delivery system comprising a drug delivery device and a drug refill,
wherein the drug delivery device comprises a hydrogel and a target recognition molecule and is suitable for implantation at a desirable location within a subject;
wherein the drug refill comprises a pharmaceutical composition attached to a target molecule via a cleavable linker and the drug refill is suitable for subsequent administration to the subject;
wherein the drug refill is mobile within the subject until the target molecule on the drug refill binds to the target recognition molecule on the drug delivery device at the desirable location within the subject; and the target molecule and the target recognition molecule form a two-component binding pair;
wherein upon binding of the target molecule to the target recognition molecule, the cleavable linker is cleaved and the drug refill delivers the pharmaceutical composition to the drug delivery device, thereby refilling the drug delivery device;
wherein the cleavable linker comprises a linkage selected from the group consisting of a a hydrazone linkage, an acetal linkage, a ketal linkage, an oxime linkage, an imine linkage, and disulfide linkage and
wherein the target molecule comprises a biorthogonal functional group and the target recognition molecule comprises a complementary functional group, wherein the biorthogonal functional group is capable of reacting by click chemistry with the complementary functional group to form a covalent bond.

2. The refillable drug delivery system of claim 1, wherein
(i) the bioorthogonal functional group comprises an alkene and the complementary functional group comprises a tetrazine (Tz); optionally wherein the alkene comprises a cyclooctene; optionally wherein the cyclooctene comprises transcyclooctene (TCO); or
(ii) the bioorthogonal functional group comprises an alkyne and the complementary functional group comprises an azide; optionally wherein the alkyne comprises a cyclooctyne; optionally wherein the cyclooctyne comprises dibenzocyclooctyne (DBCO); or
(iii) the target molecule comprises dibenzocycloocytne (DBCO) and the target recognition molecule comprises an azide; or
(iv) the bioorthogonal functional group comprises an alkene and the complementary functional group comprises a tetrazine (Tz); optionally wherein the alkene comprises a norbornene (NOR).

3. The refillable drug delivery system of claim 1, wherein the bioorthogonal functional group comprises an alkene and the complementary functional group comprises a tetrazine (Tz); wherein the alkene comprises a transcyclooctene (TCO).

4. The refillable drug delivery system of claim 1, wherein the bioorthogonal functional group comprises an alkene and the complementary functional group comprises a tetrazine (Tz); wherein the alkene comprises norbornene (NOR).

5. The refillable drug delivery system of claim 1, wherein the bioorthogonal functional group comprises an alkyne and the complementary functional group comprises an azide; wherein the alkyne comprises a cyclooctene.

6. The refillable drug delivery system of claim 1, wherein the bioorthogonal functional group comprises an alkyne and the complementary functional group comprises an azide; wherein the alkyne comprises a dibenzocyclooctyne (DBCO).

7. The refillable drug delivery system of claim 1, wherein the pharmaceutical composition comprises an anti-cancer drug, a drug that promotes wound healing, a drug that promotes vascularization, a drug that treats or prevents infection, a drug that prevent restenosis, a drug that reduces macular degeneration, a drug that prevents immunological rejection, a drug that prevents thrombosis, or a drug that treats inflammation; optionally wherein (i) the pharmaceutical composition comprises an anti-cancer drug; optionally wherein the anti-cancer drug comprises doxorubicin; or (ii) the pharmaceutical composition comprises a drug that promotes wound healing; or (iii) the pharmaceutical composition comprises a drug that promotes vascularization or a drug that prevents restenosis; or (iv) the pharmaceutical composition comprises a drug that treats or prevents infection.

8. The refillable drug delivery system of claim 1, wherein the hydrogel comprises collagen, alginate, polysaccharide, hyaluronic acid (HA), polyethylene glycol (PEG), polyglycolide (PGA), poly(L-lactide) (PLA), poly(lactide-co-glycolide (PLGA), or polylactic-coglycoli acid; optionally wherein the hydrogel comprises alginate.

9. The refillable drug delivery system of claim 8, wherein the hydrogel comprises an alginate hydrogel.

10. The refillable drug delivery system of claim 9, wherein the hydrogel comprises a modified alginate.

11. The refillable drug delivery system of claim 10, wherein the hydrogel comprises a partially oxidized alginate.

12. The refillable drug delivery system of claim 1, wherein the drug delivery device comprises an azide-modified alginate having the following structure:

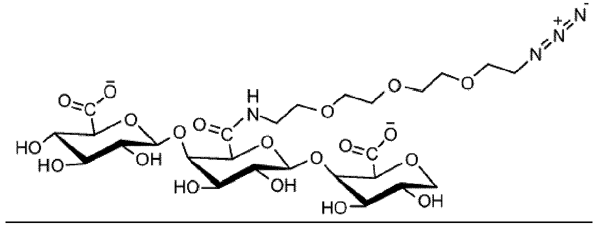

13. The refillable drug delivery system of claim 1, wherein the cleavable linker is a hydrazone linker.

14. The refillable drug delivery system of claim 1, wherein the pharmaceutical composition comprises doxorubicin.

15. The refillable drug delivery system of claim 1, wherein said system comprises at least two drug delivery devices; optionally wherein said system comprises at least two drug refills; optionally wherein each of the drug refills comprises a different pharmaceutical composition and a different target molecule; optionally wherein each drug delivery device comprises a different target recognition molecule; optionally wherein each drug refill binds to a different drug delivery device.

16. The refillable drug delivery system of claim 1 for use in refilling a drug delivery device *in vivo,* wherein
i) the drug delivery device is for administration to the subject and implant at a desired location within the subject; and
ii) the drug refill is for subsequent administration to the subject; optionally wherein the drug refill is for subsequent administration to the subject at a site located away from the device;
wherein the drug refill is mobile within the subject until the target molecule on the drug refill binds to the target recognition molecule on the drug delivery device at the desirable location within the subject; and the target recognition molecule and the target recognition molecule form a two-component binding pair; and
wherein upon binding of the target molecule to the target recognition molecule, the cleavable linker is cleaved and the drug refill delivers the pharmaceutical composition to the drug delivery device, thereby refilling the drug delivery device;
optionally wherein said drug refill is for administration orally, buccally, sublingually, rectally, intravenously, intra-arterially, intraosseously, intra-muscularly, intracerebrally, intracerebroventricularly, intrathecally, subcutaneously, intraperitoneally, intraocularly, intranasally, transdermally, epidurally, intracranially, percutaneously, intravaginally, intrauterineally, intravitreally, transmucosally, or via injection, via aerosol-based delivery, or via implantation.

17. The refillabledrug delivery system of claim 1 for use in maintaining or reducing the size of a tumor in a subject, wherein the pharmaceutical composition comprises an anti-cancer drug; optionally
wherein the drug delivery device is refilled a plurality of times by administration of the drug refill; optionally
wherein the drug refill is for administration orally, buccally, sublingually, rectally, intravenously, intra-arterially, intraosseously, intra-muscularly, intracerebrally, intracerebroventricularly, intrathecally, subcutaneously, intraperitoneally, intraocularly, intranasally, transdermally, epidurally, intracranially, percutaneously, intravaginally, intrauterineally, intravitreally, transmucosally, or via injection, via aerosol-based delivery, or via implantation; optionally
wherein the anti-cancer drug comprises doxorubicin; optionally wherein the subject suffers from a cancer which is a solid cancer or a hematological cancer.

18. The refillable drug delivery system of claim 1 for use in
(a) reducing cancer progression in a subject, wherein the pharmaceutical composition comprises an anti-cancer drug; optionally
wherein (i) the drug delivery device is refilled by administration of the drug refill to the subject orally, intraperitoneally, intravenously, or intra-arterially; (ii) optionally repeating step (i); thereby reducing cancer progression in the subject; or
(b) promoting wound healing in a subject, wherein the pharmaceutical composition promotes angiogenesis and/or maturation or remodeling of an existing blood vessel; optionally
wherein (i) the drug delivery device is refilled by administration of the drug refill to the subject; (ii) optionally repeating step (i); thereby promoting wound healing in the subject; optionally
wherein said refill is for administration orally, buccally, sublingually, rectally, intravenously, intra-arterially, intraosseously, intra-muscularly, intracerebrally, intracerebroventricularly, intrathecally, subcutaneously, intraperitoneally, intraocularly, intranasally, transdermally, epidurally, intracranially, percutaneously, intravaginally, intrauterineally, intravitreally, transmucosally, or via injection, via aerosol-based delivery, or via implantation; or
(c) reducing or controlling inflammation in a subject, wherein the pharmaceutical composition comprises an anti-inflammatory agent; optionally
wherein (i) the drug delivery device is refilled by administration of the drug refill to the subject; (ii) optionally repeating step (i); thereby reducing or controlling inflammation in the subject; optionally
wherein said refill is for administration orally, buccally, sublingually, rectally, intravenously, intra-arterially, intraosseously, intra-muscularly, intracerebrally, intracerebroventricularly, intrathecally, subcutaneously, intra peritoneally, intraocularly, intranasally, transdermally, epidurally, intracranially, percutaneously, intravaginally, intrauterineally, intravitreally, transmucosally, or via injection, via aerosol-based delivery, or via implantation; or
(d) treating an eye disease in a subject, wherein the pharmaceutical composition treats said eye disease; optionally
wherein the eye disease includes cataracts, glaucoma, retinal disease, corneal disease, temporal arteritis, or macular degeneration; optionally
wherein (i) the drug delivery device is refilled by administration of the drug refill to the eye of the subject; (ii) optionally repeating step (i); thereby treating an eye disease in the subject; or
(e) treating arrhythmia in a subject, wherein the pharmaceutical composition treats said arrhythmia; optionally
wherein (i) the drug delivery device is refilled by administration of the drug refill to the heart of the subject; (ii) optionally repeating step (i); thereby treating arrhythmia in the subject.

## Patentansprüche

1. Nachfüllbares Wirkstoffverabreichungssystem, welches eine Wirkstoffverabreichungseinrichtung und eine Wirkstoffnachfüllung umfasst, worin die Wirkstoffverabreichungseinrichtung ein Hydrogel und ein Zielerkennungsmolekül umfasst und zur Implantation an einer gewünschten Stelle in einer Person geeignet ist;
worin die Wirkstoffnachfüllung eine pharmazeutische Zusammensetzung umfasst, die an einem Zielmolekül über eine spaltbare Bindung angefügt ist und die Wirkstoffnachfüllung für anschließende Verabreichung an die Person geeignet ist;
worin die Wirkstoffnachfüllung in der Person mobil ist, bis das Zielmolekül auf der Wirkstoffnachfüllung an das Zielerkennungsmolekül auf der Wirkstoffverabreichungseinrichtung an der gewünschten Stelle in der Person bindet; und worin das Zielmolekül und das Zielerkennungsmolekül ein Zweikomponenten-Bindungspaar ausbilden;
wobei nach Binden des Zielmoleküls an das Zielerkennungsmolekül, die spaltbare Bindung gespalten wird und die Wirkstoffnachfüllung die pharmazeutische Zusammensetzung an die Wirkstoffverabreichungseinrichtung liefert, wodurch die Wirkstoffverabreichungseinrichtung nachgefüllt wird;
worin die spaltbare Bindung eine Bindung umfasst, ausgewählt aus der Gruppe bestehend aus einer Hydrazonbindung, einer Acetalbindung, einer Ketalbindung, einer Oximbindung, einer Iminbindung, und einer Disulfidbindung; und
worin das Zielmolekül eine biorthogonale funktionelle Gruppe und das Zielerkennungsmolekül eine komplementäre funktionelle Gruppe umfasst, worin die biorthogonale funktionelle Gruppe geeignet ist, durch Click-Chemie mit der komplementären funktionellen Gruppe zu reagieren, um eine kovalente Bindung auszubilden.

2. Nachfüllbares Wirkstoffverabreichungssystem nach Anspruch 1, worin
(i) die bioorthogonale funktionelle Gruppe ein Alken und die komplementäre funktionelle Gruppe ein Tetrazin (Tz) umfasst; wahlweise worin das Alken ein Cycloocten umfasst; wahlweise worin das Cycloocten Transcycloocten (TCO) umfasst; oder
(ii) die bioorthogonale funktionelle Gruppe ein Alkin und die komplementäre funktionelle Gruppe ein Azid umfasst; wahlweise worin das Alkin ein Cyclooctin umfasst; wahlweise worin das Cyclooctin Dibenzocyclooctin (DBCO) umfasst; oder
(iii) das Zielmolekül Dibenzocyclooctin (DBCO) und das Zielerkennungsmolekül ein Azid umfasst; oder
(iv) die bioorthogonale funktionelle Gruppe ein Alken und die komplementäre funktionelle Gruppe ein Tetrazin (Tz) umfasst; wahlweise worin das Alken ein Norbornen (NOR) umfasst.

3. Nachfüllbares Wirkstoffverabreichungssystem nach Anspruch 1, worin die bioorthogonale funktionelle Gruppe ein Alken und die komplementäre funktionelle Gruppe ein Tetrazin (Tz) umfasst; worin das Alken ein Transcycloocten (TCO) umfasst.

4. Nachfüllbares Wirkstoffverabreichungssystem nach Anspruch 1, worin die bioorthogonale funktionelle Gruppe ein Alken und die komplementäre funktionelle Gruppe ein Tetrazin (Tz) umfasst; worin das Alken Norbornen (NOR) umfasst.

5. Nachfüllbares Wirkstoffverabreichungssystem nach Anspruch 1, worin die bioorthogonale funktionelle Gruppe ein Alkin und die komplementäre funktionelle Gruppe ein Azid umfasst; worin das Alkin ein Cycloocten umfasst.

6. Nachfüllbares Wirkstoffverabreichungssystem nach Anspruch 1, worin die bioorthogonale funktionelle Gruppe ein Alkin und die komplementäre funktionelle Gruppe ein Azid umfasst; worin das Alkin ein Dibenzocyclooctin (DBCO) umfasst.

7. Nachfüllbares Wirkstoffverabreichungssystem nach Anspruch 1, worin die pharmazeutische Zusammensetzung umfasst, einen Anti-Krebswirkstoff, einen Wirkstoff, der Wundheilung fördert, einen Wirkstoff, der Vaskularisierung fördert, einen Wirkstoff, der eine Infektion behandelt oder dieser vorbeugt, einen Wirkstoff, der Restenose vorbeugt, einen Wirkstoff, der Makuladegeneration reduziert, einen Wirkstoff, der immunologische Abstoßung verhindert, einen Wirkstoff, der Thrombose verhindert, oder einen Wirkstoff, der Entzündung behandelt; wahlweise worin (i) die pharmazeutische Zusammensetzung einen Anti-Krebswirkstoff umfasst; wahlweise worin der Anti-Krebswirkstoff Doxorubicin umfasst; oder (ii) die pharmazeutische Zusammensetzung einen Wirkstoff umfasst, der Wundheilung fördert; oder (iii) die pharmazeutische Zusammensetzung einen Wirkstoff, der Vaskularisierung fördert oder einen Wirkstoff umfasst, der Restenose verhindert; oder (iv) die pharmazeutische Zusammensetzung einen Wirkstoff umfasst, der eine Infektion verhindert oder dieser vorbeugt.

8. Nachfüllbares Wirkstoffverabreichungssystem nach Anspruch 1, worin das Hydrogel Kollagen, Alginat, Polysaccharid, Hyaluronsäure (HA), Polyethylenglykol (PEG), Polyglykolsäure (PGA), Poly-(L-lactid) (PLA), Poly-(lactid-co-glykolid (PLGA), oder Polylactid-co-glykolsäure umfasst; wahlweise worin das Hydrogel Alginat umfasst.

9. Nachfüllbares Wirkstoffverabreichungssystem nach Anspruch 8, worin das Hydrogel ein Alginat-Hydrogel umfasst.

10. Nachfüllbares Wirkstoffverabreichungssystem nach Anspruch 9, worin das Hydrogel ein modifiziertes Alginat umfasst.

11. Nachfüllbares Wirkstoffverabreichungssystem nach Anspruch 10, worin das Hydrogel ein teilweise oxidiertes Alginat umfasst.

12. Nachfüllbares Wirkstoffverabreichungssystem nach Anspruch 1, worin die Wirkstoffverabreichungseinrichtung ein Azid-modifiziertes Alginat mit der nachstehenden Struktur umfasst:

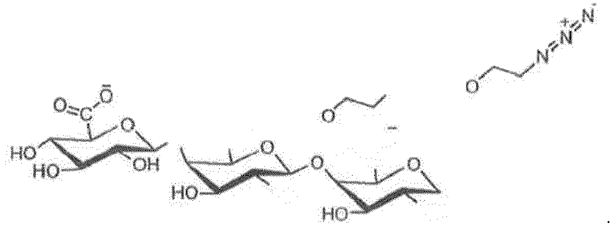

13. Nachfüllbares Wirkstoffverabreichungssystem nach Anspruch 1, worin die spaltbare Bindung eine Hydrazinbindung ist.

14. Nachfüllbares Wirkstoffverabreichungssystem nach Anspruch 1, worin die pharmazeutische Zusammensetzung Doxorubicin umfasst.

15. Nachfüllbares Wirkstoffverabreichungssystem nach Anspruch 1, worin das System mindestens zwei Wirkstoffverabreichungseinrichtungen umfasst; wahlweise worin das System mindestens zwei Wirkstoffnachfüllungen umfasst; wahlweise worin jede der Wirkstoffnachfüllungen eine unterschiedliche pharmazeutische Zusammensetzung und ein unterschiedliches Zielmolekül umfasst; wahlweise worin jede Wirkstoffverabreichungseinrichtung ein unterschiedliches Zielerkennungsmolekül umfasst; wahlweise worin jede Wirkstoffnachfüllung an eine unterschiedliche Wirkstoffverabreichungseinrichtung bindet.

16. Nachfüllbares Wirkstoffverabreichungssystem nach Anspruch 1 zur Verwendung bei dem Nachfüllen einer Wirkstoffverabreichungseinrichtung *in vivo,* worin
i) die Wirkstoffverabreichungseinrichtung zur Verabreichung an die Person und zum Implantieren an einer gewünschten Stelle in der Person ist; und
ii) die Wirkstoffnachfüllung zur anschließenden Verabreichung an die Person ist; wahlweise worin die Wirkstoffnachfüllung zur anschließenden Verabreichung an die Person an einem Ort ist, der von der Einrichtung entfernt ist;
worin die Wirkstoffnachfüllung in der Person mobil ist, bis das Zielmolekül auf der Wirkstoffnachfüllung an das Zielerkennungsmolekül auf der Wirkstoffverabreichungseinrichtung an der gewünschten Stelle in der Person bindet; und das Zielmolekül und das Zielerkennungsmolekül ein Zweikomponenten-Bindungspaar ausbilden; und
wobei nach Binden des Zielmoleküls an das Zielerkennungsmolekül, die spaltbare Bindung gespalten wird und die Wirkstoffnachfüllung die pharmazeutische Zusammensetzung an die Wirkstoffverabreichungseinrichtung liefert, wodurch die Wirkstoffverabreichungseinrichtung nachgefüllt wird;
wahlweise worin die Wirkstoffnachfüllung zur oralen, bukkalen, sublingualen, rektalen, intravenösen, intraarteriellen, intraossären, intramuskulären, intracerebralen, intracerebroventrikulären, intrathekalen, subkutanen, intraperitonealen, intraokularen, intranasalen, transdermalen, epiduralen, intracranialen, perkutanen, intravaginalen, intrauterinen, intravitrealen oder transmukosalen Verabreichung, oder zur Verabreichung über Injektion, über Aerosol-basierte Verabreichung, oder über Implantation ist.

17. Nachfüllbares Wirkstoffverabreichungssystem nach Anspruch 1 zur Verwendung bei der Erhaltung oder der Reduktion der Größe eines Tumors in einer Person, worin die pharmazeutische Zusammensetzung einen Anti-Krebswirkstoff umfasst; wahlweise
worin die Wirkstoffverabreichungseinrichtung mehrmals durch Verabreichung der Wirkstoffnachfüllung nachgefüllt wird; wahlweise worin die Wirkstoffnachfüllung zur oralen, bukkalen, sublingualen, rektalen, intravenösen, intraarteriellen, intraossären, intramuskulären, intracerebralen, intracerebroventrikulären, intrathekalen, subkutanen, intraperitonealen, intraokularen, intranasalen, transdermalen, epiduralen, intracranialen, perkutanen, intravaginalen, intrauterinen, intravitrealen oder transmukosalen Verabreichung, oder zur Verabreichung über Injektion, über Aerosol-basierte Verabreichung, oder über Implantation ist; wahlweise
worin der Anti-Krebswirkstoff Doxorubicin umfasst; wahlweise worin die Person an einem Krebs leidet, der ein solider Krebs oder ein hämatologischer Krebs ist.

18. Nachfüllbares Wirkstoffverabreichungssystem nach Anspruch 1 zur Verwendung bei
(a) der Reduktion der Krebsprogression in einer Person, worin die pharmazeutische Zusammensetzung einen Anti-Krebswirkstoff umfasst; wahlweise
wobei (i) die Wirkstoffverabreichungseinrichtung durch orale, intraperitoneale, intravenöse, oder intraarterielle Verabreichung der Wirkstoffnachfüllung an die Person nachgefüllt wird; (ii) wahlweise wobei Schritt (i) wiederholt wird; wodurch die Krebsprogression in der Person reduziert wird; oder
(b) der Förderung von Wundheilung in einer Person, worin die pharmazeutische Zusammensetzung Angiogenese und/oder Reifung oder Umbildung eines bestehenden Blutgefäßes fördert; wahlweise
wobei (i) die Wirkstoffverabreichungseinrichtung durch Verabreichung der Wirkstoffnachfüllung an die Person nachgefüllt wird; (ii) wahlweise wobei Schritt (i) wiederholt wird; wodurch die Wundheilung in der Person gefördert wird; wahlweise
worin die Nachfüllung zur oralen, bukkalen, sublingualen, rektalen, intravenösen, intraarteriellen, intraossären, intramuskulären, intracerebralen, intracerebroventrikulären, intrathekalen, subkutanen, intraperitonealen, intraokularen, intranasalen, transdermalen, epiduralen, intracranialen, perkutanen, intravaginalen, intrauterinen, intravitrealen oder transmukosalen Verabreichung, oder zur Verabreichung über Injektion, über Aerosol-basierte Verabreichung, oder über Implantation ist; oder
(c) der Reduktion oder der Entzündungskontrolle in einer Person, worin die pharmazeutische Zusammensetzung ein Anti-inflammatorisches Agens umfasst; wahlweise
wobei (i) die Wirkstoffverabreichungseinrichtung durch Verabreichung der Wirkstoffnachfüllung an die Person nachgefüllt wird; (ii) wahlweise wobei Schritt (i) wiederholt wird; wodurch die Entzündung in der Person reduziert oder kontrolliert wird; wahlweise
worin die Nachfüllung zur oralen, bukkalen, sublingualen, rektalen, intravenösen, intraarteriellen, intraossären, intramuskulären, intracerebralen, intracerebroventrikulären, intrathekalen, subkutanen, intraperitonealen, intraokularen, intranasalen, transdermalen, epiduralen, intracranialen, perkutanen, intravaginalen, intrauterinen, intravitrealen oder transmukosalen Verabreichung, oder zur Verabreichung über Injektion, über Aerosol-basierte Verabreichung, oder über Implantation ist; oder
(d) der Behandlung einer Augenerkrankung in einer Person, worin die pharmazeutische Zusammensetzung die Augenerkrankung behandelt; wahlweise
worin die Augenerkrankung ein Katarakt, ein Glaukom, eine Netzhauterkrankung, eine Hornhauterkrankung, Arteriitis temporalis, oder Makuladegeneration umfasst; wahlweise
wobei (i) die Wirkstoffverabreichungseinrichtung durch Verabreichung der Wirkstoffnachfüllung an das Auge der Person nachgefüllt wird; (ii) wahlweise wobei Schritt (i) wiederholt wird; wodurch eine Augenerkrankung in der Person behandelt wird; oder
(e) der Behandlung von Arrhythmie in einer Person, worin die pharmazeutische Zusammensetzung die Arrhythmie behandelt; wahlweise
wobei (i) die Wirkstoffverabreichungseinrichtung durch Verabreichung der Wirkstoffnachfüllung an das Herz der Person nachgefüllt wird; (ii) wahlweise wobei Schritt (i) wiederholt wird; wodurch die Arrhythmie in der Person behandelt wird.

## Revendications

1. Système d'administration de médicament rechargeable comprenant un dispositif d'administration de médicament et une recharge de médicament,
dans lequel le dispositif d'administration de médicament comprend un hydrogel et une molécule de reconnaissance de cible et est approprié pour une implantation à un emplacement souhaité dans un sujet ;
dans lequel la recharge de médicament comprend une composition pharmaceutique attachée à une molécule cible par l'intermédiaire d'un lieur clivable et la recharge de médicament est appropriée pour une administration subséquente au sujet ;
dans lequel la recharge de médicament est mobile dans le sujet jusqu'à ce que la molécule cible sur la recharge de médicament se lie à la molécule de reconnaissance de cible sur le dispositif d'administration de médicament à un emplacement souhaité dans le sujet ; et la molécule cible et la molécule de reconnaissance de cible forment une paire de liaison à deux composants ;
dans lequel, lors de la liaison de la molécule cible à la molécule de reconnaissance de cible, le lieur clivable est clivé et la recharge de médicament délivre la composition pharmaceutique au dispositif d'administration de médicament, rechargeant ainsi le dispositif d'administration de médicament ;
dans lequel le lieur clivable comprend une liaison choisie dans le groupe consistant en une liaison hydrazone, une liaison acétal, une liaison cétal, une liaison oxime, une liaison imine et une liaison disulfure ; et
dans lequel la molécule cible comprend un groupe fonctionnel bioorthogonal et la molécule de reconnaissance de cible comprend un groupe fonctionnel complémentaire, dans lequel le groupe fonctionnel bioorthogonal est capable de réagir par chimie click avec le groupe fonctionnel complémentaire pour former une liaison covalente.

2. Système rechargeable d'administration de médicament selon la revendication 1, dans lequel
(i) le groupe fonctionnel bioorthogonal comprend un alcène et le groupe fonctionnel complémentaire comprend une tétrazine (Tz) ; facultativement dans lequel l'alcène comprend un cyclooctène ; facultativement dans lequel le cyclooctène comprend du transcyclooctène (TCO) ; ou
(ii) le groupe fonctionnel bioorthogonal comprend un alcyne et le groupe fonctionnel complémentaire comprend un azide ; facultativement dans lequel l'alcyne comprend un cyclooctyne ; facultativement dans lequel le cyclooctyne comprend du dibenzocyclooctyne (DBCO) ; ou
(iii) la molécule cible comprend du dibenzocyclooctyne (DBCO) et la molécule de reconnaissance de cible comprend un azide ; ou
(iv) le groupe fonctionnel bioorthogonal comprend un alcène et le groupe fonctionnel complémentaire comprend une tétrazine (Tz) ; facultativement dans lequel l'alcène comprend un norbornène (NOR).

3. Système d'administration de médicament rechargeable selon la revendication 1, dans lequel le groupe fonctionnel bioorthogonal comprend un alcène et le groupe fonctionnel complémentaire comprend une tétrazine (Tz) ; dans lequel l'alcène comprend un transcyclooctène (TCO).

4. Système d'administration de médicament rechargeable selon la revendication 1, dans lequel le groupe fonctionnel bioorthogonal comprend un alcène et le groupe fonctionnel complémentaire comprend une tétrazine (Tz) ; dans lequel l'alcène comprend du norbornène (NOR).

5. Système d'administration de médicament rechargeable selon la revendication 1, dans lequel le groupe fonctionnel bioorthogonal comprend un alcyne et le groupe fonctionnel complémentaire comprend un azide ; dans lequel l'alcyne comprend un cyclooctène.

6. Système d'administration de médicament rechargeable selon la revendication 1, dans lequel le groupe fonctionnel bioorthogonal comprend un alcyne et le groupe fonctionnel complémentaire comprend un azide ; dans lequel l'alcyne comprend un dibenzocyclooctyne (DBCO).

7. Système d'administration de médicament rechargeable selon la revendication 1, dans lequel la composition pharmaceutique comprend un médicament anticancéreux, un médicament qui favorise la cicatrisation des plaies, un médicament qui favorise la vascularisation, un médicament qui traite ou prévient une infection, un médicament qui prévient la resténose, un médicament qui réduit la dégénérescence maculaire, un médicament qui prévient un rejet immunologique, un médicament qui prévient une thrombose ou un médicament qui traite une inflammation ; facultativement dans lequel (i) la composition pharmaceutique comprend un médicament anticancéreux ; facultativement dans lequel le médicament anticancéreux comprend de la doxorubicine ; ou (ii) la composition pharmaceutique comprend un médicament qui favorise la cicatrisation des plaies ; ou (iii) la composition pharmaceutique comprend un médicament qui favorise la vascularisation ou un médicament qui prévient la resténose ; ou (iv) la composition pharmaceutique comprend un médicament qui traite ou prévient une infection.

8. Système d'administration de médicament rechargeable selon la revendication 1, dans lequel l'hydrogel comprend du collagène, de l'alginate, un polysaccharide, de l'acide hyaluronique (HA), du polyéthylène glycol (PEG), du polyglycolide (PGA), du poly(L-lactide) (PLA), du poly(lactide-co-glycolide) (PLGA), ou de l'acide polylactique-co-glycolique ; facultativement dans lequel l'hydrogel comprend de l'alginate.

9. Système d'administration de médicament rechargeable selon la revendication 8, dans lequel l'hydrogel comprend un hydrogel d'alginate.

10. Système d'administration de médicament rechargeable selon la revendication 9, dans lequel l'hydrogel comprend un alginate modifié.

11. Système d'administration de médicament rechargeable selon la revendication 10, dans lequel l'hydrogel comprend un alginate partiellement oxydé.

12. Système d'administration de médicament rechargeable selon la revendication 1, dans lequel le dispositif d'administration de médicament comprend un alginate modifié par azide ayant la structure suivante :

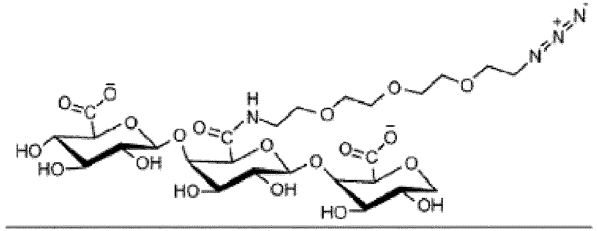

13. Système d'administration de médicament rechargeable selon la revendication 1, dans lequel le lieur clivable est un lieur hydrazone.

14. Système d'administration de médicament rechargeable selon la revendication 1, dans lequel la composition pharmaceutique comprend de la doxorubicine.

15. Système d'administration de médicament rechargeable selon la revendication 1, dans lequel ledit système comprend au moins deux dispositifs d'administration de médicament ; facultativement dans lequel ledit système comprend au moins deux recharges de médicament ; facultativement dans lequel chacune des recharges de médicament comprend une composition pharmaceutique différente et une molécule cible différente ; facultativement dans lequel chaque dispositif d'administration de médicament comprend une molécule de reconnaissance de cible différente ; facultativement dans lequel chaque recharge de médicament se lie à un dispositif d'administration de médicament différent.

16. Système d'administration de médicament rechargeable selon la revendication 1 pour une utilisation dans la recharge d'un dispositif d'administration de médicament *in vivo,* dans lequel :
i) le dispositif d'administration de médicament est destiné à l'administration au sujet et à l'implantation à un emplacement souhaité dans le sujet ; et
ii) la recharge de médicament est destinée à une administration ultérieure au sujet ; facultativement dans lequel la recharge de médicament est destinée à une administration ultérieure au sujet au niveau d'un site situé à distance du dispositif ;
dans lequel la recharge de médicament est mobile à l'intérieur du sujet jusqu'à ce que la molécule cible sur la recharge de médicament se lie à la molécule de reconnaissance de cible sur le dispositif d'administration de médicament à l'emplacement souhaité dans le sujet ; et la molécule cible et la molécule de reconnaissance de cible forment une paire de liaison à deux composants ; et
dans lequel, lors de la liaison de la molécule cible à la molécule de reconnaissance de cible, le lieur clivable est clivé et la recharge de médicament délivre la composition pharmaceutique au dispositif d'administration de médicament, rechargeant ainsi le dispositif d'administration de médicament ;
facultativement dans lequel ladite recharge de médicament est destinée à une administration par voie orale, buccale, sublinguale, rectale, intraveineuse, intra-artérielle, intra-osseuse, intramusculaire, intracérébrale, intracérébroventriculaire, intrathécale, sous-cutanée, intrapéritonéale, intra-oculaire, intranasale, transdermique, épidurale, intracrânienne, percutanée, intravaginale, intra-utérine, intravitréenne, transmuqueuse ou par injection, par administration à base d'aérosol ou par implantation.

17. Système d'administration de médicament rechargeable selon la revendication 1 pour une utilisation dans le maintien ou la réduction de la taille d'une tumeur chez un sujet, dans lequel la composition pharmaceutique comprend un médicament anticancéreux ; facultativement
dans lequel le dispositif d'aministration de médicament est rechargé plusieurs fois par administration de la recharge de médicament ; facultativement
dans lequel la recharge de médicament est pour une administration par voie orale, buccale, sublinguale, rectale, intraveineuse, intra-artérielle, intra-osseuse, intramusculaire, intracérébrale, intracérébroventriculaire, intrathécale, sous-cutanée, intrapéritonéale, intra-oculaire, intranasale, transdermique, épidurale, intracrânienne, percutanée, intravaginale, intra-utérine, intravitréenne, transmuqueuse ou par injection, par administration à base d'aérosol ou par implantation ; facultativement
dans lequel le médicament anticancéreux comprend de la doxorubicine ; facultativement dans lequel le sujet souffre d'un cancer qui est un cancer solide ou un cancer hématologique.

18. Système d'administration de médicament rechargeable selon la revendication 1 pour une utilisation
(a) dans la réduction de la progression du cancer chez un sujet, la composition pharmaceutique comprenant un médicament anticancéreux ; facultativement
dans lequel (i) le dispositif d'administration de médicament est rechargé par administration de la recharge de médicament au sujet par voie orale, intrapéritonéale, intraveineuse ou intra-artérielle ; (ii) facultativement dans la répétition de l'étape (i) ; réduisant ainsi la progression du cancer chez le sujet ; ou
(b) pour favoriser la cicatrisation de plaies chez un sujet, la composition pharmaceutique favorisant l'angiogenèse et / ou la maturation ou le remodelage d'un vaisseau sanguin existant ; facultativement dans lequel (i) le dispositif d'administration de médicament est rechargé par administration de la recharge de médicament au sujet ; (ii) facultativement dans la répétition de l'étape (i) ; favorisant ainsi la cicatrisation des plaies chez le sujet ; facultativement
dans lequel ladite recharge est pour une administration par voie orale, buccale, sublinguale, rectale, intraveineuse, intra-artérielle, intra-osseuse, intramusculaire, intracérébrale, intracérébroventriculaire, intrathécale, sous-cutanée, intrapéritonéale, intra-oculaire, intranasale, transdermique, épidurale, intracrânienne, percutanée, intravaginale, intra-utérine, intravitréenne, transmuqueuse ou par injection, par administration à base d'aérosol ou par implantation ; ou
(c) dans la réduction ou le contrôle de l'inflammation chez un sujet, la composition pharmaceutique comprenant un agent anti-inflammatoire ; facultativement
dans lequel (i) le dispositif d'administration de médicament est rechargé par administration de la recharge de médicament au sujet ; (ii) facultativement dans la répétition de l'étape (i) ; réduisant ou contrôlant ainsi l'inflammation chez le sujet ; facultativement
dans lequel ladite recharge est pour une administration par voie orale, buccale, sublinguale, rectale, intraveineuse, intra-artérielle, intra-osseuse, intramusculaire, intracérébrale, intracérébroventriculaire, intrathécale, sous-cutanée, intrapéritonéale, intra-oculaire, intranasale, transdermique, épidurale, intracrânienne, percutanée, intravaginale, intra-utérine, intravitréenne, transmuqueuse ou par injection, par administration à base d'aérosol ou par implantation ; ou
(d) dans le traitement d'une maladie oculaire chez un sujet, la composition pharmaceutique traitant ladite maladie oculaire ; facultativement
dans lequel la maladie oculaire comprend la cataracte, le glaucome, une maladie rétinienne, une maladie cornéenne, l'artérite temporale ou la dégénérescence maculaire ; facultativement
dans lequel (i) le dispositif d'administration de médicament est rechargé par administration de la recharge de médicament dans l'œil du sujet ; (ii) facultativement dans la répétition de l'étape (i) ; traitant ainsi une maladie oculaire chez le sujet ; ou
(e) dans le traitement de l'arythmie chez un sujet, la composition pharmaceutique traitant ladite arythmie ; facultativement
dans lequel (i) le dispositif d'administration de médicament est rechargé par administration de la recharge de médicament dans le cœur du sujet ; (ii) facultativement dans la répétition de l'étape (i) ; traitant ainsi l'arythmie chez le sujet.
